(19) **Europäisches Patentamt** / **European Patent Office** / **Office européen des brevets**

(11) **EP 4 722 360 A1**

(12) **EUROPEAN PATENT APPLICATION**
published in accordance with Art. 153(4) EPC

(43) Date of publication:
08.04.2026 Bulletin 2026/15

(21) Application number: 24807289.4

(22) Date of filing: 17.05.2024

(51) International Patent Classification (IPC):
$C12N\ 15/09^{(2006.01)}$    $C12N\ 1/13^{(2006.01)}$
$C12N\ 1/15^{(2006.01)}$    $C12N\ 1/19^{(2006.01)}$
$C12N\ 5/10^{(2006.01)}$    $C12N\ 15/63^{(2006.01)}$

(52) Cooperative Patent Classification (CPC):
C12N 5/10; C12N 15/09; C12N 15/63; C12N 15/79;
C12N 15/80; C12N 15/81

(86) International application number:
PCT/JP2024/018374

(87) International publication number:
WO 2024/237339 (21.11.2024 Gazette 2024/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 18.05.2023 JP 2023082263

(71) Applicants:
• MAZDA MOTOR CORPORATION
Hiroshima 730-8670 (JP)
• Hiroshima University
Higashi-Hiroshima-shi
Hiroshima 739-8511 (JP)
• INSTITUTE OF SCIENCE TOKYO
Tokyo 152-8550 (JP)

(72) Inventors:
• KURITA Tomokazu
Hiroshima-shi, Hiroshima 739-8511 (JP)

• SAKUMA Tetsushi
Hiroshima-shi, Hiroshima 739-8511 (JP)
• YAMAMOTO Takashi
Hiroshima-shi, Hiroshima 739-8511 (JP)
• SAKAMOTO Atsushi
Hiroshima-shi, Hiroshima 739-8511 (JP)
• OKAZAKI Kumiko
Hiroshima-shi, Hiroshima 739-8511 (JP)
• MAEDA Shinichiro
Aki-gun, Hiroshima 730-8670 (JP)
• OHTA Hiroyuki
Tokyo 152-8550 (JP)
• IWAI Masako
Tokyo 152-8550 (JP)

(74) Representative: J A Kemp LLP
80 Turnmill Street
London EC1M 5QU (GB)

Remarks:
The complete document including Reference
Table(s) and the Sequence Listing(s) can be
downloaded from the EPO website

(54) **DNA EDITING METHOD, CELL PRODUCTION METHOD USING SAME, AND DNA EDITING VECTOR AND DNA EDITING KIT FOR USE IN SAID METHODS**

(57) A method for editing DNA, which is a method for inserting a desired nucleotide sequence at a target site of a target DNA in a cell or removing the target site,
the method comprising an introduction step of introducing a vector and a site-specific nuclease system into a cell to bring them into contact with a target DNA, wherein the vector comprises a first promoter P1 and structure (1): 5'-M1-Hv1-D-Hv2-M2-3', wherein M1 represents a 5' side fragment of a nucleotide sequence encoding a selection marker gene and is operably linked to P1, Hv1 represents a nucleotide sequence homologous to a first nucleotide sequence Ht1 on the 5' side of the target site of the target DNA, D represents the desired nucleotide sequence but may be absent, Hv2 represents a nucleotide sequence homologous to a second nucleotide sequence Ht2 on the 3' side of the target site of the target DNA, and M2 represents the remaining 3' side fragment of the nucleotide sequence encoding the selection marker gene,
a cleavage step of generating from the vector, by the site-specific nuclease system, a fragment represented by structure (2): 5'-Hv1-D-Hv2-3' and a fragment represented by structure (3): 5'-M2-P1-M1-3', and cleaving the target site or its vicinity in the target DNA;

an editing step in which the target DNA and structure (2) bind depending on the homology between Ht1 and Hv1 and bind depending on the homology between Ht2 and Hv2, and the desired nucleotide sequence D is inserted at the target site or the target site is removed; and

a selection step of obtaining a selection vector in which, in the fragment represented by structure (3), the 3' end of M1 and the 5' end of M2 are ligated to obtain a selection vector that contains a functional selection marker gene operably linked to P1.

[Fig. 1A]

**Description**

[Technical Field]

The present invention relates to a DNA editing method, a cell production method using the same, and a DNA editing vector and a DNA editing kit for use in said methods.

[Background Art]

In recent years, the development of DNA editing technologies in useful organisms has been actively conducted; for example, methods have been developed for specifically cleaving a target site of a target DNA in a cell or the vicinity thereof and editing the nucleotide sequence of the target site, using site-specific nuclease systems such as a CRISPR-Cas system, a system in which a nuclease is fused to a TAL effector (TALE) (TALEN), a system in which a nuclease is fused to a zinc finger (ZF), and the like.

In these DNA editing technologies, a method of also introducing into a cell a nucleotide sequence or the like encoding a selection marker gene for confirming the editing is mainly adopted. For example, NPL 1 (Yan et al., Molecular Therapy: Nucleic Acids, Vol. 19 March, 2020, p. 775-789) describes a method for selecting a cell in which a CRISPR-Cas system has been expressed, by introducing a vector including the CRISPR-Cas system and an inactive puromycin resistance gene into a cell, whereby the inactive puromycin resistance gene is activated by the cleavage activity of the CRISPR-Cas system. For example, NPL 2 (Mashiko et al., Develop. Growth Differ. 2014, 56, p. 122-129) also describes a method for selecting a cell in which a CRISPR-Cas system has been expressed by causing it to produce fluorescence, by introducing into the cell a vector in which a sequence that is cleaved by the CRISPR-Cas system is sandwiched between two divided parts of a nucleotide sequence encoding GFP, which is a fluorescent protein.

However, a technology for using these methods for a DNA insertion method for inserting a nucleotide sequence into a target DNA has not yet been developed. In a DNA insertion method, it is also necessary for the orientation and position of the nucleotide sequence to be inserted to be accurate. As such a DNA insertion method, for example, a method is known for replacing the desired nucleotide sequence with the target sequence or inserting the desired nucleotide sequence into the target sequence by means of homologous recombination (HR), by respectively adding, to both ends of a desired nucleotide sequence, a sequence homologous to 500 to 2000 bases of the nucleotide sequences at both ends of the target sequence. However, this method has drawbacks in that the vector to be used extends to a long chain and is not easy to prepare, the homologous recombination efficiency is low, or it is applicable only to limited cells. As another DNA insertion method for the purpose of resolving such drawbacks, a method utilizing microhomology-mediated end joining (MMEJ), which utilizes a short homologous sequence of about 20 bases, is known; for example, PTL 1 (International Publication No. WO 2015/068785) describes a method for inserting a desired nucleotide sequence into a predetermined site by introducing a site-specific nuclease system and a vector including the desired nucleotide sequence into a cell.

However, with conventional DNA insertion technologies, because it is necessary to also introduce into the cell a sequence or the like encoding the aforementioned selection marker gene for confirming the insertion, a foreign gene other than such a nucleotide sequence of interest remains within the obtained cell. In a cell including such a foreign gene, its use as a genetically modified cell is subject to various restrictions, and therefore its usefulness is low.

As a method for removing a foreign gene other than the nucleotide sequence of interest, for example, NPL 3 (Kurita et al., Scientific reports, 2022, 12:2480, https://doi.org/10.1038/s41598-022-06495-y) describes a method for causing an all-in-one PtTALEN-ARS vector to be eliminated from the cell by culturing it, after introducing TALEN into a cell using the all-in-one PtTALEN-ARS vector that encodes a nucleotide sequence encoding CEN/ARS and TALEN to achieve desired cleavage.

[Citation List]

[Patent Literature]

[PTL 1] International Publication No. WO 2015/068785

[Non Patent Literature]

[NPL 1] Yan et al., Molecular Therapy: Nucleic Acids, Vol. 19 March, 2020, p. 775-789
[NPL 2] Mashiko et al., Develop. Growth Differ. 2014, 56, p. 122-129
[NPL 3] Kurita et al., Scientific reports, 2022, 12:2480, https://doi.org/10.1038/s41598-022-06495-y

[Summary of Invention]

[Technical Problem]

The present invention has been made in view of the problems of the conventional art described above, and an object thereof is to provide a DNA editing method capable of accurately and efficiently inserting a desired nucleotide sequence at a target site of a target DNA in a cell or removing the target site by a vector, a cell production method using the same, and a DNA editing vector and a DNA editing kit for use in these methods.

[Solution to Problem]

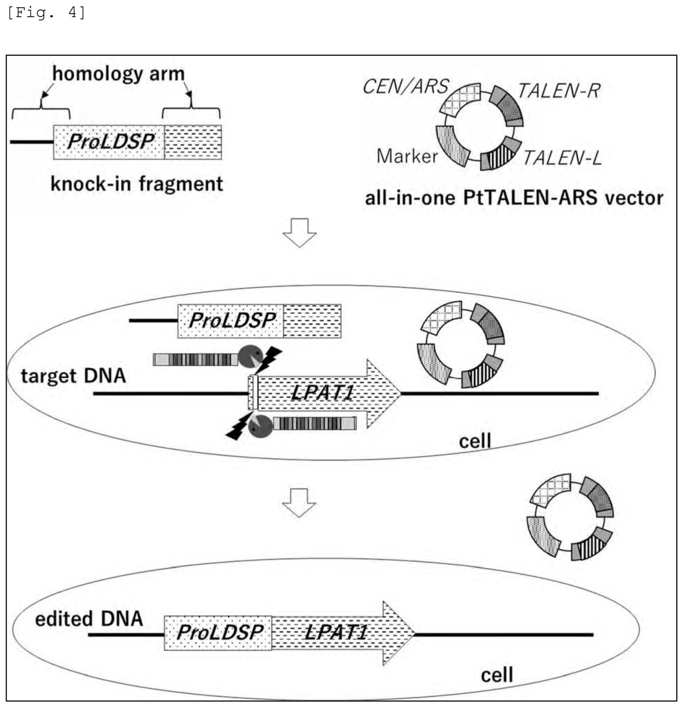

The present inventors have conducted diligent research to achieve the above object; first, they set the site immediately before the start codon of the target gene (LPAT1) of the genomic DNA as a target site, and attempted to insert an endogenous promoter (ProLDSP) here. The DNA to be inserted was a nucleotide fragment (knock-in fragment) to which was added a respectively homologous sequence (homology arm) to the sequence immediately before the start codon of LPAT1 (first nucleotide sequence) and the sequence after the start codon (second nucleotide sequence) at both ends of the nucleotide sequence of ProLDSP, and they designed a TALEN, which is a site-specific nuclease, so as to cleave the target site or its vicinity. It was considered that by introducing these into a cell, the genomic DNA is cleaved by TALEN, and both ends of the nucleotide fragment and the first nucleotide sequence and the second nucleotide sequence of the genomic DNA respectively bind in a homology-dependent manner, causing ProLDSP to be inserted at the target site and making it possible to overexpress LPAT1, which is a membrane lipid synthesis-related gene. Furthermore, by introducing and expressing, as the TALEN, the expression vector described in NPL 3 (all-in-one PtTALEN-ARS vector) including CEN/ARS as an autonomously replicating sequence, because it is possible to remove it by culturing after DNA insertion, it was considered possible to obtain a DNA-edited cell that does not include foreign genes other than ProLDSP (Fig. 4).

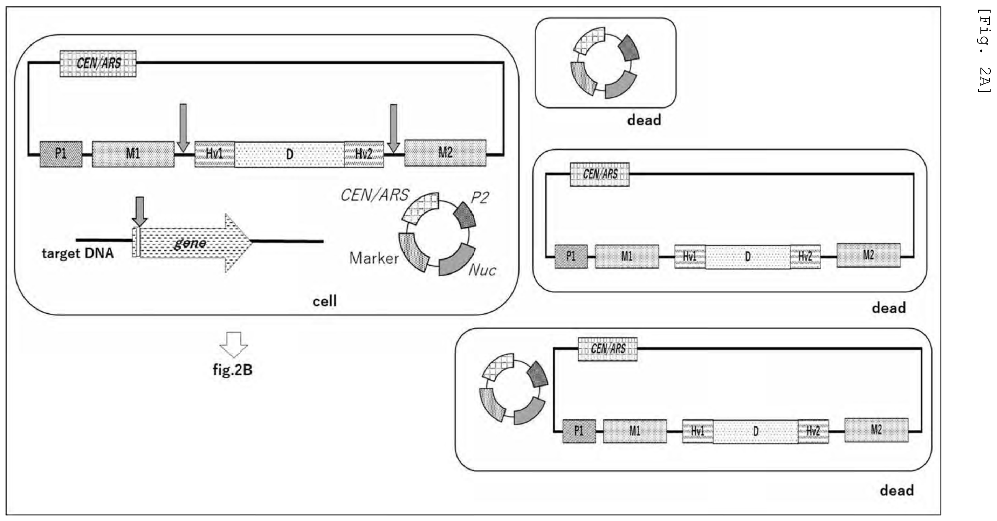

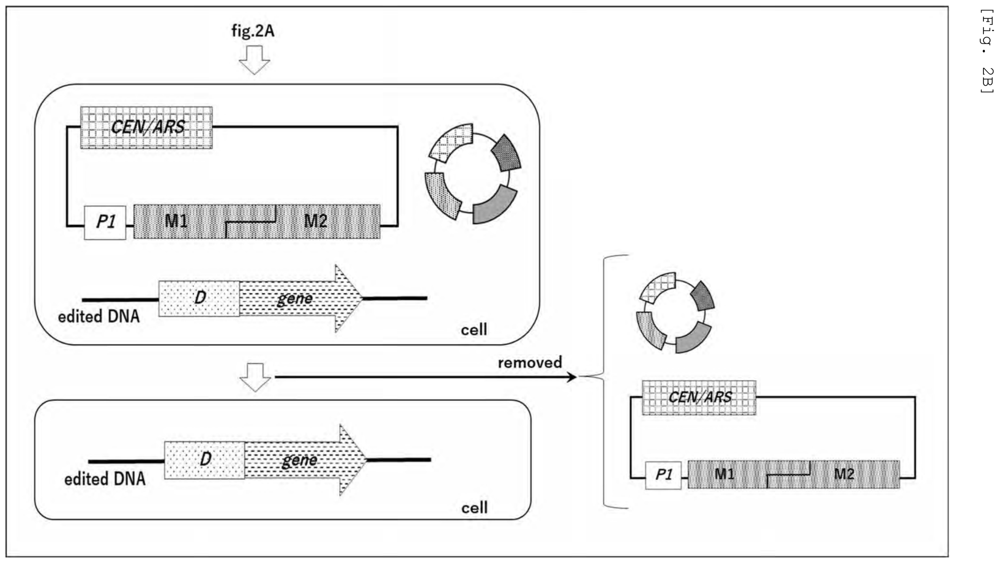

However, with the above method, it was difficult to obtain a desired DNA-edited cell into which the nucleotide fragment was inserted. Therefore, the present inventors conducted further studies and considered that one of the causes was that in the above method, the selection method for a cell into which the nucleotide fragment and the expression vector for the site-specific nuclease (all-in-one PtTALEN-ARS vector) were simultaneously introduced depends only on the selection marker derived from the expression vector for the site-specific nuclease, and therefore the acquisition efficiency of cells into which both were introduced is low, and they further constructed a new method. That is, in the above method, they designed the nucleotide fragment to be introduced on a vector including a selection marker gene (a selection marker gene different from that of the expression vector for the site-specific nuclease), and, they divided the selection marker gene into a 5' side fragment and a 3' side fragment, sandwiched the nucleotide fragment between them, and designed it so that this is excised by the site-specific nuclease. By introducing this and the expression vector for the site-specific nuclease into a cell, the genomic DNA is cleaved by the site-specific nuclease, and the nucleotide fragment is also excised; both ends of the nucleotide fragment and the first nucleotide sequence and the second nucleotide sequence of the genomic DNA respectively bind in a homology-dependent manner. At the same time, in a cell in which the cleavage and insertion have occurred, because the 5' side fragment and the 3' side fragment of the remaining vector fragment from which the nucleotide fragment was excised bind to form a functional selection marker gene, by performing selection according to the selection marker gene, it becomes possible to efficiently acquire the cell, that is, a cell into which the vector including the nucleotide fragment and the expression vector for the site-specific nuclease were simultaneously introduced, with high efficiency. Furthermore, cell growth is difficult with each introduced vector alone, and, in cases where the introduced vector includes CEN/ARS, because the vector in which the selection marker gene has functioned includes CEN/ARS, it is also possible to remove it by culturing after DNA insertion, together with the expression vector for the site-specific nuclease (Fig. 2A, Fig. 2B).

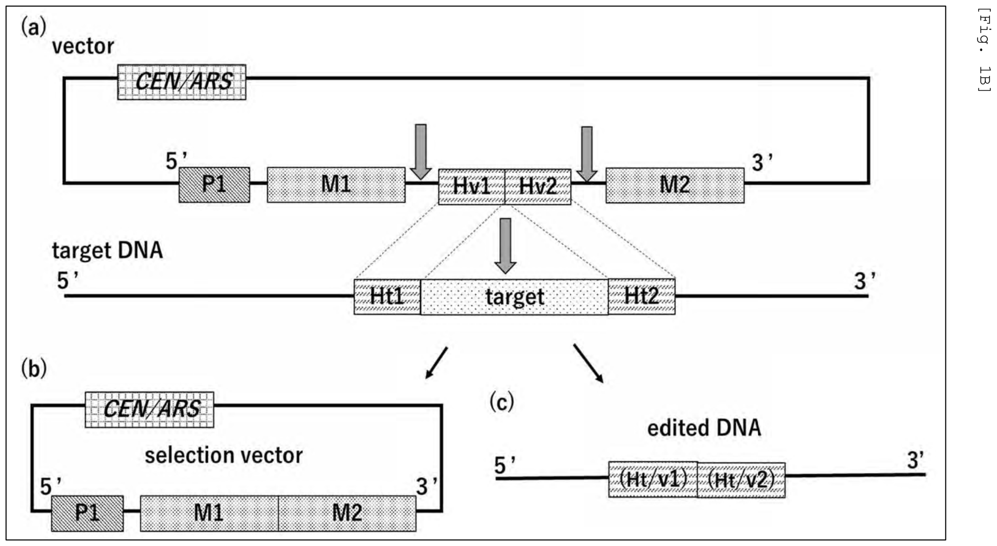

When the present inventors developed each vector based on the newly constructed method described above and conducted tests, they succeeded in accurately inserting a desired nucleotide fragment at a target site in a cell; furthermore, in cases where the introduced vector included CEN/ARS, they also succeeded in causing each vector to be eliminated from the cell after DNA insertion. The present inventors named this new DNA editing method the Non-Integrative Cleavage-dependent system (NICS system), and further named the vector used in this method, in which the selection marker gene is divided into a 5' side fragment and a 3' side fragment, and the above-described nucleotide fragment is sandwiched between them, a NICS vector; they found that according to these, it is possible to accurately and efficiently insert a desired nucleotide sequence at a target site of a target DNA in a cell by a vector; furthermore, they found that by including CEN/ARS in the vector, thereafter, the vector can be eliminated, and a genomic DNA-edited cell from which foreign genes other than the inserted nucleotide sequence have been removed can be obtained with high efficiency. Also, according to this method, they found that, conversely, by setting the nucleotide sequence to be inserted to 0 bases and the target site to several or more bases, the target site can be accurately and highly efficiently removed from the target DNA (Fig. 1B), and have completed the present invention. That is, the present invention includes the following aspects.

[1] A method for editing DNA, which is a method for inserting a desired nucleotide sequence at a target site of a target DNA in a cell or removing the target site,

the method comprising an introduction step of introducing a vector and a site-specific nuclease system into a cell to bring them into contact with a target DNA, wherein
the vector comprises

a first promoter P1, and
the following structure (1):

$$5\text{'-M1-Hv1-D-Hv2-M2-3'...}\qquad(1)$$

[in (1), M1 represents a 5' side fragment of a nucleotide sequence encoding a selection marker gene and is operably linked to the first promoter P1, Hv1 represents a nucleotide sequence homologous to a first nucleotide sequence Ht1 on the 5' side of the target site of the target DNA, D represents the desired nucleotide sequence but may be absent, Hv2 represents a nucleotide sequence homologous to a second nucleotide sequence Ht2 on the 3' side of the target site of the target DNA, M2 represents the remaining 3' side fragment of the nucleotide sequence encoding the selection marker gene, and in M1, Hv1, D, Hv2, and M2, adjacent sequences may partially overlap with each other.],

a cleavage step of generating from the vector, by the site-specific nuclease system, a fragment represented by the following structure (2):

$$5\text{'-Hv1-D-Hv2-3'...}\qquad(2)$$

and a fragment represented by the following structure (3):

$$5\text{'-M2-P1-M1-3'...}\qquad(3)$$

and cleaving the target site or its vicinity in the target DNA,
an editing step in which the target DNA and the fragment represented by structure (2) bind depending on the homology between Ht1 and Hv1 and bind depending on the homology between Ht2 and Hv2, and the desired nucleotide sequence D is inserted at the target site or the target site is removed, and
a selection step of obtaining a selection vector in which, in the fragment represented by structure (3), the 3' end of M1 and the 5' end of M2 are ligated to obtain a selection vector that contains a functional selection marker gene operably linked to P1.

[2] The DNA editing method according to [1], wherein

the vector further comprises CEN/ARS, which is an autonomously replicating sequence,
structure (3) generated in the cleavage step further comprises CEN/ARS, and
the selection step is a step of obtaining a selection vector in which, in the fragment represented by structure (3), the 3' end of M1 and the 5' end of M2 are ligated to obtain a selection vector that contains a functional selection marker gene operably linked to P1 and also contains CEN/ARS.

[3] The DNA editing method according to [1] or [2], wherein the length of Hv1 and the length of Hv2 are each independently 30 to 600 bases.
[4] The DNA editing method according to any one of [1] to [3], wherein

the vector comprises, as structure (1), the following structure (11):

$$5\text{'-M1-T1-Hv1-D-Hv2-T2-M2-3'...}\qquad(11)$$

[in (11), T1 represents a TALEN_1 binding region comprising a first DNA binding domain recognition sequence or its complementary sequence L1 and a second DNA binding domain recognition sequence or its complementary sequence R1, T2 represents a TALEN_2 binding region comprising a third DNA binding domain recognition sequence or its complementary sequence L2 and a fourth DNA binding domain recognition sequence or its complementary sequence R2, M1, Hv1, D, Hv2, and M2 are each as defined in structure (1), and in M1, T1, Hv1, D, Hv2, T2, and M2, adjacent sequences may partially overlap with each other.],

the site-specific nuclease system is a TALEN comprising a DNA binding domain and a nuclease domain, and comprises

a TALEN_1 that comprises a first DNA binding domain and a second DNA binding domain and generates the 5' end of structure (2),
a TALEN_2 that comprises a third DNA binding domain and a fourth DNA binding domain and generates the 3' end of structure (2), and
a TALEN_3 that comprises a fifth DNA binding domain that recognizes a nucleotide sequence on the 5' side of a region T3 comprising the target site of the target DNA as a fifth DNA binding domain recognition sequence or its complementary sequence L3, and a sixth DNA binding domain that recognizes a nucleotide sequence on the 3' side as a sixth DNA binding domain recognition sequence or its complementary sequence R3, and cleaves the target site or its vicinity in the target DNA.

[5] The DNA editing method according to any one of [1] to [3], wherein

the vector comprises in structure (1) a first guide RNA recognition sequence or its complementary sequence G1 and a second guide RNA recognition sequence or its complementary sequence G2, G1 is located between M1 and Hv1 and may overlap with at least one of M1 and Hv1, and G2 is located between M2 and Hv2 and may overlap with at least one of M2 and Hv2,
the site-specific nuclease system is a CRISPR-Cas system comprising a Cas protein and its guide RNA, and comprises

a CRISPR-Cas system_1 that comprises a first guide RNA, wherein the Cas protein generates the 5' end of structure (2),
a CRISPR-Cas system_2 that comprises a second guide RNA, wherein the Cas protein generates the 3' end of structure (2), and
a CRISPR-Cas system_3 that comprises a third guide RNA that recognizes a nucleotide sequence existing so as to comprise the target site of the target DNA as a third guide RNA recognition sequence or its complementary sequence G3, wherein the Cas protein cleaves the target site or its vicinity in the target DNA.

[6] The DNA editing method according to any one of [1] to [5], wherein the introduction of the site-specific nuclease system into the cell is
the introduction of a nuclease system expression vector comprising

CEN/ARS, which is an autonomously replicating sequence,
a second promoter P2, and
a nucleotide sequence Nuc that is operably linked to the second promoter P2 and encodes the site-specific nuclease system.

[7] The DNA editing method according to any one of [1] to [6], wherein

the introduction of the vector and the site-specific nuclease system into the cell is the introduction of an all-in-one vector comprising

a first promoter P1,
structure (1),
a second promoter P2, and
a nucleotide sequence Nuc that is operably linked to the second promoter P2 and encodes the site-specific nuclease system,
structure (3) generated in the cleavage step further comprises P2 and Nuc operably linked to P2, and

the selection step is a step of obtaining a selection vector in which, in the fragment represented by structure (3), the 3' end of M1 and the 5' end of M2 are ligated to obtain a selection vector that contains a functional selection marker gene operably linked to P1 and Nuc operably linked to P2.

[8] The DNA editing method according to [7], wherein

the all-in-one vector further comprises CEN/ARS, which is an autonomously replicating sequence,
structure (3) generated in the cleavage step further comprises CEN/ARS, and
the selection step is a step of obtaining a selection vector in which, in the fragment represented by structure (3), the 3' end of M1 and the 5' end of M2 are ligated to obtain a selection vector that contains a functional selection marker gene operably linked to P1, CEN/ARS, and Nuc operably linked to P2.

[9] The DNA editing method according to any one of [6] to [8], wherein the nucleotide sequence Nuc encoding the site-specific nuclease system is a nucleotide sequence encoding a fusion protein of a DNA binding domain and a nuclease domain.

[10] The DNA editing method according to any one of [6] to [8], wherein the nucleotide sequence Nuc encoding the site-specific nuclease system is a nucleotide sequence encoding a Cas protein and a nucleotide sequence encoding its guide RNA.

[11] A method for producing a DNA-edited cell in which a desired nucleotide sequence is inserted at a target site of a target DNA or the target site is removed, the method comprising:

a step of obtaining a cell in which the desired nucleotide sequence D is inserted at the target site or the target site is removed, and which comprises the selection vector, by the DNA editing method according to any one of [1] to [10], and
a step of selecting a DNA-edited cell in which the desired nucleotide sequence D is inserted at the target site or the target site is removed, using the selection marker gene contained in the selection vector as an indicator.

[12] The cell production method according to [11], further comprising a step of culturing the DNA-edited cell to eliminate the selection vector from the cell, wherein the vector is a vector further comprising CEN/ARS, which is an autonomously replicating sequence.

[13] A vector for inserting a desired nucleotide sequence at a target site of a target DNA or removing the target site by a site-specific nuclease system, the vector comprising:

a first promoter P1, and
the following structure (1'):

$$5'\text{-M1-Hv1'-D'-Hv2'-M2-3'}... \qquad (1')$$

[in (1'), M1 represents a 5' side fragment of a nucleotide sequence encoding a selection marker gene and is operably linked to the first promoter P1, Hv1' represents a nucleotide sequence homologous to a first nucleotide sequence Ht1 on the 5' side of the target site of the target DNA or an insertion site thereof, D' represents the desired nucleotide sequence or an insertion site thereof but may be absent, Hv2' represents a nucleotide sequence homologous to a second nucleotide sequence Ht2 on the 3' side of the target site of the target DNA or an insertion site thereof, M2 represents the remaining 3' side fragment of the nucleotide sequence encoding the selection marker gene, and in M1, Hv1', D', Hv2', and M2, adjacent sequences may partially overlap with each other.],

wherein a fragment represented by the following structure (2'):

$$5'\text{-Hv1'-D'-Hv2'-3'}... \qquad (2')$$

and a fragment represented by the following structure (3'):

$$5'\text{-M2-P1-M1-3'}... \qquad (3')$$

are generated by the site-specific nuclease system.

[14] The DNA editing vector according to [13], further comprising CEN/ARS, which is an autonomously replicating sequence, wherein a fragment further comprising CEN/ARS as structure (3') is generated by the site-specific nuclease system.

[15] The DNA editing vector according to [13] or [14],

comprising, as structure (1'), the following structure (11'):

$$5'\text{-}M1\text{-}T1'\text{-}Hv1'\text{-}D'\text{-}Hv2'\text{-}T2'\text{-}M2\text{-}3'... \qquad (11')$$

[in (11'), T1' represents a TALEN_1 binding region comprising a first DNA binding domain recognition sequence or its complementary sequence L1 and a second DNA binding domain recognition sequence or its complementary sequence R1, or an insertion site thereof, T2' represents a TALEN_2 binding region comprising a third DNA binding domain recognition sequence or its complementary sequence L2 and a fourth DNA binding domain recognition sequence or its complementary sequence R2, or an insertion site thereof, M1, Hv1', D', Hv2', and M2 are each as defined in structure (1'), and in M1, T1', Hv1', D', Hv2', T2', and M2, adjacent sequences may partially overlap with each other.],
wherein the site-specific nuclease system is a TALEN comprising a DNA binding domain and a nuclease domain, and comprises

a TALEN_1 that comprises a first DNA binding domain and a second DNA binding domain and generates the 5' end of structure (2'),
a TALEN_2 that comprises a third DNA binding domain and a fourth DNA binding domain and generates the 3' end of structure (2'), and
a TALEN_3 that comprises a fifth DNA binding domain that recognizes a nucleotide sequence on the 5' side of a region T3 comprising the target site of the target DNA as a fifth DNA binding domain recognition sequence or its complementary sequence L3, and a sixth DNA binding domain that recognizes a nucleotide sequence on the 3' side as a sixth DNA binding domain recognition sequence or its complementary sequence R3, and cleaves the target site or its vicinity in the target DNA.

[16] The DNA editing vector according to [13] or [14],

comprising in structure (1') a first guide RNA recognition sequence or its complementary sequence or an insertion site thereof G1', and a second guide RNA recognition sequence or its complementary sequence or an insertion site thereof G2', wherein G1' is located between M1 and Hv1' and may overlap with at least one of M1 and Hv1', and G2' is located between M2 and Hv2' and may overlap with at least one of M2 and Hv2',
wherein the site-specific nuclease system is a CRISPR-Cas system comprising a Cas protein and its guide RNA, and comprises

a CRISPR-Cas system_1 that comprises a first guide RNA, wherein the Cas protein generates the 5' end of structure (2'),
a CRISPR-Cas system_2 that comprises a second guide RNA, wherein the Cas protein generates the 3' end of structure (2'), and
a CRISPR-Cas system_3 that comprises a third guide RNA that recognizes a nucleotide sequence existing so as to comprise the target site of the target DNA as a third guide RNA recognition sequence or its complementary sequence G3, wherein the Cas protein cleaves the target site or its vicinity in the target DNA.

[17] The DNA editing vector according to any one of [13] to [16], which is an all-in-one vector comprising

a first promoter P1,
structure (1'),
a second promoter P2, and
a nucleotide sequence Nuc that is operably linked to the second promoter P2 and encodes the site-specific nuclease system,

wherein a fragment further comprising P2 and Nuc operably linked to P2 as structure (3') is generated by the site-specific nuclease system.
[18] The DNA editing vector according to any one of [13] to [17], wherein M1 and M2 are a nucleotide sequence in which the selection marker gene is divided such that it can function when the 3' end of M1 and the 5' end of M2 of the fragment represented by structure (3') bind.
[19] The DNA editing vector according to [18], wherein the 3' end of M1 and the 5' end of M2 are a nucleotide sequence that overlaps with each other, and the 3' end of M1 and the 5' end of M2 bind via the overlapping nucleotide sequence, and the selection marker gene functions.
[20] The DNA editing vector according to [19], wherein the length of the overlapping nucleotide sequence is 40 to 500 bases.

[21] The DNA editing vector according to [20], wherein a stop codon is added to the 3' end of M1 and/or the 5' end of M2, and the stop codon is removed from M1 and/or M2 by binding via the overlapping nucleotide sequence.

[22] A DNA editing kit, comprising the DNA editing vector according to any one of [13] to [21] and the site-specific nuclease system.

[23] The DNA editing kit according to [22], wherein the site-specific nuclease system is a nuclease system expression vector comprising

CEN/ARS, which is an autonomously replicating sequence,
a second promoter P2, and
a nucleotide sequence Nuc that is operably linked to the second promoter P2 and encodes the site-specific nuclease system.

[Advantageous Effects of Invention]

According to the present invention, it becomes possible to provide a DNA editing method capable of accurately and efficiently inserting a desired nucleotide sequence at a target site of a target DNA in a cell or removing the target site by a vector, a cell production method using the same, and a DNA editing vector and a DNA editing kit for use in these methods.

In addition, in cases where the vector further includes CEN/ARS, which is an autonomously replicating sequence, it also becomes possible to provide a DNA editing method by which, after inserting a desired nucleotide sequence at the target site of the target DNA or removing the target site from the target DNA by the vector, the vector can be eliminated, and a genomic DNA-edited cell from which foreign genes other than the inserted nucleotide sequence have been removed can be obtained with high efficiency, a cell production method using the same, and a DNA editing vector and a DNA editing kit for use in these methods.

[Brief Description of Drawings]

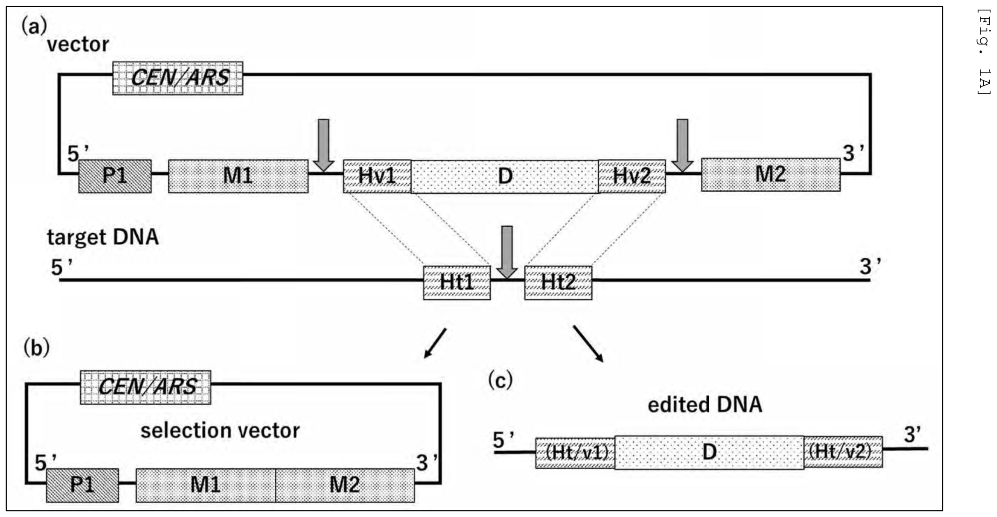

[Fig. 1A] Fig. 1A is a schematic diagram showing one embodiment of the insertion of a nucleotide sequence into a target DNA by a vector in the DNA editing method of the present invention. (a) is a schematic diagram showing the configuration of a DNA editing vector (vector) and a target DNA (target DNA) according to the DNA editing method of the present invention, (b) is a schematic diagram showing the configuration of a selection vector formed in the DNA editing method of the present invention, and (c) is a schematic diagram showing the configuration of a DNA (edited DNA) into which the nucleotide sequence has been inserted by the DNA editing method of the present invention.

[Fig. 1B] Fig. 1B is a schematic diagram showing one embodiment of the removal of a target site (target) from a target DNA by a vector in the DNA editing method of the present invention. (a) is a schematic diagram showing the configuration of a DNA editing vector (vector) and a target DNA (target DNA) according to the DNA editing method of the present invention, (b) is a schematic diagram showing the configuration of a selection vector formed in the DNA editing method of the present invention, and (c) is a schematic diagram showing the configuration of a DNA (edited DNA) from which the target site has been removed by the DNA editing method of the present invention.

[Fig. 1C] Fig. 1C is a schematic diagram showing one embodiment of the configuration of a DNA editing vector (vector) and a target DNA (target DNA) according to the DNA editing method of the present invention. (a) shows a preferred configuration of a DNA editing vector (vector) and a target DNA (target DNA) when using TALEN as the site-specific nuclease system, and (b) shows one embodiment of a preferred configuration of a DNA editing vector (vector) and a target DNA (target DNA) when using a CRISPR-Cas system as the site-specific nuclease system.

[Fig. 2A] Fig. 2A is, among schematic diagrams showing one embodiment of the removal of a selection vector in the DNA editing method of the present invention, a schematic diagram showing combinations when a DNA editing vector and a nuclease system expression vector are introduced into a cell and the resulting modes of cell life and death.

[Fig. 2B] Fig. 2B is, among schematic diagrams showing one embodiment of the removal of a selection vector in the DNA editing method of the present invention, a schematic diagram showing the intracellular mode when a DNA editing vector and a nuclease system expression vector are introduced into a cell and editing of the target site occurs (left) and the mode in which the selection vector is removed from the cell (right).

[Fig. 3] Fig. 3 is a schematic diagram showing a preferred embodiment when the DNA editing vector according to the DNA editing method of the present invention is an all-in-one NICS vector.

[Fig. 4] Fig. 4 is a schematic diagram showing the DNA editing method constructed in Test Example 1.

[Fig. 5] Fig. 5 is a schematic diagram showing the positions where primers were designed in the PCR reaction performed in Test Example 1 (a) and an electrophoresis photograph of the amplification product after the PCR reaction (b).

[Fig. 6A] Fig. 6A is a schematic diagram showing the configuration of the plasmid "pMD20-ARS-aphVIII-sep-BamHI" used for the preparation of each vector in Test Example 2.

[Fig. 6B] Fig. 6B is a schematic diagram showing the configuration of the validation vector_NoNR (validation vector) prepared in (1) Validation Test 1 of Test Example 2 (a), and a schematic diagram showing the configuration of the vector when the validation vector_NoNR is cleaved and expresses paromomycin resistance (b).

[Fig. 7A] Fig. 7A is a graph showing the number of colonies measured in (1) Validation Test 1 of Test Example 2.

[Fig. 7B] Fig. 7B is a schematic diagram showing the positions where primers were designed in the PCR reaction performed in (1) Validation Test 1 of Test Example 2 (a) and an electrophoresis photograph of the amplification product after the PCR reaction (b).

[Fig. 8] Fig. 8 is a graph showing the number of colonies measured in (2) Validation Test 2 of Test Example 2.

[Fig. 9] Fig. 9 is a schematic diagram showing the configuration of the pNICS36 vector (or pNICS210 vector) and the configuration of the TALEN vector_PDAT1 prepared in (3) Construction of NICS Vector of Test Example 2 (a), and a schematic diagram showing the configuration of the all-in-one NICS36 vector (or all-in-one NICS210 vector) (b).

[Fig. 10] Fig. 10 is a graph showing the number of colonies measured in the (4) Cell Introduction Test of NICS Vector of Test Example 2.

[Fig. 11] Fig. 11 is a schematic diagram showing the positions where primers were designed in the PCR reaction performed in (5) PCR Analysis and Sequence Analysis of Test Example 2.

[Fig. 12] Fig. 12 is an electrophoresis photograph of the amplification product after PCR reaction performed on a paromomycin-resistant colony formed by introducing a combination of the pNICS210 vector and the TALEN vector_PDAT1 in (5) PCR Analysis and Sequence Analysis of Test Example 2.

[Fig. 13] Fig. 13 is an electrophoresis photograph of the amplification product after the PCR reaction performed on a paromomycin-resistant colony formed by introducing the all-in-one NICS210 vector in (5) PCR Analysis and Sequence Analysis of Test Example 2.

[Fig. 14] Fig. 14 is the appearance showing the respective growth statuses of strains (6 strains each) that grew on an F2N plate (F2N) but did not grow on a plate containing paromomycin (F2N+Paro) in the (6) CEN/ARS Vector Elimination Test of Test Example 2 using paromomycin-resistant colonies No. 5 and No. 8 formed by introducing the all-in-one NICS210 vector.

[Fig. 15] Fig. 15 is an electrophoresis photograph of the amplification product after a PCR reaction performed using, as a template, DNA extracted with a simple DNA extraction kit from the paromomycin-resistant colonies No. 5 and No. 8 (strains before vector elimination) formed by introducing the all-in-one NICS210 vector and each of the 6 strains derived therefrom confirmed in Fig. 14 (vector-eliminated strains), and using a primer that detects FokI.

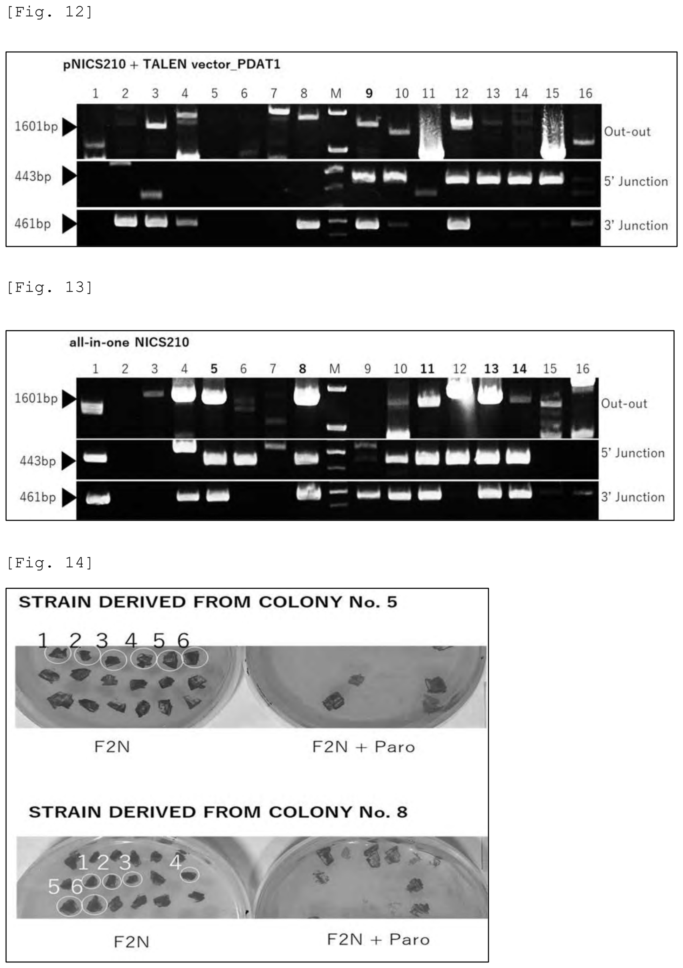

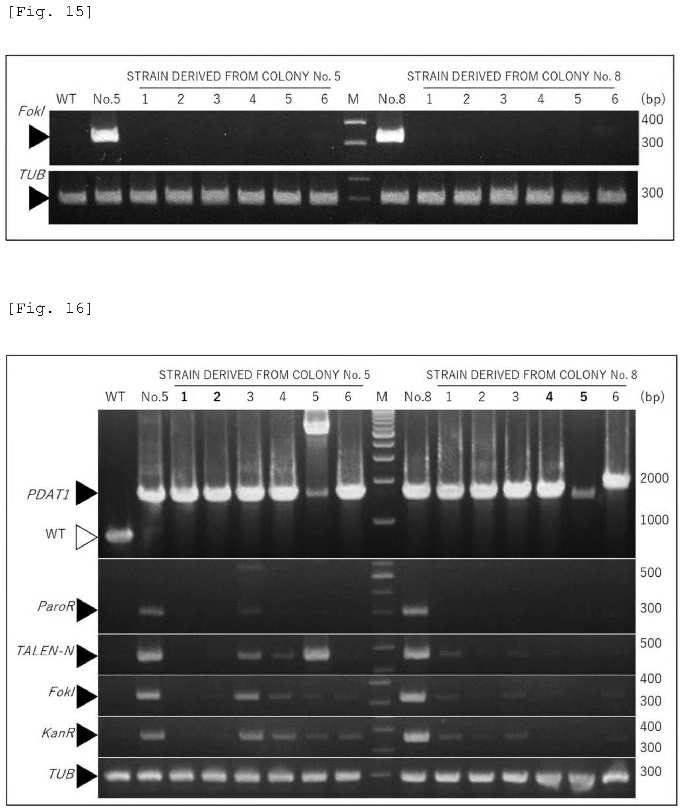

[Fig. 16] Fig. 16 is an electrophoresis photograph of the amplification product after a PCR reaction performed using, as a template, DNA extracted by a method using phenol/chloroform from the paromomycin-resistant colonies No. 5 and No. 8 (strains before vector elimination) formed by introducing the all-in-one NICS210 vector and each of the 6 strains derived therefrom confirmed in Fig. 14 (vector-eliminated strains), and using primers that detect FokI, the N-terminal domain of TALEN (TALEN-N), the paromomycin resistance gene (ParoR), the kanamycin resistance gene (KanR), and PDAT1, respectively.

[Fig. 17] Fig. 17 is a schematic diagram showing one embodiment of an application of the DNA editing method (NICS system) of the present invention.

[Fig. 18] Fig. 18 is a schematic diagram when there are two types (X, Y) of nucleotide sequences to be inserted in one embodiment of an application of the DNA editing method (NICS system) of the present invention.

[Fig. 19] Fig. 19 is a schematic diagram showing the detailed configuration of the "all-in-one NICS210 vector" prepared in (3) Construction of NICS Vector of Test Example 2.

[Fig. 20] Fig. 20 is an electrophoresis photograph of the amplification product after a PCR reaction performed on a paromomycin-resistant colony formed by introducing the all-in-one NICS210-GPAT1 vector in the (2) Cell Introduction Test of NICS Vector of Test Example 3.

[Fig. 21] Fig. 21 is the appearance showing the respective growth statuses of strains (6 strains each) that grew on an F2N plate (F2N) but did not grow on a plate containing paromomycin (F2N+Paro) in the (3) CEN/ARS Vector Elimination Test of Test Example 3 using paromomycin-resistant colonies No. 3 and No. 4 formed by introducing the all-in-one NICS210-GPAT1 vector.

[Fig. 22] Fig. 22 is an electrophoresis photograph of the amplification product after a PCR reaction performed on a paromomycin-resistant colony formed by introducing the all-in-one NICS400 vector in the (2) Cell Introduction Test of NICS Vector of Test Example 4.

[Fig. 23] Fig. 23 is a schematic diagram showing the configuration of the NICS vector and the configuration of the CRISPR/Cas9 vector prepared in (1) Construction of NICS Vector of Test Example 5 (upper panel); a schematic diagram showing the configuration of the vector when the NICS vector is cleaved and expresses puromycin resistance (lower left panel); and a schematic diagram showing the mode when insertion of a knock-in fragment (3xFlag tag sequence) occurs at the target site (lower right panel).

[Fig. 24] Fig. 24 is a graph showing the viability obtained in the (2) Validation Test of Test Example 5.

[Fig. 25] Fig. 25 is a graph showing the viability under each puromycin concentration for each combination of a NICS vector (pNICS60-PARP1 vector, pNICS192-PARP1 vector) and a CRISPR/Cas9 vector targeting PARP1 in the (3) Cell Introduction Test of NICS Vector of Test Example 5.

[Fig. 26] Fig. 26 is a graph showing the viability under each puromycin concentration for each combination of a NICS vector (pNICS192-ATP5B vector) and a CRISPR/Cas9 vector targeting ATP5B in the (3) Cell Introduction Test of NICS Vector of Test Example 5.

[Fig. 27A] Fig. 27A is an electrophoresis photograph of the amplification product after a PCR reaction performed on colonies formed under each puromycin concentration by introducing a NICS vector (pNICS60-PARP1 vector) and a CRISPR/Cas9 vector targeting PARP1 in the (2) PCR Analysis of Test Example 5.

[Fig. 27B] Fig. 27B is an electrophoresis photograph of the amplification product after a PCR reaction performed on colonies formed under each puromycin concentration by introducing a NICS vector (pNICS192-PARP1 vector) and a CRISPR/Cas9 vector targeting PARP1 in the (2) PCR Analysis of Test Example 5.

[Fig. 28A] Fig. 28A is an electrophoresis photograph of the amplification product after a PCR reaction performed on colonies formed under each puromycin concentration by introducing a NICS vector (pNICS60-ATP5B vector) and a CRISPR/Cas9 vector targeting ATP5B in the (2) PCR Analysis of Test Example 5.

[Fig. 28B] Fig. 28B is an electrophoresis photograph of the amplification product after a PCR reaction performed on colonies formed under each puromycin concentration by introducing a NICS vector (pNICS192-ATP5B vector) and a CRISPR/Cas9 vector targeting ATP5B in the (2) PCR Analysis of Test Example 5.

[Fig. 29] Fig. 29 is a graph showing the HDR efficiency in colonies formed under each puromycin concentration for each combination of a NICS vector (pNICS192-PARP1 vector) and a CRISPR/Cas9 vector targeting PARP1 in the (3) TIDER Analysis of Test Example 5.

[Description of Embodiments]

Hereinafter, the present invention will be described in detail with reference to preferred embodiments thereof. Preferred embodiments of the present invention will be described by way of example with reference to the drawings as appropriate, but the present invention is not limited thereto. In the following description and drawings, the same or corresponding elements are denoted by the same reference numerals, and redundant descriptions are omitted.

<DNA Editing Method/NICS>

The DNA editing method of the present invention is a method for inserting a desired nucleotide sequence at a target site of a target DNA in a cell, or removing the target site from the target DNA. Here, "insertion" of the desired nucleotide sequence also includes replacement of a sequence, in which the target site is removed and the desired nucleotide sequence is inserted there. The DNA editing method of the present invention includes an introduction step of introducing a vector and a site-specific nuclease system into a cell to bring them into contact with a target DNA, wherein

the vector includes

a first promoter P1, and
the following structure (1):

$$5'\text{-M1-Hv1-D-Hv2-M2-}3'... \qquad (1)$$

[in (1), M1 represents a 5' side fragment of a nucleotide sequence encoding a selection marker gene and is operably linked to the first promoter P1, Hv1 represents a nucleotide sequence homologous to a first nucleotide sequence Ht1 on the 5' side of the target site of the target DNA, D represents the desired nucleotide sequence but may be absent, Hv2 represents a nucleotide sequence homologous to a second nucleotide sequence Ht2 on the 3' side of the target site of the target DNA, M2 represents the remaining 3' side fragment of the nucleotide sequence encoding the selection marker gene, and in M1, Hv1, D, Hv2, and M2, adjacent sequences may partially overlap with each other.],

a cleavage step of generating from the vector, by the site-specific nuclease system, a fragment represented by the following structure (2):

$$5'\text{-Hv1-D-Hv2-}3'... \qquad (2)$$

and a fragment represented by the following structure (3):

5'-M2-P1-M1-3'...          (3)

and cleaving the target site or its vicinity in the target DNA,

an editing step in which the target DNA and the fragment represented by structure (2) bind depending on the homology between Ht1 and Hv1 and bind depending on the homology between Ht2 and Hv2, and the desired nucleotide sequence D is inserted at the target site or the target site is removed, and

a selection step of obtaining a selection vector in which, in the fragment represented by structure (3), the 3' end of M1 and the 5' end of M2 are ligated to obtain a selection vector that contains a functional selection marker gene operably linked to P1.

(Target DNA)

In the present invention, with a site for the purpose of inserting a desired nucleotide sequence or a nucleotide sequence for the purpose of removal as a target site, a DNA including the target site is referred to as "target DNA." The target DNA according to the present invention is a double-stranded DNA, and in order to show the correspondence with the DNA editing vector and the site-specific nuclease system described below, for convenience, it shall be assumed to have a structure in which, of at least one strand, the 5' side of the target site is a first nucleotide sequence (Ht1) and the 3' side of the target site is a second nucleotide sequence (Ht2), and which includes these.

As one embodiment of the configuration of the target DNA, a schematic diagram in the case where the site for the purpose of inserting a desired nucleotide sequence is the target site is shown in the lower part of Fig. 1A(a). In this case, in the target DNA, it is preferable that the first nucleotide sequence (Ht1) and the second nucleotide sequence (Ht2) exist adjacent to each other, but as shown in Fig. 1A(a), they may be separated by a region of several bases, preferably 1 to 10000 bases, more preferably 1 to 3000 bases, and still more preferably 1 to 1000 bases.

As one embodiment of the configuration of the target DNA, a schematic diagram in the case where the nucleotide sequence for the purpose of removal is the target site is shown in the lower part of Fig. 1B(a). In this case, as for the length of the target site (target in Fig. 1B(a)), it is 1 to 10000 bases, more preferably 1 to 3000 bases, and still more preferably 1 to 1000 bases. In this case, Ht1 is adjacent to the 5' end of the target site, and Ht2 is adjacent to the 3' end. In this specification, "base" indicating the length of a nucleotide sequence is synonymous with "nucleotide," or "bp" when it is double-stranded.

In Fig. 1A(a) and Fig. 1B(a), the target DNA according to the present invention is cleaved at the target site (arrow) by a site-specific nuclease, but the cleavage site does not have to be the target site, and may be in the vicinity thereof, and may be on the first nucleotide sequence (Ht1) or on the second nucleotide sequence (Ht2). In the present invention, by setting Ht1 and Ht2 as described above and designing a DNA editing vector including the following Hv1 and Hv2 corresponding thereto, because they bind depending on the homology between Ht1 and Hv1 and the homology between Ht2 and Hv2, it is possible to obtain a sequence in which a desired nucleotide sequence is inserted between Ht1 and Ht2, or a sequence from which the target site is removed from between Ht1 and Ht2. In the target DNA according to the present invention, as for the length between the cleavage site and the target site, although it depends on the lengths of Ht1 and Ht2, it is preferably within 400 bases, and more preferably 0 to 200 bases.

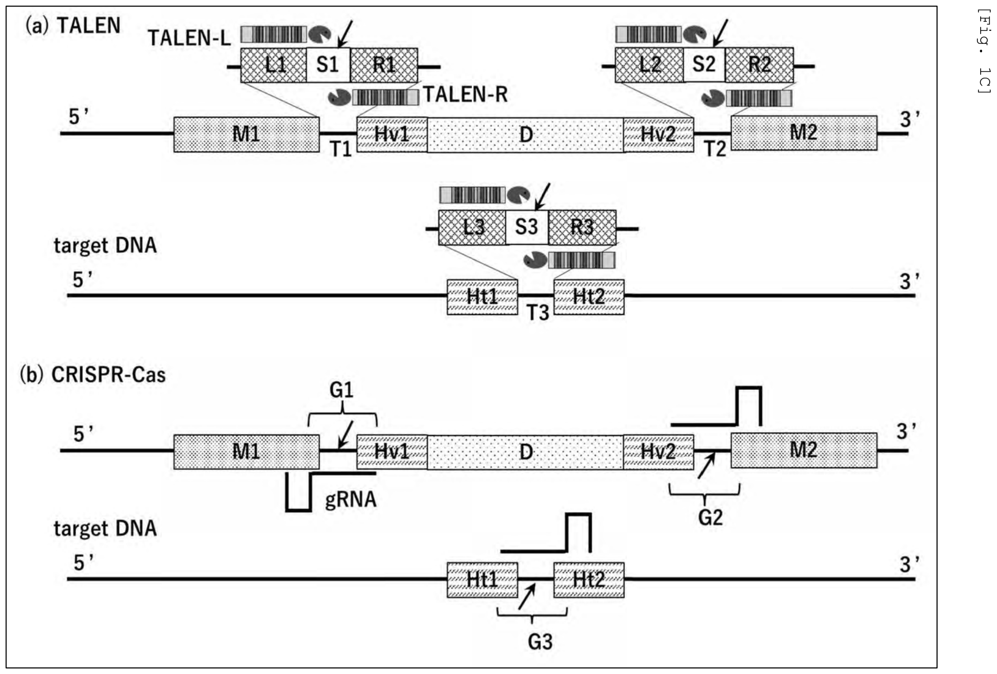

In cases where the site-specific nuclease according to the present invention is the TALEN described below, in order to show the correspondence with such TALEN, for convenience, the target DNA according to the present invention shall be assumed to have a structure in which the strand including Ht1 and Ht2 further includes a TALEN_3 binding region T3 as a region including the target site or at least partially overlapping with the target site. A schematic diagram showing one embodiment of the configuration of the target DNA when the site-specific nuclease system according to the present invention is TALEN (in the case where the site for the purpose of inserting a desired nucleotide sequence is the target site) is shown in the lower part of Fig. 1C(a). In Fig. 1C(a), T3 is sandwiched between Ht1 and Ht2, but as described above, Ht1 and Ht2 may be adjacent to each other, and part or all of T3 may overlap with a part on the 3' end side of Ht1 and/or a part on the 5' end side of Ht2. In the case where the nucleotide sequence for the purpose of removal is the target site, the length of the target site is as described above.

T3 shall, for convenience, include, adjacent in this order from the 5' end side, a fifth DNA binding domain recognition sequence or its complementary sequence L3, a spacer S3, and a sixth DNA binding domain recognition sequence or its complementary sequence R3. By designing the fifth DNA binding domain of TALEN_3 (TALE of Left-TALEN) so as to bind to L3, and the sixth DNA binding domain (TALE of Right-TALEN) so as to bind to R3, respectively, the inside of S3 is cleaved (arrow) by the nuclease activity of TALEN_3. However, as described above, the cleavage site does not have to be the target site, and may be on the first nucleotide sequence (Ht1) or on the second nucleotide sequence (Ht2). Each DNA binding domain recognition sequence is a sequence recognized by each DNA binding domain (fifth DNA binding domain, sixth DNA binding domain) of the TALEN_3 described below. Normally, because Left-TALEN and Right-TALEN bind to opposite strands from each other, L3 and R3 are in a relationship where if one is a DNA binding domain recognition sequence, the other is the complementary sequence of the DNA binding domain recognition sequence. As for the length of L3 and R3, it is each independently 10 to 30 bases, preferably 13 to 25 bases, more preferably 15 to 20 bases. As for the length of S3, it is 10 to 20 bases, preferably 12 to 19 bases, more preferably 15 to 16 bases.

Furthermore, in cases where the site-specific nuclease according to the present invention is the CRISPR-Cas system described below, in order to show the correspondence with such a CRISPR-Cas system, for convenience, the target DNA according to the present invention shall be assumed to have a structure in which at least one strand further includes a third guide RNA recognition sequence or its complementary sequence G3. A schematic diagram showing one embodiment of the configuration of the target DNA when the site-specific nuclease system according to the present invention is a CRISPR-Cas system (in the case where the site for the purpose of inserting a desired nucleotide sequence is the target site) is shown in the lower part of Fig. 1C(b). Also in Fig. 1C(b), the space between Ht1 and Ht2 is separated, but as described above, Ht1 and Ht2 may be adjacent to each other. G3 may be sandwiched between Ht1 and Ht2, or part or all of G3 may overlap with a part on the 3' end side of Ht1 and/or a part on the 5' end side of Ht2. In the case where the nucleotide sequence for the purpose of removal is the target site, the length of the target site is as described above. It is preferable that the CRISPR-Cas system cleaves (arrow) the space between Ht1 and Ht2 as a target site, and in a CRISPR-Cas system, because a site included in the guide RNA recognition sequence is cleaved by the Cas protein, G3 exists so as to include the target site or its complementary sequence.

As for the CRISPR-Cas system, for example, if it is a CRISPR-Cas9 system, by designing the third guide RNA to recognize the third guide RNA recognition sequence which is G3 or its complementary sequence, and the Cas9 protein to recognize a sequence existing on the 3' side of the complementary sequence of the third guide RNA recognition sequence as a PAM sequence, respectively, the target site is cleaved by the Cas9 protein. As for the length of the guide RNA recognition sequence or its complementary sequence, it is 12 to 50 bases, preferably 17 to 30 bases, more preferably 17 to 25 bases. The PAM sequence differs depending on the type of Cas protein, and typical PAM sequences are publicly known, but it is possible to expand the options for the target site by modifying the PAM recognition by modifying the Cas protein (for example, introducing a mutation).

The base sequence of such a target DNA is not particularly limited, and by designing the DNA editing vector and the site-specific nuclease system described below in accordance with that sequence, it can be made the target of the DNA editing method of the present invention.

Furthermore, the target DNA according to the present invention can be a DNA existing in the nucleus or a DNA existing outside the nucleus, depending on the purpose, and may be an endogenous DNA or an exogenous DNA. Examples of the endogenous DNA include genomic DNA, and examples of the exogenous DNA include, for example, DNA introduced into a cell.

(Cell)

The cell in which the target DNA exists, that is, the cell that is the subject of the DNA editing method of the present invention, is not particularly limited, and may be a eukaryotic cell or a prokaryotic cell, but is preferably a eukaryotic cell. Examples of the eukaryotic cell include animal cells (mammalian, fish, avian, reptilian, amphibian, insect cells, etc.), plant cells, algal cells, slime molds, and fungi (yeast, etc.), and examples of the prokaryotic cell include Escherichia coli, Salmonella, Bacillus subtilis, lactic acid bacteria, hyperthermophilic bacteria, actinomycetes, and archaea.

"Animal cell" includes, for example, cells constituting an animal individual, cells constituting organs and tissues extracted from an animal, and cultured cells derived from animal tissues. Specifically, examples include germ cells such as oocytes and sperm; embryonic cells at each stage (e.g., 1-cell stage embryo, 2-cell stage embryo, 4-cell stage embryo, 8-cell stage embryo, 16-cell stage embryo, morula stage embryo, etc.); stem cells such as induced pluripotent stem (iPS) cells and embryonic stem (ES) cells; and somatic cells such as fibroblasts, hematopoietic cells, neurons, muscle cells, bone cells, liver cells, pancreatic cells, brain cells, and kidney cells. As the oocyte that is the subject of the DNA editing method, oocytes before and after fertilization can be used, but post-fertilization oocytes, that is, fertilized eggs, are preferable. Particularly preferably, the fertilized egg is from a pronuclear stage embryo. The oocytes can be used after being thawed from cryopreservation.

"Plant cell" includes, for example, cells constituting a plant individual, cells constituting organs and tissues isolated from a plant, and cultured cells derived from plant tissues. Examples of plant organs and tissues include leaves, stems, apical meristems (growing points), roots, tubers, and calli.

Among these, as the cell according to the present invention, at least one selected from the group consisting of algal cells and animal cells is preferable, and as for the algal cell, it is more preferably a unicellular alga such as Nannochloropsis, Chlamydomonas, Euglena, Chlorella, Botryococcus, diatoms, coccolithophores, cyanobacteria, Cyanidioschyzon, Pseudococcomyxa, Spirulina, or Haematococcus.

(Site-Specific Nuclease System)

In the present invention, a "site-specific nuclease system" refers to a system that includes a nuclease that cleaves a DNA double strand and that is controllable so that cleavage by the nuclease occurs specifically at a target location. Examples of such a site-specific nuclease system include a system in which a nuclease is fused to a TAL effector (TALE) (TALEN), a system in which a nuclease is fused to a zinc finger (ZF), a CRISPR-Cas system, and the like, and in the present invention, a TALEN and/or a CRISPR-Cas system is preferable.

[TALEN]

TALEN (Transcription activator-like effector nuclease) is a fusion protein with the type IIS restriction enzyme FokI as a nuclease domain and TALE as a DNA binding domain; the respective DNA binding domains of a pair of TALENs (Left-TALEN and Right-TALEN) each bind to opposite strands of a target DNA, and FokI forms a dimer with each other, thereby exhibiting DNA site-specific double-strand cleavage activity (nuclease activity) and cleaving a region between the pair of DNA binding domains. A typical nucleotide sequence encoding FokI is publicly known, but it may be one that has been appropriately modified, and as such a TALEN, a publicly known one or one based thereon may be appropriately used. A method for adjusting the amino acid sequence of each DNA binding domain is publicly known, and a person skilled in the art can design a DNA binding domain that can recognize and bind to a nucleotide sequence (DNA binding domain recognition sequence) on a target DNA, and a nucleotide sequence encoding it, based on the DNA binding domain recognition sequence.

In cases where a TALEN is used in the present invention, such a TALEN shall include a TALEN_1 including a first DNA binding domain and a second DNA binding domain, a TALEN_2 including a third DNA binding domain and a fourth DNA binding domain, and a TALEN_3 including a fifth DNA binding domain and a sixth DNA binding domain.

TALEN_1 and TALEN_2 respectively recognize T1 and T2 on the DNA editing vector corresponding to each DNA binding domain and respectively cleave the DNA editing vector in S1 between L1 and R1, and S2 between L2 and R2. TALEN_3 also recognizes T3 on the target DNA and cleaves the target DNA in S3 between L3 and R3. At this time, the location where the target DNA is cleaved is the target site or its vicinity.

The first DNA binding domain, the third DNA binding domain, and the fifth DNA binding domain may be the same as each other, the second DNA binding domain, the fourth DNA binding domain, and the sixth DNA binding domain may also be the same as each other, and TALEN_1, TALEN_2, and TALEN_3 may be the same as each other. By using a common single type as the three types of TALEN, it becomes possible to cleave DNA more simply and efficiently. For example, a TALEN_3 is designed that has a fifth DNA binding domain that recognizes L3 on the 5' side of the region (T3) including the target site of the above-described target DNA, and a sixth DNA binding domain that recognizes R3 on the 3' side, and that cleaves the target DNA in S3 between L3 and R3 (Fig. 1C(a)). By designing the DNA editing vector so that the sequences of T1 and T2 are the same as the sequence of T3 in accordance with this, it is possible to use only one type of TALEN_3 as TALEN_1 and TALEN_2.

[CRISPR-Cas system]

The CRISPR-Cas system according to the present invention includes at least a Cas protein and its guide RNA as components. The guide RNA consists of a crRNA (CRISPR RNA) having a base sequence complementary to a target base sequence (guide RNA recognition sequence) and a tracrRNA (trans-activating crRNA). The crRNA further includes, on its 3' side, a base sequence capable of interacting (hybridizing) with the tracrRNA. On the other hand, because the tracrRNA includes, on its 5' side, a base sequence capable of interacting (hybridizing) with a part of the base sequence of the crRNA, the guide RNA forms a double-stranded RNA that interacts with the Cas protein by the interaction of these base sequences. For this reason, by introducing the Cas protein and its guide RNA into a cell, the guide RNA binds to a guide RNA recognition sequence of a DNA, guides the Cas protein that forms a complex with the guide RNA to the DNA, and the guided Cas protein recognizes a PAM sequence existing on the 3' side of the complementary sequence of the guide RNA recognition sequence and cleaves the target site of the DNA by its nuclease activity.

The CRISPR-Cas system may be of any class, including class 1, which forms a complex with a guide RNA and one Cas protein to cleave a nucleic acid, and class 2, which forms a complex (cascade complex) with a guide RNA and multiple Cas proteins to cleave a nucleic acid, and may be of any type, including type I, type II, type III, type IV, type V, and type VI. Among these, from the viewpoint of ease, class 2 is preferable, and a CRISPR-Cas9 system in which the Cas protein is a Cas9 protein is more preferable. The configurations of these various CRISPR-Cas systems and the typical nucleotide sequences encoding each Cas protein are all publicly known, but they may be ones that have been appropriately modified, and publicly known ones or ones based thereon may be appropriately used. For example, the Cas protein may be one that has been appropriately modified, such as by the introduction of a mutation to modify PAM sequence recognition.

Various methods are known for designing the PAM sequence and the guide RNA recognition sequence of such a Cas protein, and for example, they can be designed using E-CRISP (http://www.e-crisp.org/E-CRISP/), CRISPRdirect (http://crispr.dbcls.jp/) (The University of Tokyo), Guide RNA Target Design Tool. (https://wwws.blueheronbio.com/external/tools/gRNASrc. jsp) (Blue Heron Biotech), and the like.

In cases where a CRISPR-Cas system is used in the present invention, such a CRISPR-Cas system shall include a CRISPR-Cas system_1 including a first guide RNA, a CRISPR-Cas system_2 including a second guide RNA, and a CRISPR-Cas system_3 including a third guide RNA.

CRISPR-Cas system_1 and CRISPR-Cas system_2 respectively recognize G1 and G2 on the DNA editing vector corresponding to each guide RNA and respectively cleave the DNA editing vector on G1 and on G2. CRISPR-Cas system_3 also recognizes G3 on the target DNA and cleaves the target DNA on G3.

The first guide RNA, the second guide RNA, and the third guide RNA may be the same as each other, and CRISPR-Cas system_1, CRISPR-Cas system_2, and CRISPR-Cas system_3 may also be the same as each other. By using a common single type as the three types of CRISPR-Cas systems, it becomes possible to cleave DNA more simply and efficiently. For example, a CRISPR-Cas system_3 is designed that has a third guide RNA that recognizes a region (G3) including the target site of the above-described target DNA, and a Cas9 protein that binds thereto and recognizes a PAM sequence existing on the 3' side of the complementary sequence of the third guide RNA recognition sequence, and that cleaves the target DNA on G3 (Fig. 1C(b)). By designing the DNA editing vector so that the sequences of G1 and G2 become the same as or the complementary sequence of G3 and also include the PAM sequence or its complementary sequence in accordance with this, it is possible to use only one type of CRISPR-Cas system_3 as CRISPR-Cas system_1 and CRISPR-Cas system_2.

(DNA Editing Vector/NICS Vector)

The vector used in the DNA editing method of the present invention (referred to as "DNA editing vector" or "NICS vector" in this specification, as the case may be) is a vector including a first promoter P1 and the following structure (1):

$$5' \text{-M1-Hv1-D-Hv2-M2-3'...} \qquad (1)$$

As a preferred embodiment of the configuration of the DNA editing vector according to the DNA editing method of the present invention, Fig. 1A(a) shows an embodiment in the case where the site for the purpose of inserting a desired nucleotide sequence is the target site, that is, when the DNA editing method of the present invention is a DNA insertion method (knock-in method), and Fig. 1B(a) shows an embodiment in the case where the nucleotide sequence for the purpose of removal is the target site, that is, when the DNA editing method of the present invention is a DNA removal method (knockout method), respectively.

The vector used in the DNA editing method of the present invention preferably further includes CEN/ARS, which is an autonomously replicating sequence. CEN/ARS indicates "Centromere and autonomous replication sequence" and is a sequence related to the stability of chromosomes in budding yeast, and is widely used as an autonomously replicating sequence for vectors for budding yeast. It has been reported that a vector including this CEN/ARS is also maintained intracellularly in diatoms, unicellular algae (for example, Nannochloropsis), and the like. A vector including such CEN/ARS can be eliminated from a host cell by repeatedly culturing the host cell (NPL 3, etc.). The nucleotide sequence of such CEN/ARS is typically shown in SEQ ID NO: 1, but as long as the function as the above-mentioned CEN/ARS (such as the vector replication function in a cell) is maintained, the sequence may be appropriately modified.

Structure (1) included in the DNA editing vector according to the DNA editing method of the present invention includes M1, Hv1, D, Hv2, and M2 in this order from the 5' side.

In structure (1), M1 represents a 5' side fragment of a nucleotide sequence encoding a selection marker gene, M2 represents a 3' side fragment of the nucleotide sequence encoding the selection marker gene, and M1 and M2 are fragments in which the selection marker gene has been divided so that it can function by binding after being cleaved by the site-specific nuclease system. Here, "divided so that it can function" means any division in which, in a nucleotide sequence encoding a selection marker gene that has been divided into two, the 3' end of the 5' side fragment and the 5' end of the 3' side fragment bind, and the original selection marker gene can function (it does not function as a 5' side fragment alone or a 3' side fragment alone (before binding)), and a person skilled in the art can appropriately design it according to the type of selection marker gene. For example, preferably, M1 is the 5' side fragment of the two fragments obtained by dividing the nucleotide sequence encoding the selection marker gene, and M2 is the remaining 3' side fragment to which a nucleotide sequence is added so that its 5' end side overlaps with the sequence of several bases on the 3' end side of M1. That is, M1 and M2 are designed so that the 3' end side of M1 and the 5' end side of M2 become a nucleotide sequence that overlaps with each other (overlapping sequence). As a result, the 3' end of M1 and the 5' end of M2 bind via this overlapping sequence, that is, the protruding end on the 3' end side of M1 generated by cleavage in the cell and the protruding end on the 5' end side of M2 complementary thereto can complementarily bind, and the selection marker gene can function. The length of the nucleotide sequence (overlapping sequence) that overlaps between the 3' end side of M1 and the 5' end side of M2 at this time is, for example, preferably 40 bases or more, more preferably 40 to 500 bases, still more preferably 50 to 400 bases, further more preferably 70 to 300 bases, and particularly preferably 150 to 300 bases.

As the selection marker gene, those conventionally used in DNA editing methods can be appropriately used, and examples include drug resistance genes and reporter genes. Examples of the drug resistance genes include an ampicillin resistance gene, a zeocin resistance gene, a bleomycin resistance gene, a paromomycin resistance gene, a kanamycin resistance gene, a neomycin resistance gene, an emetine resistance gene, a spectinomycin resistance gene, a hygromycin resistance gene, a chloramphenicol resistance gene, an erythromycin resistance gene, and a puromycin resistance gene, and examples of the reporter genes include green fluorescent protein (GFP), DsRed, mCherry, mOrange, mBanana, mStrawberry, mRaspberry, and mPlum. The nucleotide sequence encoding each selection marker gene is publicly known and may be one that has been appropriately modified, as long as its function is maintained.

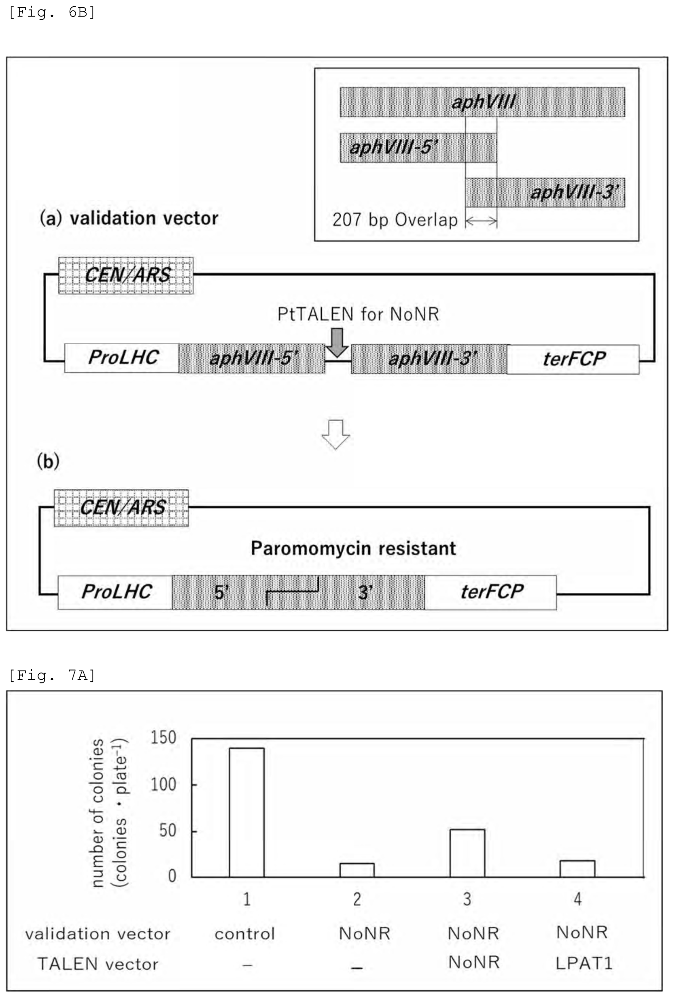

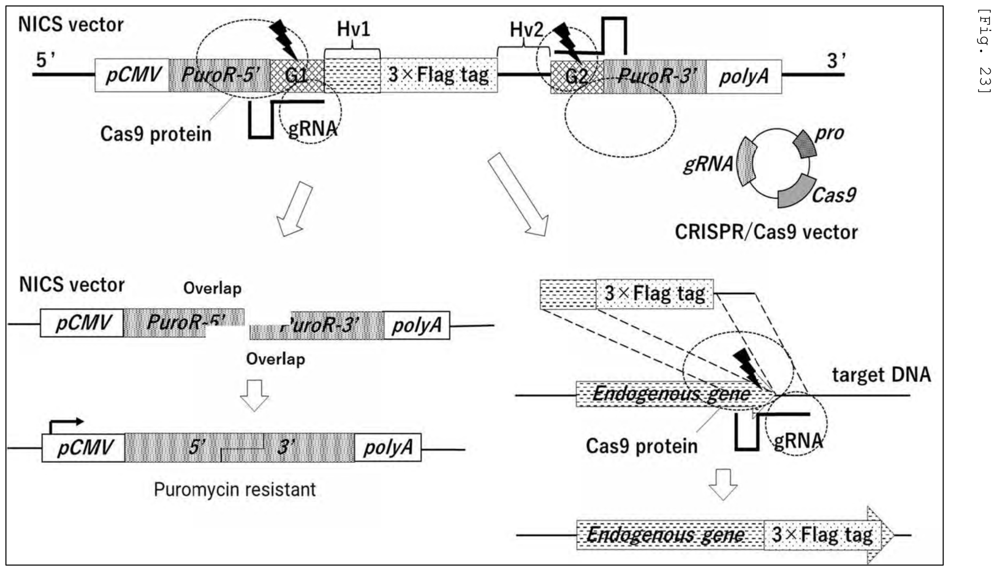

Among these, as the selection marker gene according to the present invention, from the viewpoint that the elimination of the vector from a cell can be more accurately confirmed in a medium containing a drug, a drug resistance gene is preferable, and a paromomycin resistance gene, a hygromycin resistance gene, a neomycin resistance gene, or a puromycin resistance gene is more preferable. For example, in a paromomycin resistance gene or a puromycin resistance gene, by setting the nucleotide sequence (the overlapping sequence) that overlaps between the 3' end side of M1 and the 5' end side of M2 to 50 to 300 bases (for example, 207 bases in Fig. 6B(a), and 60 bases or 192 bases in Fig. 23), the 3' end of M1 and the 5' end of M2 generated by cleavage in the cell bind via the overlapping sequence, and sufficient paromomycin resistance or puromycin resistance is expressed. The nucleotide sequences of M1 and M2 at this time are, for example, typically shown in SEQ ID NO: 3 and SEQ ID NO: 8, respectively, for a paromomycin resistance gene, and typically shown in SEQ ID NO: 43 and SEQ ID NO: 44, or SEQ ID NO: 43 and SEQ ID NO: 53, respectively, for a puromycin resistance gene, but are not particularly limited as long as they bind and function as a selection marker gene, and may also be appropriately modified, respectively.

The lengths of M1 and M2 in structure (1) (the length including the overlapping sequence if there is one) may be the same length or different lengths, and are each independently preferably 50 to 10000 bases, more preferably 100 to 5000 bases, still more preferably 200 to 3000 bases, further more preferably 200 to 2000 bases, and particularly preferably 300 to 2000 bases.

In structure (1), as for M1 and M2, it is more preferable that a stop codon is added to the 3' end of M1 and/or the 5' end of M2, more preferably to both the 3' end of M1 and the 5' end of M2. This makes it possible to more reliably suppress the expression of a selection marker gene that has not been cleaved by the site-specific nuclease system. This stop codon is normally removed from M1 and/or M2 by the intracellular repair system when the 3' end side of M1 and the 5' end side of M2 are cleaved in the cell. In particular, it is preferable that the 3' end side of M1 and the 5' end side of M2 are the overlapping sequence, because it is removed by binding via the overlapping sequence.

In structure (1), Hv1 represents a nucleotide sequence homologous to the first nucleotide sequence Ht1 on the 5' side of the target site of the target DNA, and Hv2 represents a nucleotide sequence homologous to the second nucleotide sequence Ht2 on the 3' side of the target site of the target DNA. In the present invention, as for "homologous," it is preferable that the nucleotide sequences are identical to each other (100% sequence identity), but it also includes, for example, having a sequence identity of 85% or more, preferably 90% or more, 95% or more (for example, 96% or more, 97% or more, 98% or more, 99% or more) with each other. From the viewpoint of the accuracy of DNA editing, the sequence identity between Hv1 and Hv2 is preferably 40% or less, and more preferably 20% or less.

The lengths of Hv1 and Hv2 may be the same length or different lengths, and are each independently preferably 30 to 600 bases, more preferably 40 to 600 bases, further preferably 100 to 500 bases, further more preferably 140 to 500 bases, further more preferably 150 to 450 bases, 150 to 400 bases, or 200 to 450 bases, and further more preferably 150 to 250 bases, or 200 to 300 bases. The present inventors have found that in the DNA editing method of the present invention (preferably a DNA insertion method), in particular, the DNA editing efficiency (preferably DNA insertion efficiency) is improved with a homologous sequence of a length different from that of conventional homologous recombination (HR) or microhomology-mediated end joining (MMEJ), for example, a length of 100 to 500 bases, preferably 200 to 450 bases, and further 200 to 400 bases.

When the DNA editing method of the present invention is a method for inserting a desired nucleotide sequence at a target site of a target DNA (DNA insertion method), in structure (1), D represents a desired nucleotide sequence for insertion at the target site. Such a sequence is not particularly limited, and various sequences are mentioned, for example, a promoter, terminator, enhancer, insulator, binding sequence of an expression control gene, etc., for controlling the expression of a target gene; a fluorescent protein, luminescent protein, epitope tag, etc., for observing gene expression and localization; a stop codon, etc., for gene disruption; a mutant sequence, etc., for modifying gene function; and an expression cassette for expressing an endogenous or exogenous gene. The length of D in this case is preferably 1 to 10000 bases, more preferably 5000 bases or less, still more preferably 2000 bases or less, and further more preferably 1000 bases or less. When the DNA editing method of the present invention is a method for removing a target site from a target DNA (DNA removal method), D is not included in structure (1) (that is, the length of D is 0 bases). That is, in structure (1), Hv1 and Hv2 are adjacent.

In structure (1), adjacent sequences may partially overlap with each other. In this case, the length of the overlapping nucleotide sequence between M1 and Hv1, between Hv1 and D (in the case of a DNA insertion method), between D and Hv2 (in the case of a DNA insertion method), and between Hv2 and M2 is each independently preferably 150 bases or less, more preferably 1 to 50 bases, and still more preferably 1 to 30 bases. In cases where the 3' end side of M1 and the 5' end side of M2 are the overlapping sequence, the number of bases obtained by subtracting the sum of the length of the nucleotide sequence overlapping between M1 and Hv1 and the length of the nucleotide sequence overlapping between Hv2 and M2 from the length of the overlapping sequence is preferably 40 bases or more, more preferably 50 bases or more, and still more preferably 150 bases or more.

In structure (1), between M1 and Hv1, and between Hv2 and M2, adjacent sequences may be separated by a region of several bases, preferably 1 to 500 bases, more preferably 1 to 100 bases, each independently.

The DNA editing vector according to the DNA editing method of the present invention further includes a first promoter P1, and M1 in structure (1) is operably linked to the first promoter P1. Here, "operably linked" between M1 and P1 means that the first promoter P1 and M1 in structure (1) are linked so that a selection marker gene formed by the binding of M1 and M2 can be expressed, and normally, P1 is placed upstream of M1. The first promoter P1 and other control sequences are not particularly limited, but a constitutive promoter, a tissue-specific promoter, a stage-specific promoter, an inducible promoter, a CMV promoter, etc., can be appropriately selected according to the type of cell to be introduced, etc. For example, when the cell according to the present invention is Nannochloropsis, as P1, a Nannochloropsis LDSP promoter, a Nannochloropsis LHC promoter, a Nannochloropsis VCP1 promoter, a Nannochloropsis TUB promoter, a Nannochloropsis NR promoter, etc., can be used. For example, when the cell according to the present invention is an animal cell, as P1, a CMV promoter, etc., can be used. Furthermore, the DNA editing vector according to the DNA editing method of the present invention may further appropriately include other regulatory elements (for example, a terminator, enhancer, insulator, transcription factor expression cassette, transcription factor binding sequence), etc.

In the DNA editing vector according to the DNA editing method of the present invention, the site-specific nuclease system is designed, or the cleavage site is designed in accordance with the site-specific nuclease system, such that a fragment represented by the following structure (2):

$$5'-Hv1-D-Hv2-3'... \qquad (2)$$

and a fragment represented by the following structure (3):

$$5'-M2-P1-M1-3'... \qquad (3)$$

are generated by the site-specific nuclease system according to the present invention.

For example, as the DNA editing vector according to the DNA editing method of the present invention, an embodiment when the site-specific nuclease according to the present invention is TALEN is shown in the upper part of Fig. 1C(a). When the site-specific nuclease according to the present invention is TALEN, the DNA editing vector according to the DNA editing method of the present invention includes, as structure (1), the following structure (11):

$$5'-M1-T1-Hv1-D-Hv2-T2-M2-3'... \qquad (11).$$

In structure (11), M1, Hv1, D, Hv2, and M2 are as described in the above structure (1), respectively. In structure (11), T1 is a TALEN_1 binding region including, adjacent in this order from the 5' end side, a first DNA binding domain recognition sequence or its complementary sequence L1, a spacer S1, and a second DNA binding domain recognition sequence or its complementary sequence R1, and T2 is a TALEN_2 binding region including, adjacent in this order from the 5' end side, a third DNA binding domain recognition sequence or its complementary sequence L2, a spacer S2, and a fourth DNA binding domain recognition sequence or its complementary sequence R2. However, L1 and L2 may be the same as each other, and may also be the same as L3 on the target DNA, and R1 and R2 may be the same as each other, and may also be the same as R3 on the target DNA, and the TALEN_1 binding region, the TALEN_2 binding region, and the TALEN_3 binding region may be the same as each other. From the viewpoint of the accuracy of DNA editing, the sequence identity between L1 and R1, and between L2 and R2, is preferably 40% or less, and more preferably 20% or less, respectively.

By designing the first DNA binding domain (TALE of Left-TALEN) in TALEN_1 to bind to L1, the second DNA binding domain (TALE of Right-TALEN) to bind to R1, and in TALEN_2, the third DNA binding domain (TALE of Left-TALEN) to bind to L2, and the fourth DNA binding domain (TALE of Right-TALEN) to bind to R2, respectively, the inside of S1 and the inside of S2 are cleaved (arrow in Fig. 1C(a)) by the nuclease activity of each TALEN. Normally, because Left-TALEN and Right-TALEN bind to opposite strands from each other, the relationship between L1 and R1, and the relationship between L2 and R2, are such that if one is a DNA binding domain recognition sequence, the other is the complementary sequence of the DNA binding domain recognition sequence. The lengths of L1, R1, L2, and R2 are each independently 10 to 30 bases, preferably 13 to 25 bases, more preferably 15 to 20 bases. The lengths of S1 and S2 are also each independently 10 to 20 bases, preferably 12 to 19 bases, more preferably 15 to 16 bases.

Between M1 and L1, between R1 and Hv1, between Hv2 and L2, and between R2 and M2, it is preferable that they do not overlap with each other, but they may partially overlap. In this case, the length of the overlapping nucleotide sequence is each independently preferably 15 bases or less, more preferably 15 to 10 bases, and still more preferably 1 to 10 bases. In cases where the 3' end side of M1 and the 5' end side of M2 are the overlapping sequence, the number of bases obtained by subtracting the sum of the length of the nucleotide sequence overlapping between M1 and T1 and the length of the nucleotide sequence overlapping between T2 and M2 from the length of the overlapping sequence is preferably 40 bases or more, more preferably 50 bases or more, and still more preferably 150 bases or more.

In structure (11), between M1 and L1, between R1 and Hv1, between Hv2 and L2, and between R2 and M2, they may be separated by a region of several bases, preferably 1 to 500 bases, more preferably 1 to 100 bases, each independently.

Furthermore, for example, as the DNA editing vector according to the DNA editing method of the present invention, an embodiment when the site-specific nuclease according to the present invention is the CRISPR-Cas system described below is shown in the upper part of Fig. 1C(b). When the site-specific nuclease according to the present invention is a CRISPR-Cas system, the DNA editing vector according to the DNA editing method of the present invention includes, in structure (1), a first guide RNA recognition sequence or its complementary sequence G1, and a second guide RNA recognition sequence or its complementary sequence G2, preferably the following structure (12):

$$5'\text{-M1-G1-Hv1-D-Hv2-G2-M2-3'}... \qquad (12).$$

In a CRISPR-Cas system, because a site included in a guide RNA recognition sequence is cleaved by a Cas protein, in structure (12), G1 is located between M1 and Hv1 and may overlap with at least one of M1 and Hv1, and G2 is located between M2 and Hv2 and may overlap with at least one of M2 and Hv2. In cases where the 3' end side of M1 and the 5' end side of M2 are the overlapping sequence, the number of bases obtained by subtracting the sum of the length of the nucleotide sequence overlapping between M1 and G1 and the length of the nucleotide sequence overlapping between G2 and M2 from the length of the overlapping sequence is preferably 40 bases or more, more preferably 50 bases or more, and still more preferably 150 bases or more.

When the site-specific nuclease according to the present invention is a CRISPR-Cas system, the DNA editing vector according to the DNA editing method of the present invention also includes a PAM sequence recognized by a Cas protein. The PAM sequence, for example, in a CRISPR-Cas9 system, is present several bases ahead on the 3' side of the complementary strand of each of the guide RNA recognition sequences of G1 and G2.

G1 and G2 may be the same as each other, and may also be the same as G3 on the target DNA. The lengths of G1 and G2 are also each independently 12 to 50 bases, preferably 17 to 30 bases, more preferably 17 to 25 bases.

The DNA editing vector according to the DNA editing method of the present invention may further include other components, and for example, may further include a nucleotide sequence encoding a selection marker gene for confirming the introduction of the vector. The selection marker gene in this case may be the same as those mentioned above, but it is necessary that it be different from the selection marker gene related to M1 and M2.

The DNA editing vector according to the DNA editing method of the present invention is a doublestranded, circular vector. Examples of such a vector include plasmid vectors, cosmid vectors, viral vectors, and artificial chromosome vectors, and in addition to those consisting only of DNA, they may consist of RNA, GNA, LNA, BNA, PNA, TNA, etc., or a mixture thereof. It may also be modified with components other than nucleic acids, such as sugars. The DNA editing vector according to the DNA editing method of the present invention can be prepared by a publicly known method or a method based thereon, and for example, can be obtained by a method of artificial synthesis or a method of introducing the above-mentioned respective components based on an existing vector.

(Introduction Step)

In the DNA editing method of the present invention, the DNA editing vector and the site-specific nuclease system are introduced into the cell to bring them into contact with a target DNA. The introduction of the site-specific nuclease system into the cell may be performed by introducing it in the form of a protein or RNA, by introducing it in the form of a DNA encoding the protein or RNA and expressing it in the cell, or by introducing it in the form of a vector (expression vector) that expresses the protein or RNA and expressing it in the cell. When the site-specific nuclease system is TALEN, Left-TALEN and Right-TALEN may be introduced together or separately, and when there are multiple TALENs, they may be introduced together or separately. Furthermore, when the site-specific nuclease system is a CRISPR-Cas system, the Cas protein and the guide RNA may be introduced together or separately, and when there are multiple CRISPR-Cas systems, they may be introduced together or separately. Among these, from the viewpoint of being easily introducible together with the DNA editing vector, it is preferable to introduce it in the form of a vector that expresses the site-specific nuclease system (nuclease system expression vector) and express it in the cell.

Such a nuclease system expression vector is not particularly limited, but from the viewpoint of being able to be eliminated from a cell, it preferably includes CEN/ARS as an autonomously replicating sequence. Examples of such a vector include the ARS+all-in-one vector described in NPL 3, and more specific examples in the present invention include a nuclease system expression vector including

CEN/ARS, which is an autonomously replicating sequence,
a second promoter P2, and
a nucleotide sequence Nuc that is operably linked to the second promoter P2 and encodes the site-specific nuclease system.

CEN/ARS is as described above. The second promoter P2 and other control sequences are not particularly limited, but the same ones as mentioned for the first promoter P1 are mentioned, and P1 and P2 may be the same. Here, "operably linked" between Nuc and P2 means that the second promoter P2 and Nuc are linked so that the site-specific nuclease system encoded by Nuc can be expressed, and normally, P2 is placed upstream of Nuc. In cases where there are multiple independent nucleotide sequences as Nuc (for example, a TALEN including a nucleotide sequence encoding Left-TALEN and a nucleotide sequence encoding Right-TALEN as described below, etc.), there may be multiple P2s corresponding to each nucleotide sequence, and the multiple P2s may be the same as or different from each other.

Nuc is a nucleotide sequence encoding the site-specific nuclease system, and if the site-specific nuclease system is TALEN, a combination of a nucleotide sequence encoding a fusion protein of a DNA binding domain and a nuclease domain as Left-TALEN, and a nucleotide sequence encoding a fusion protein of a DNA binding domain and a nuclease domain as Right-TALEN, is mentioned, and these may be on different vectors, but it is preferable that they are on the same vector. Furthermore, when there are multiple TALENs, they may be on different vectors, but it is preferable that they are on the same vector.

If Nuc is in a CRISPR-Cas system, a combination of a nucleotide sequence encoding a Cas protein and a nucleotide sequence encoding its guide RNA is mentioned, and these may be on different vectors, but it is preferable that they are on the same vector. Furthermore, when there are multiple CRISPR-Cas systems, they may be on different vectors, but it is preferable that they are on the same vector.

As described above, a nucleotide sequence encoding the nuclease domain (FokI) or a Cas protein is publicly known, and a nucleotide sequence encoding a DNA binding domain or a guide RNA can be appropriately designed according to the sequence near the target site of the target DNA, etc.

The nuclease system expression vector may further appropriately include other regulatory elements (for example, a terminator, an inducible promoter, a transcription factor expression cassette, a transcription factor binding sequence), etc., and may further include a nucleotide sequence encoding a selection marker gene for confirming the introduction of the vector. The selection marker gene may be the same as those mentioned for the DNA editing vector in the DNA editing method of the present invention, but it is necessary that it be at least different from the selection marker gene related to M1 and M2 of the DNA editing vector. When the DNA editing vector further includes a nucleotide sequence encoding a selection marker gene different from the selection marker gene related to M1 and M2, it may be the same as this selection marker gene as necessary, but it is preferable that it be different. Such a nuclease system expression vector can be prepared by a publicly known method or a method based thereon, and for example, can be obtained by a method of artificial synthesis or a method of introducing the above-mentioned respective components based on an existing vector.

For the introduction of the DNA editing vector and the nuclease system expression vector into a cell, from the viewpoint of further improving DNA editing efficiency, it is more preferable to introduce, as the DNA editing vector, an all-in-one vector (all-in-one NICS vector) that integrates the DNA editing vector and the nuclease system expression vector. Examples of such an all-in-one vector include
an all-in-one vector including

a first promoter P1,
structure (1),
a second promoter P2, and
a nucleotide sequence Nuc that is operably linked to the second promoter P2 and encodes the site-specific nuclease system.

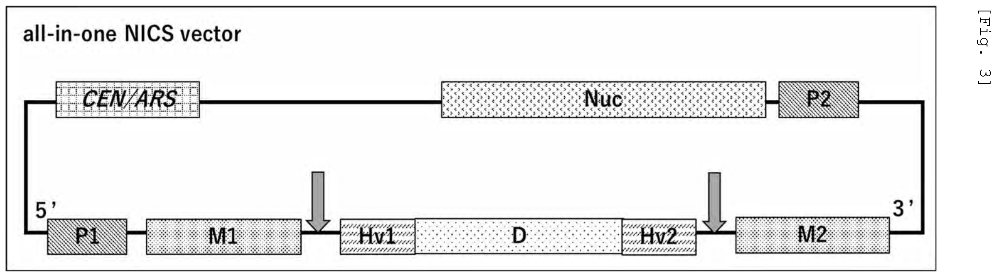

The all-in-one vector preferably further includes CEN/ARS, which is an autonomously replicating sequence. A preferred embodiment of the configuration of the all-in-one vector of the present invention is shown in Fig. 3. CEN/ARS, P1, structure (1), P2, and Nuc operably linked to P2 are as described above, respectively. When designing T1 and T2, or G1 and G2, corresponding to each site-specific nuclease system, each is based on the above-mentioned DNA editing vector.

The all-in-one vector, based on the above-mentioned DNA editing vector, may further appropriately include other regulatory elements (for example, a terminator, enhancer, insulator, transcription factor expression cassette, transcription factor binding sequence), etc., and may further include a nucleotide sequence encoding a selection marker gene for confirming the introduction of the vector. However, the selection marker gene may be the same as those mentioned above, but it is necessary that it be different from the selection marker gene related to M1 and M2. Such an all-in-one vector can be prepared by a publicly known method or a method based thereon, and for example, can be obtained by a method of artificial synthesis or a method of introducing the above-mentioned respective components based on an existing vector.

As a method for introducing the DNA editing vector (including the all-in-one vector) and the site-specific nuclease system into the cell, various publicly known methods can be used, for example, a polyethylene glycol method, an electroporation method, a method via Agrobacterium, a particle gun method, a method using laser ablation, a whisker method, etc.

(Cleavage Step)

In the DNA editing method of the present invention, by introducing the DNA editing vector and the site-specific nuclease system into a cell in the introduction step to bring them into contact with a target DNA, by the site-specific nuclease system, from the vector, a fragment represented by the following structure (2):

$$5'\text{-}Hv1\text{-}D\text{-}Hv2\text{-}3'...\qquad (2)$$

and a fragment represented by the following structure (3):

$$5'\text{-}M2\text{-}P1\text{-}M1\text{-}3'...\qquad (3)$$

(when the vector further includes CEN/ARS, 5'-M2-CEN/ARS-P1-M1-3' ... (3))
can be generated, and the target site or its vicinity in the target DNA can be cleaved.

Structure (2) includes Hv1, D, and Hv2 in this order from the 5' side, and their configurations are as described in structure (1). Structure (3) is the remaining structure obtained by excising structure (2) from the DNA editing vector, and includes M2, P1, and M1 in this order from the 5' side, and preferably includes M2, CEN/ARS, P1, and M1 in this order. Each component of structure (3) is as described for the DNA editing vector, and may further appropriately include other components. The cleavage sites in the DNA editing vector and the target DNA are as described above.

For example, by cleaving the inside of S1 and the inside of S2 with TALEN, in some cases, a part of T1 and T2 remains added to the 5' end side of Hv1 and the 3' end side of Hv2 of structure (2), respectively, but in the homology-dependent binding of Hv1 and Hv2 with Ht1 and Ht2 in the cell, it is normally removed by the intracellular repair system. By cleaving the inside of S1 and the inside of S2 with TALEN, in some cases, a part of T1 and T2 also remains added to the 3' end side of M1 and the 5' end side of M2 of structure (3), respectively, but it is removed by the intracellular repair system or by the binding of M1 and M2 in the cell (preferably, complementarity-dependent binding via the overlapping sequence). Similarly, when the inside of G1 and the inside of G2 are cleaved by a CRISPR-Cas system, even if a part of a base remains added to each end, as in the case of cleavage by TALEN, it is normally removed at the time of cleavage and/or binding by the intracellular repair system, etc.

(Editing Step)

The target DNA that has been cleaved at or near its target site in the above cleavage step and the fragment represented by structure (2) that has been excised from the DNA editing vector bind depending on the homology between Ht1 and Hv1, and bind depending on the homology between Ht2 and Hv2. In Ht1 and Hv1, and Ht2 and Hv2, "bind depending on homology" means binding by homologous sequence-dependent repair (HDR), in which each homologous sequence aligns according to its sequence and undergoes annealing and ligation, as well as removal of protruding ends, etc., by the intracellular repair system. This makes it possible to obtain a DNA-edited cell in which the desired nucleotide sequence D has been accurately inserted between Ht1 and Ht2, that is, at the target site, or in which the target site has been removed from between Ht1 and Ht2.

Confirmation of the presence or absence of insertion of the desired nucleotide sequence D or the presence or absence of removal of the target site can be performed based on conventionally known methods, and for example, a PCR method, a sequencing method, a Southern blotting method, etc., can be appropriately used, and the DNA editing method of the present invention may further include a confirmation step using these methods.

(Selection Step)

In the fragment represented by structure (3) generated by excising the fragment represented by structure (2) in the above cleavage step, M1 and M2 are divided so that they can function; the 3' end of M1 therefore binds to the 5' end of M2, whereby a selection vector containing P1 and a selection marker gene that is operably linked to P1 and is functional is obtained. Preferably, a selection vector containing CEN/ARS, P1, and a selection marker gene that is operably linked to P1 and is functional is obtained. When the all-in-one vector is used as the DNA editing vector, a selection vector is obtained that includes P1, a functional selection marker gene operably linked to P1, P2, and Nuc operably linked to P2, preferably a selection vector including CEN/ARS, P1, a functional selection marker gene operably linked to P1, P2, and Nuc operably linked to P2.

In a cell in which the selection vector has been obtained, because the selection marker gene functions, the cell can be selected and obtained using the selection marker gene as an indicator. For example, when the selection marker gene is a drug resistance gene, it can be selected based on survival under an environment containing that drug, and when the selection marker gene is a reporter gene, it can be selected based on its reporter activity (for example, fluorescence, etc.).

The fact that the selection vector was obtained also means that it is extremely likely that the above-mentioned cleavage step and editing step were also performed in the same cell, and therefore, a cell selected using the selection marker gene as an indicator can be selected as a cell in which it is extremely likely that the desired nucleotide sequence D has been inserted at the target site or the target site has been removed, and a DNA-edited cell in which the nucleotide sequence D has been inserted at the target site or the target site has been removed can be obtained with high efficiency.

(Removal of Selection Vector)

The DNA-edited cell obtained by the DNA editing method of the present invention also includes the selection vector, but if this has CEN/ARS, it is preferable because it can be eliminated from the cell by culturing the cell. Furthermore, when an expression vector is used for the introduction of a nuclease system, for example, if it is a vector having CEN/ARS like the above-mentioned nuclease system expression vector, it can be similarly eliminated from the cell.

For example, when the selection marker gene is a drug resistance gene, the above selection step is performed under conditions where a selection pressure corresponding to the selection marker gene is applied by adding the corresponding drug (selection medium), and a DNA-edited cell is obtained in which the selection marker gene functions and the desired nucleotide sequence D is inserted at the target site or the target site is removed. Then, by culturing the cell whose survival has been confirmed in the selection medium under conditions where no selection pressure is applied, that is, in a medium not containing the drug, the selection vector, etc., including CEN/ARS are eliminated. The culture period under the conditions where no selection pressure is applied is normally preferably 10 days or more, and more preferably 10 to 14 days. The fact that the selection vector, etc., have been eliminated from the cell can be confirmed, for example, by the inability to survive when a part of the strain cultured under the conditions where no selection pressure is applied is transferred again to conditions where selection pressure is applied (selection medium), or by the fact that a nucleotide sequence derived from the selection vector, etc., is not detected by PCR, etc.

Also, for example, when the selection marker gene is a reporter gene, for example, up to the above selection step, cells are selected based on the confirmation of fluorescence, etc., derived from the selection marker gene, and a DNA-edited cell is obtained in which the selection marker gene functions and the desired nucleotide sequence D is inserted at the target site or the target site is removed. Then, by culturing this, normally for 10 days or more, preferably for 10 to 14 days, the selection vector, etc., including CEN/ARS are eliminated, and thus a cell in which the fluorescence, etc., are no longer confirmed is obtained as a cell from which the selection marker, etc., have been eliminated. The fact that the selection vector, etc., have been eliminated from the cell can also be confirmed, for example, by the fact that a nucleotide sequence derived from the selection vector, etc., is not detected by PCR, etc.

In the DNA editing method of the present invention, after the introduction step, the cleavage step, the editing step, and the selection step may occur in this order, but they may occur simultaneously in parallel with each other. Hereinafter, with reference to Fig. 2A and Fig. 2B, taking as an example a method in which the nuclease system expression vector is used for the introduction of a site-specific nuclease system, a preferred embodiment up to the removal of the selection vector by the DNA editing method of the present invention will be described, but the present invention is not limited thereto.

In the DNA editing method of the present invention, first, by introducing the above-mentioned DNA editing vector and the nuclease system expression vector into a cell, a nuclease system (Nuc) is expressed from the nuclease system expression vector, and the DNA editing vector and the target DNA are cleaved (three arrows on the left of Fig. 2A: introduction step and cleavage step). As a result, a cell that includes the selection vector and in which the desired nucleotide sequence D has been inserted at the target site or the target site has been removed is obtained (in Fig. 2A and Fig. 2B, D is inserted at the target site) (upper left of Fig. 2B: editing step and selection step). This cell can be selected by the selection marker gene contained in the selection vector, and for example, when the selection marker gene is a drug resistance gene, cells into which only the DNA editing vector or the nuclease system expression vector has been introduced, or cells into which both have been introduced but cleavage has not occurred, cannot survive by culturing under selective pressure conditions with the addition of the corresponding drug (right of Fig. 2A), and therefore this can be selected and obtained as an indicator. Furthermore, by culturing this cell (for example, when the selection marker gene is a drug resistance gene, by culturing in a medium to which no drug is added), the selection vector including CEN/ARS and the nuclease system expression vector are eliminated (right of Fig. 2B), and therefore a cell that does not include foreign genes other than the desired nucleotide sequence D can be obtained (lower left of Fig. 2B).

<Cell Production Method>

The present invention is a method for producing a DNA-edited cell in which a desired nucleotide sequence is inserted at a target site of a target DNA or the target site is removed, and also provides a method for producing a DNA-edited cell, including

a step of obtaining a cell in which the desired nucleotide sequence D is inserted at the target site or the target site is removed, and which includes the selection vector, by the DNA editing method of the present invention, and
a step of selecting a DNA-edited cell in which the desired nucleotide sequence D is inserted at the target site or the target site is removed, using the selection marker gene contained in the selection vector as an indicator.

The step of obtaining a cell in which the desired nucleotide sequence D is inserted at the target site or the target site is removed, and which includes the selection vector, by the DNA editing method of the present invention, is as described from the introduction step to the selection step. The step of selecting a DNA-edited cell in which the desired nucleotide sequence D is inserted at the target site or the target site is removed, using the selection marker gene contained in the selection vector as an indicator, is also as described in the selection step.

The cell production method of the present invention preferably further includes a step of obtaining a cell including a selection vector including CEN/ARS using a vector including CEN/ARS as the DNA editing vector, further culturing the DNA-edited cell, and eliminating the selection vector from the cell. Such a step is as described in the removal of the selection vector. This makes it possible to obtain a cell that does not include foreign genes other than the inserted desired nucleotide sequence.

Therefore, the present invention also provides a method for producing a non-human individual including a cell in which a desired nucleotide sequence has been inserted into a target DNA or the target site has been removed, preferably further a non-human individual including a cell that does not include foreign genes other than the desired nucleotide sequence. This method includes a step of producing a non-human individual from a cell obtained by the cell production method of the present invention.

Examples of the non-human individual include non-human animals, plants, and algae. Examples of non-human animals include mammals (such as mice, rats, guinea pigs, hamsters, rabbits, monkeys, pigs, cows, goats, and sheep), fish, birds, reptiles, and amphibians, and insects, and examples of plants include cereals, oil crops, feed crops, fruits, and vegetables. Examples of algae include Nannochloropsis, Chlamydomonas, Euglena, Chlorella, Botryococcus, diatoms, coccolithophores, cyanobacteria, Cyanidioschyzon, Pseudococcomyxa, Spirulina, and Haematococcus.

As a method for producing a non-human individual from a cell, a publicly known method can be appropriately used. When producing a non-human individual from a cell in an animal, germ cells or pluripotent stem cells are normally used. For example, the DNA editing vector and the site-specific nuclease system are microinjected into an oocyte, and the obtained oocyte is then transplanted into the uterus of a pseudopregnant female non-human mammal, and then offspring are obtained. Transplantation can be performed with a fertilized egg at the 1-cell stage embryo, 2-cell stage embryo, 4-cell stage embryo, 8-cell stage embryo, 16-cell stage embryo, or morula stage embryo. The microinjected oocyte can be cultured under appropriate conditions until transplanted, as necessary. Transplantation and culturing of oocytes can be performed based on conventionally known methods.

In plants, it has long been known that their somatic cells have totipotency, and methods for regenerating a plant body from a plant cell have been established in various plants. Therefore, for example, by microinjecting the DNA editing vector and the site-specific nuclease system into a plant cell and regenerating a plant body from the obtained plant cell, a DNA-edited plant body in which a desired nucleotide sequence has been inserted or the target site has been removed can be obtained.

Confirmation of the presence or absence of DNA editing can be performed based on conventionally known methods, and for example, a PCR method, a sequencing method, a Southern blotting method, etc., can be appropriately used. From the obtained non-human individual, offspring or clones in which a desired DNA has been edited can also be obtained.

<DNA Editing Vector>

The present invention provides a DNA editing vector for use in the DNA editing method of the present invention. The DNA editing vector of the present invention includes

a first promoter P1, and
the following structure (1'):

$$5'-M1-Hv1'-D'-Hv2'-M2-3'... \qquad (1")$$

and is a vector in which a fragment represented by the following structure (2'):

$$5'-Hv1'-D'-Hv2'-3'... \qquad (2')$$

and a fragment represented by the following structure (3'):

$$5'-M2-P1-M1-3'... \qquad (3')$$

are generated by the site-specific nuclease system. The DNA editing vector of the present invention preferably further includes CEN/ARS, which is an autonomously replicating sequence, and in this case, a fragment represented by 5'-M2-CEN/ARS-P1-M1-3' is generated as structure (3').

The DNA editing vector of the present invention is the same as the DNA editing vector according to the DNA editing method of the present invention described above, including its preferred embodiments, except that in the above structure (1), instead of D, it is the desired nucleotide sequence or its insertion site D' (however, it may be absent in the case of a DNA removal method), and instead of Hv1 and Hv2, it is a nucleotide sequence homologous to the first nucleotide sequence Ht1 or its insertion site Hv1' and a nucleotide sequence homologous to the second nucleotide sequence Ht2 or its insertion site Hv2', respectively. In the DNA editing vector provided by the present invention, these components may be their insertion sites so that a user can design them according to a target site of interest. In this case, Hv1'-D'-Hv2' may be a single insertion site together. When Hv1', D', or Hv2' is an insertion site, structure (2') is a structure in which the structure after inserting each component into structure (1') is cleaved, that is, 5'-Hv1-D-Hv2-3'...(2) (structure (2) is as described above).

When the site-specific nuclease system is TALEN, the DNA editing vector of the present invention is the same as the DNA editing vector according to the DNA editing method of the present invention when the site-specific nuclease system is TALEN, including its preferred embodiments, except that in the above structure (11), in addition to the above structure (1'), instead of T1 and T2, it is a TALEN_1 binding region or its insertion site T1' and a TALEN_2 binding region or its insertion site T2', respectively. Also in such a DNA editing vector, these components may be their insertion sites so that a user can design them according to a target site of interest. In this case, T1'-Hv1'-D'-Hv2'-T2' may be a single insertion site together.

Furthermore, when the site-specific nuclease system is a CRISPR-Cas system, the DNA editing vector of the present invention is the same as the DNA editing vector according to the DNA editing method of the present invention when the site-specific nuclease system is a CRISPR-Cas system, including its preferred embodiments, except that in the above structure (12), in addition to the above structure (1'), instead of G1 and G2, it is a first guide RNA recognition sequence or its complementary sequence or their insertion site G1', and a second guide RNA recognition sequence or its complementary sequence or their insertion site G2', respectively. Also in such a DNA editing vector, these components may be their insertion sites so that a user can design them according to a target site of interest. In this case, G1'-Hv1'-D'-Hv2'-G2' may be a single insertion site together.

Examples of the insertion site in each of the above components include, but are not limited to, a multiple cloning site.

<DNA Editing Kit>

The present invention also provides a kit for use in the DNA editing method of the present invention. The kit of the present invention includes the DNA editing vector of the present invention and the site-specific nuclease system. The DNA editing vector and the site-specific nuclease system are as described above, including their preferred embodiments, respectively. The DNA editing vector and the site-specific nuclease system may also be in the form of the all-in-one vector described above.

The DNA editing kit of the present invention may further include one or more reagents. Examples of the reagents include, but are not limited to, a drug or substrate corresponding to the selection marker gene, a restriction enzyme and its reaction solution for inserting a target sequence into the insertion site, a dilution buffer, a reconstitution solution, a washing buffer, a control reagent (for example, a control DNA-targeting nuclease system), and a reagent for introducing a vector, etc., into a cell.

Each element included in the DNA editing kit of the present invention may be contained in separate containers, or may be contained in the same container. It may also be contained in a container for each single use, or an amount for multiple uses may be contained in one container (the user can take out the necessary amount for a single use and use it). Each element may be contained in a container in a dry form, or may be contained in a container in a form dissolved in a suitable solvent.

<Application Aspects of NICS System>

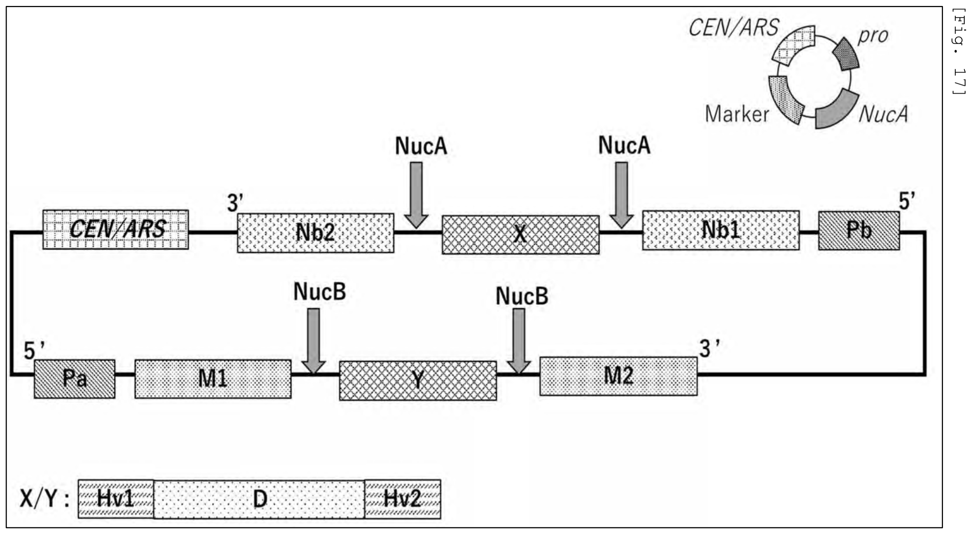

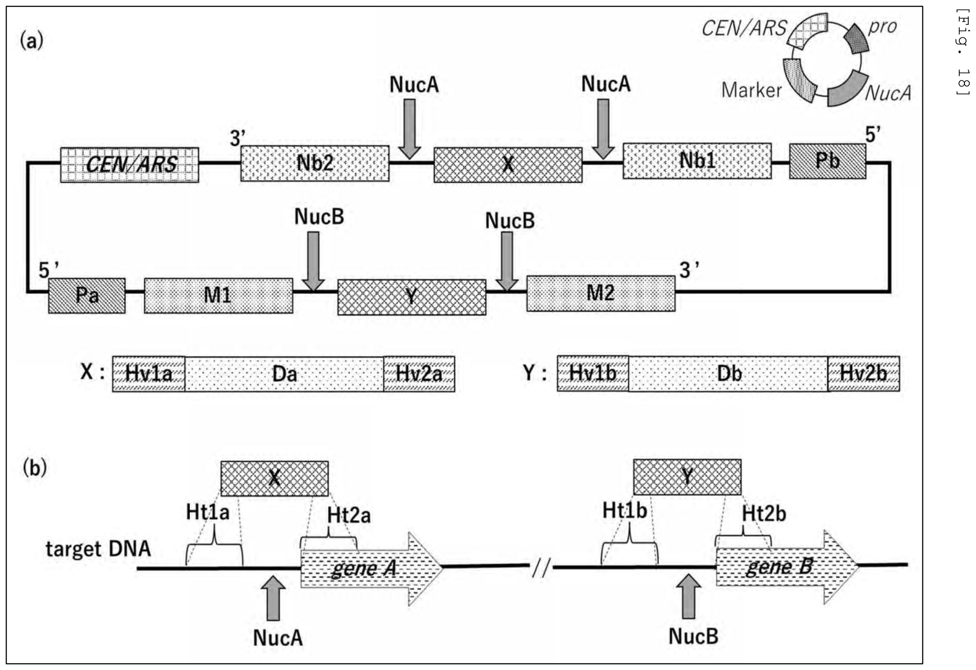

In the DNA editing method of the present invention, as described above, by a site-specific nuclease system, cleavage of a target DNA and excision of a fragment (structure (2)) including a nucleotide sequence homologous to a nucleotide sequence before and after a target site of the target DNA are performed, and at the same time, a selection vector is obtained in which a selection marker gene divided into a 5' side fragment M1 and a 3' side fragment M2 binds and functions. Therefore, the presence of such a selection vector makes it possible to obtain, with high efficiency, a DNA-edited cell in which a desired nucleotide sequence has been inserted at a target site of a target DNA or the target site has been removed. The finding of the NICS system discovered by the present inventors can be applied, and as an aspect in which such a NICS system is applied, for example, an aspect is mentioned in which the site-specific nuclease system is a combination of two or more types of nuclease systems that each cleave a different site, and multi-stage excision and binding are performed. An example of such an aspect is shown in Fig. 17 and Fig. 18.

For example, when the site-specific nuclease system is a combination of two types, a nuclease system A (NucA) and a nuclease system B (NucB), as an application of the all-in-one vector according to the present invention, an aspect is mentioned in which the configuration of the vector introduced into a cell includes

a promoter Pa,
the following structure (a): 5'-M1-Y-M2-3'...(a),
a promoter Pb,
the following structure (b): 5'-Nb1-X-Nb2-3'...(b), and
the following structure (c): 5'-Hv1-D-Hv2-3'...(c).

The all-in-one vector preferably further includes CEN/ARS, which is an autonomously replicating sequence.

Here, in structure (a), M1 and M2 are as defined in structure (1), except that M1 is operably linked to promoter Pa (note that Pa is also as defined for P1), and Y represents an arbitrary nucleotide sequence. In structure (b), Nb1 represents a 5' side fragment of a nucleotide sequence NucB encoding nuclease system B and is operably linked to promoter Pb (Pb is as defined for P2), X represents an arbitrary nucleotide sequence different from Y, and Nb2 represents the remaining 3' side fragment of the nucleotide sequence encoding nuclease system B. Furthermore, structure (c) is as defined for the above structure (2), but there may be one or two types. Structure (c) is included in the vector as X and/or Y. When there is one type of structure (c) and X or Y is other than structure (c), the nucleotide sequence of X or Y other than structure (c) is not particularly limited as long as it can divide the selection marker gene or nuclease system B so that it does not function, and may be any sequence (Fig. 17).

Nb1 and Nb2 are, similarly to M1 and M2 described above, a nucleotide sequence in which nuclease system B (NucB) has been divided so that it can function when the 3' end of Nb1 and the 5' end of Nb2 bind. Therefore, by designing nuclease system A (NucA, for example, the above-mentioned nuclease system expression vector) to excise X from structure (b), and introducing the vector and NucA into a cell, X is excised, and at the same time, the 3' end of Nb1 and the 5' end of Nb2 bind, and NucB functions. By designing the functional NucB to further excise Y from structure (a), Y is excised, and at the same time, the 3' end of M1 and the 5' end of M2 bind, forming a functional selection marker gene, and the same selection vector as described above, including the selection marker gene (and NucB), is obtained. X and/or Y, which is structure (c), is excised, and similarly to the NICS system described above, depending on the homology between Ht1 and Hv1 and the homology between Ht2 and Hv2, the nucleotide sequence D is inserted at a target site of a target DNA (Fig. 17 to Fig. 18) or a target site is removed from a target DNA (not shown). When there is one type of structure (c), the site-specific nuclease system that cleaves the target DNA may be NucA or NucB. When there are two types of structure (c), it is preferable that the nuclease that excises the structure and the nuclease that cleaves the site into which the structure is inserted or its vicinity are common (for example, in Fig. 18, NucA excises X and also cleaves the target site or its vicinity into which X is inserted, and NucB excises Y and also cleaves the target site or its vicinity into which Y is inserted).

[Examples]

Hereinafter, the present invention will be described more specifically based on Examples and Comparative Examples, but the present invention is not limited to the following Examples. Each of the following tests was conducted under the following respective conditions and methods.

1. Target Cell and its Culture Conditions 1

As one embodiment of a target cell, a strain of the unicellular alga Nannochloropsis oceanica (NIES Collection accession number: NIES-2145), preserved at the Microbial Culture Collection, National Institute for Environmental Studies, National Research and Development Agency, was used. For the medium, the F2N liquid medium shown in 2. below was used. Using an LP-200P incubator (manufactured by NK system), culturing was performed in a filter-equipped Erlenmeyer flask under the conditions of a temperature of 25°C, a light intensity of 57 $\mu$mol photons·m$^{-2}$·s$^{-1}$, and a shaking speed of 102 rpm. For normal plate culturing, the F2N plate shown in 3. below was used. For culturing with the addition of antibiotics (culturing on a selection plate), an F2N 50% seawater medium plate prepared in the same manner as the F2N plate shown in 3. below was used, except that the amount of Daigo's Artificial Seawater SP in (B) was halved (18 g) and the antibiotics were added aseptically when mixing (A) to (D). The final concentrations of the antibiotics were zeocin: 2 $\mu$g/mL, and paromomycin: 150 $\mu$g/mL.

2. Preparation of F2N Liquid Medium

The F2N liquid medium was prepared by mixing the following (A), (B), and (C). The composition for preparing 1 L of F2N liquid medium is shown below.
(A)

| | |
|---|---|
| NaNO$_3$ (75 mg/mL) | 1 mL |
| NaH$_2$PO$_4$·2H$_2$O (30 mg/mL) | 1 mL |
| Na$_2$SiO$_3$·9H$_2$O (10 mg/mL) | 1 mL |
| f/2 metal | 5 mL |
| 1 M Tris-HCl (pH 7.6) (12.11 g/100 mL) | 10 mL |
| 500 mM NH$_4$Cl (2.67 g/100 mL) | 10 mL |

The above composition was made up to 500 mL with ultrapure water and sterilized by autoclaving.
The f/2 metal used was one prepared by making up a solution mixed with the following composition to 100 mL with ultrapure water, and storing it at 4°C after autoclaving.

| | |
|---|---|
| Na$_2$·EDTA | 440 mg |
| FeCl$_3$·6H$_2$O | 316 mg |
| CoSO$_4$·7H$_2$O (1.2 mg/mL) | 1 mL |
| ZnSO$_4$·7H$_2$O (2.1 mg/mL) | 1 mL |
| MnCl$_2$·4H$_2$O (18 mg/mL) | 1 mL |
| CuSO$_4$·7H$_2$O (0.7 mg/mL) | 1 mL |
| Na$_2$MoO$_4$·7H$_2$O (0.7 mg/mL) | 1 mL. |

(B) Daigo's Artificial Seawater SP (FUJIFILM Wako Pure Chemical Corporation) 36 g
This was made up to 500 mL with ultrapure water and then sterilized by autoclaving.
(C)

| | |
|---|---|
| Thiamine HCl (1 mg/mL) | 500 $\mu$L |
| Vitamin B12 (50 $\mu$g/mL) | 50 $\mu$L |
| Biotin (50 $\mu$L/mL) | 50 $\mu$L |

Each was stored at -20°C after filter sterilization and added aseptically to the mixed solution of (A) and (B) after autoclaving.

### 3. Preparation of F2N Plate

The F2N plate (solid medium) was prepared by mixing the following (A), (B), (C), and (D), pouring it into a 9 mm diameter plastic plate, and air-cooling to solidify. The composition for preparing 1 L of F2N solid medium is shown below.

(A)

| | |
|---|---|
| $NaNO_3$ (75 mg/mL) | 1 mL |
| $NaH_2PO_4 \cdot 2H_2O$ (30 mg/mL) | 1 mL |
| $Na_2SiO_3 \cdot 9H_2O$ (10 mg/mL) | 1 mL |
| f/2 metal | 5 mL |
| 1 M Tris-HCl (pH 7.6) (12.11 g/100 mL) | 10 mL |
| 500 mM $NH_4Cl$ (2.67 g/100 mL) | 10 mL |

The above composition was made up to 250 mL with ultrapure water and sterilized by autoclaving. The f/2 metal is as shown in 2. above.
(B) Daigo's Artificial Seawater SP (FUJIFILM Wako Pure Chemical Corporation) 36 g
This was made up to 450 mL with ultrapure water and then sterilized by autoclaving.
(C)

| | |
|---|---|
| Thiamine HCl (1 mg/mL) | 500 $\mu$L |
| Vitamin B12 (50 $\mu$g/mL) | 50 $\mu$L |
| Biotin (50 $\mu$L/mL) | 50 $\mu$L |

Each was stored at -20°C after filter sterilization and added aseptically to the mixed solution of (A), (B), and (D) after autoclaving.
(D) Agar 8 g
This was suspended by adding 300 mL of ultrapure water and sterilized by autoclaving.

### 4. Cell Count of Nannochloropsis

The culture medium of Nannochloropsis was collected, 2 $\mu$L was added to a bacteria counter counting chamber (SLGC) using a pipette, and the number of cells was counted by observation using an ECLIPSE E100 microscope (manufactured by Nikon). The measurement was performed twice, and the average value was calculated as the cell count.

### 5. Electroporation Method

For electroporation, the conventional method of 5-2. below and the new method of 5-3. below were used. The method of 5-3. does not require a carrier DNA using salmon sperm DNA, which is a foreign gene, unlike the conventional method. Pre-culture 5-1. and plating 5-4. are common to both methods.

### 5-1. Pre-culture

The Nannochloropsis strain was cultured for about 7 to 10 days under the normal culture conditions of 1. above, subcultured into 250 mL of F2N liquid medium so that the cell concentration became $2.5 \times 10^6$ cells/mL, and further cultured.

### 5-2. Electroporation Using Gene Pulser Xcell (manufactured by BIORAD)

For the Nannochloropsis strain cultured for about 4 to 6 days after the pre-culture, the cell concentration was measured by cell count. 200 mL of the culture medium was collected into four 50 mL Falcon tubes and centrifuged at 4°C, 4,900 rpm for 7 minutes. The supernatant was removed, 37.5 mL of ice-cold 375 mM sorbitol was added to each tube and suspended, centrifuged at 4°C, 4,900 rpm for 7 minutes, and the supernatant was removed. This operation was repeated twice for washing, and then the cells were suspended in 375 mM sorbitol so that the final concentration of cells became $1.5 \times 10^9$ cells/mL. 3 $\mu$L of salmon sperm DNA (10 mg/mL) as a carrier DNA was incubated at 95°C for 1 minute and rapidly cooled on ice. Thereafter, the DNA to be introduced (each vector, knock-in fragment, etc.) and 3 $\mu$L of carrier DNA were mixed, placed in a Gene Pulser Cuvette 0.2 cm (manufactured by BIORAD), 150 μL of the solution in which the cells were suspended was added, and the mixture was cooled on ice for 5 minutes or more. The moisture on the cuvette was wiped off, and electroporation was performed under the following conditions:

Voltage: 2200 V, Capacitance: 50 μF
Resistance: 600 Ω
Cuvette length: 2 mm.

After electroporation, the entire volume of the solution was transferred as quickly as possible to a 15 mL Falcon tube containing 5 mL of F2N liquid medium. The lid of the Falcon tube was loosened and fixed with surgical tape, a double layer of paper towels was wrapped around the side to create low-light conditions, and recovery culturing was performed with shaking.

5-3. Electroporation Using ELEPO21 (manufactured by Nepa Gene)

In the same manner as 5-2. above, the Nannochloropsis strain cultured for about 4 to 6 days after the pre-culture was collected, washed twice, then the cell suspension was combined into two centrifuge tubes, and the cells were washed twice more with 40 mL each of 375 mM sorbitol to remove the salt from the medium. Thereafter, the cells were suspended in ice-cold 375 mM sorbitol so that the final concentration of cells became $1.0 \times 10^{10}$ cells/mL. 2 μL (or 1 μL) (total amount) of the DNA to be introduced, 38 μL of the solution in which the cells were suspended, and 40 μL of 375 mM sorbitol were suspended, allowed to stand at room temperature for 10 minutes, then transferred to a 1 mm gap cuvette and cooled on ice for 5 minutes or more. The moisture on the ice-cold cuvette was wiped off, the corner of the cuvette was lightly tapped on the lab bench to remove air bubbles, and it was then set in a chamber and electroporation was performed. The electroporation was performed under the following conditions:

Poring pulse:
Voltage: 1750 V, Pulse width: 3.5 ms, Pulse interval: 50 ms, Number of times: 1 time, Polarity: +
Transfer pulse:
Voltage: 100 V, Pulse width: 50 ms, Pulse interval: 50 ms, Number of times: 3 times, Polarity: +/-.

After electroporation, recovery culturing was performed with the same operation as in 5-2. above.

5-4. Plating

After performing recovery culturing for 2 to 4 days, the paper towels and tape were removed from the 15 mL Falcon tube in which recovery culturing was performed, and it was centrifuged at 2,000 rpm for 5 minutes, and the supernatant was removed. It was suspended in 2 mL of Top Agar that had been heated in a microwave to melt and then cooled to about 40°C, and spread on an F2N 50% seawater medium plate to which antibiotics corresponding to the introduced DNA had been added; it was then dried for about 30 minutes, and the Agar was solidified. The plate was sealed by wrapping it with surgical tape, and static culturing was performed using an incubator to form colonies. As the Top Agar, F2N liquid medium containing 0.4% agar was used.

6. Extraction of Genomic DNA

For the extraction of genomic DNA from cells (Nannochloropsis), the method of 6-1. below or the method of 6-2. below was used.

6-1. Method Using Phenol/Chloroform

First, cells were cultured in a 12-well plate with 2 mL of F2N liquid medium for about 2 weeks, and the cells were collected by centrifuging the culture medium at 20°C, 4,900 rpm for 5 minutes. 500 μL of TEN buffer was added to suspend the pellet, centrifuged at 20°C, 4,900 rpm for 5 minutes, and the supernatant was removed with a pipette. The pellet was suspended in 150 μL of ice-cold sterile ultrapure water, and immediately 350 μL of SDS-EB buffer was added and mixed. Furthermore, 500 μL of a phenol/chloroform solution mixed in a 1:1 ratio was added and vigorously mixed, and centrifuged at room temperature, 14,500 rpm for 10 minutes. The upper layer was transferred to a new 1.5 mL tube, 500 μL of phenol/chloroform solution was added again and mixed, and centrifuged at room temperature, 14,500 rpm for 10 minutes. The upper layer was transferred to a new 1.5 mL tube, 50 μL of 1.82 M NaCl and 500 μL of isopropanol were added and gently mixed, centrifuged at room temperature, 14,500 rpm for 15 minutes, and the supernatant was removed. The pellet was gently suspended in 200 μL of TE buffer, 20 μL of 1.82 M NaCl and 550 mL of 100% ethanol were added and mixed, centrifuged at 20°C, 14,500 rpm for 15 minutes, and the supernatant was removed. 200 μL of 70% ethanol was added, centrifuged at room temperature, 14,500 rpm for 10 minutes, the supernatant was removed with a pipette, and vacuum-dried for 10 minutes. Thereafter, 40 μL of TE buffer was added, heated at 55°C for 10 minutes, air-cooled to room temperature, and then allowed to stand at 4°C overnight to dissolve the genomic DNA in the TE buffer. The concentration of the genomic DNA was measured, diluted to 10 ng/μL with sterile water, and used as the template DNA for PCR.

(TEN buffer)

| 1 M Tris-HCl | 1 mL |
| 500 mM EDTA | 2 mL |
| NaCl | 0.8766 g |

Made up to 100 mL with ultrapure water and sterilized by autoclaving.

(SDS-EB buffer)

| 1 M Tris-HCl | 10 mL |
| 500 mM EDTA | 8 mL |
| NaCl | 2.3376 g |
| SDS | 2.0 g |

Made up to 100 mL with ultrapure water and sterilized by autoclaving.

(1.82 M NaCl)

| NaCl | 5.3 g |

Made up to 50 mL with ultrapure water and sterilized by autoclaving.

(TE buffer)

| 1 M Tris-HCl (pH 8.0) | 1 mL |
| 0.5 M EDTA (pH 8.0) | 0.2 mL |

Made up to 100 mL with ultrapure water and sterilized by autoclaving.

6-2. Method Using a Simple DNA Extraction Kit

For the extraction of genomic DNA when simply examining many samples, a simple DNA extraction kit (KANEKA Simple DNA Extraction Kit version 2 (manufactured by KANEKA CORPORATION)) was used. That is, a colony of cells was suspended in 10 μL of Reagent A and incubated at 98°C for 8 minutes. Thereafter, it was allowed to cool to room temperature, 1.4 μL of Reagent B was added, and it was stirred by pipetting. The obtained crude extract was diluted 1/10 with sterile water and used as the template DNA for PCR.

7. PCR Reaction

The PCR reaction was performed using a reaction solution with the following composition, and the reaction was scaled up as necessary. The PCR reaction was also performed using a thermal cycler with the following reaction cycle.

(Reaction Solution)

| | |
|---|---|
| KOD FX Neo buffer (manufactured by TOYOBO) | 5 μL |
| 2 mM dNTPs | 2 μL |
| Sterile ultrapure water | 0.4 μL |
| 10 mM primer solution | 0.4 μL |
| each | |
| Template DNA | 2 μL |
| KOD FX Neo (manufactured by TOYOBO) | 0.2 μL |

(Reaction Cycle)

| | |
|---|---|
| 94°C | 2 min |
| ↓ | |
| 98°C | 10 sec |
| 68°C 1 min/kb | (1 min per 1 kb of amplification product) |
| 30 cycles | |
| ↓ | |
| 68°C | 1 min/kb + 1 min |
| 4°C | ∞. |

8. Agarose Gel Electrophoresis

50×TAE was diluted with ultrapure water to prepare 1×TAE. 1 g/100 mL or 3 g/100 mL of Agarose S (manufactured by Nippon Gene) was added to 1×TAE, heated in a microwave to dissolve, and poured into a gel maker with a comb set to prepare a 1% or 3% agarose gel. A 3% agarose gel was used when the band size of the DNA to be electrophoresed was 1 kb or less. 6×loading buffer (manufactured by New England Biolabs) was added to the electrophoresis sample, applied to a well, and electrophoresed at 100 V until it reached about 70% of the gel. 4 μL of ethidium bromide was added to ion-exchanged water, the gel after electrophoresis was immersed in it and stained by shaking for about 30 minutes, and the electrophoresis pattern was observed using a transilluminator.

(50×TAE)

| | |
|---|---|
| Tris | 48.4 g |
| 0.5 M EDTA | 20 mL |
| Acetic acid | 11.42 mL |

Made up to 200 mL with ultrapure water.

9. Sequencing

First, a reaction mix for sequencing was prepared with the following composition:

| | |
|---|---|
| 5×Sequence Buffer (manufactured by Thermo Fisher Scientific) | 2 μL |
| 10 mM primer | 0.32 μL each |
| Template DNA | 100 ng/kb, |

and the volume was adjusted to 9 μL by adding sterile ultrapure water. The prepared reaction mix for sequencing was heated at 95°C for 5 minutes, rapidly cooled on ice for 5 minutes, then 1 μL of BigDye terminator (manufactured by Thermo Fisher Scientific) was added, and the following reaction was performed using a thermal cycler:

| | |
|---|---|
| 96°C | 2 min |
| ↓ | |
| 96°C | 10 sec |
| 50°C | 5 sec |
| 60°C | 4 min |
| 30 cycles | |
| ↓ | |
| 4°C | ∞. |

For the primers, a primer designed based on the nucleotide sequence upstream of PDAT1 of Nannochloropsis (SEQ ID NO: 26 indicating the nucleotide sequence) and a primer designed based on the nucleotide sequence downstream of PDAT1 of Nannochloropsis (SEQ ID NO: 27 indicating the nucleotide sequence) were used.

After the reaction was completed, 1 μL of 1.5 M sodium acetate (pH 5.0), 1 μL of 0.25 M EDTA (pH 8.0), and 40 μL of 100% ethanol were added to 10 μL of the reaction product and mixed, and allowed to stand at room temperature for 15 minutes. After centrifuging at 20°C, 14,500 rpm for 15 minutes, the supernatant was removed. 100 μL of 70% ethanol was added, centrifuged at 20°C, 14,500 rpm for 15 minutes, then the supernatant was removed and vacuum-dried for 10 minutes. 20 μL of HiDi formamide was added to the dried sample to dissolve the pellet, heated at 95°C for 2 minutes, and rapidly cooled on ice for 5 minutes. The sample was transferred to a sequencing plate, a plate sealing mat was set, and sequencing was performed using a SeqStudioTM Genetic Analyzer (manufactured by Thermo Fisher Scientific).

10. Elimination of CEN/ARS Vector

Colonies grown on a selection plate were collected on an F2N plate (without antibiotics) to serve as a master plate. They were subcultured from the master plate into 30 mL of F2N liquid medium and cultured for about 1 to 2 weeks, then subcultured into new F2N liquid medium so that the cell concentration became $1 \times 10^6$ cells/mL and further cultured for 2 weeks; the culture medium was diluted by adding F2N medium so that the concentration became 3000 cells/plate, and spread on an F2N plate. Elimination candidate colonies formed on the F2N plate were seeded on both an F2N plate and an F2N 50% seawater medium plate containing antibiotics, and strains that showed no growth on the plate containing antibiotics were obtained as strains from which the vector including CEN/ARS had been eliminated.

11. DNA Extraction from Agarose Gel

For DNA extraction from the agarose gel of 8. above, the Wizard(R) SV Gel and PCR Clean-Up System (manufactured by Promega) was used. From the agarose gel after electrophoresis of 8. above, a gel piece including the band of interest was cut out with a razor blade and collected using a WSE-5400 Printgraph Classic (manufactured by ATTO). 100 μL of Membrane Binding Solution (manufactured by Promega) was added per 100 mg of gel piece, and finally another 100 μL was added. It was heated at 55°C using a block incubator to completely dissolve the gel piece, and added to a column. It was centrifuged at room temperature, 12,000 rpm for 2 minutes, the flow-through was removed from the collection tube, 750 μL of Membrane Wash Solution (manufactured by Promega) was added, and it was washed by centrifuging at room temperature, 12,000 rpm for 2 minutes. The column was washed again with 500 μL of Membrane Wash Solution. The Membrane Wash Solution was removed from the column by centrifuging at room temperature, 12,000 rpm for 2 minutes, the column was set in a new 1.5 mL tube, 30 μL of Nuclease-Free Water (manufactured by Promega) was added and allowed to stand for 1 minute, then eluted by centrifuging at 20°C, 12,000 rpm for 2 minutes. The concentration of the extracted DNA solution was measured using a Nanodrop (manufactured by Thermo Fisher Scientific) and used as the template DNA for sequencing in 9. above.

12. Target Cell and its Culture Conditions 2

As another embodiment of a target cell, HEK293T (human embryonic kidney cells) were used. The following cell experiments were operated in a clean bench, and before work, the instruments to be used (electric pipettor, pipettes, plates, etc.) were placed in the clean bench and UV-treated for 15 minutes or more.

12-1. Preparation of Complete Medium

To 500 mL of DMEM (High Glucose) with L-Glutamine and Phenol Red, 5.5 mL of $10\times$ Non-Essential Amino Acid (NEAA), 5.5 mL of Penicillin-Streptomycin (ST-PN), and 55 mL of Fetal Bovine Serum (FBS) that had been heat-inactivated at 56°C for 30 minutes were added and mixed, and the mixture stored at 4°C was used as the following complete medium.

12-2. Culturing of HEK293T

From a 100 mm plate in which HEK293T were being cultured, the medium was removed with an aspirator, 10 mL of the complete medium prepared in 12-1. above was added, and the cells were detached from the plate by pipetting with an electric pipettor, and cell clumps were broken up to obtain a cell suspension. 10 mL of the cell suspension was added to a 100 mm plate in which the complete medium had been placed in advance. The plate was shaken to make the cells uniform, and incubated under the conditions of 37°C and 5% $CO_2$. This operation was performed once every 2 to 3 days to adjust so that the cells in the plate did not become overgrown.

12-3. Introduction of Vector into Cultured Cells (Lipofection)

First, using each vector solution prepared at 200 ng or 100 ng/$\mu$L, the vectors were mixed as necessary, and prepared with sterile ultrapure water so that the total concentration of the introduced vectors became 100 ng/6 $\mu$L or 200 ng/6 $\mu$L. 25 $\mu$L of D-MEM was added to each well of a 96-well plate, and then 6 $\mu$L of the prepared vector was added. Furthermore, a mixture of 25 $\mu$L of D-MEM and 0.7 $\mu$L of Lipofectamine LTX (manufactured by Thermo Fisher Scientific) in a 1.5 mL tube was added to each well and allowed to stand at room temperature for 30 minutes.

Also, from a 100 mm plate in which HEK293T cells were being cultured in complete medium, the medium was removed with an aspirator, 3 mL of TrypLETM Express was added, and it was incubated for 30 seconds. To this, 7 mL of complete medium was added, the cells were suspended using an electric pipettor, and the entire volume was transferred to a 50 mL tube. It was centrifuged at 20°C, 1000 rpm for 3 minutes, the supernatant was removed with an aspirator, and the cells were resuspended in 3 mL of complete medium. The number of cells was counted using a Luna automated cell counter (manufactured by Logos Biosystems), and diluted by adding complete medium so that the concentration became $1.0\times10^4$ cells/mL. 100 $\mu$L of the diluted cell suspension was added to each well prepared above, and cultured for 24 hours under the conditions of 37°C and 5% $CO_2$.

12-4. Selection of Vector-Introduced Cells by Antibiotics

After 24 hours of culturing in 12-3. above, cells into which a GFP expression vector (pMAX_GFP) had been introduced were observed under a microscope to confirm that Lipofection had been performed, and then cell selection was carried out. First, the medium being cultured in a 96-well plate was removed with an aspirator, 150 $\mu$L of complete medium adjusted to an antibiotic (puromycin) concentration of 0 to 20 $\mu$g/mL was added to each well, and cultured for 48 hours under the conditions of 37°C and 5% $CO_2$. After culturing (72 hours after Lipofection), the cells were collected and Cell Count was performed by the method of 12-5. below. Note that the medium was not changed during the 48 hours from the addition of the antibiotic until the cells were collected.

12-5. Cell Count of HEK293T

Before collecting the cells, cells into which pMAX_GFP had been introduced were observed under a microscope to confirm that the expression of pMAX_GFP decreased in a manner dependent on the antibiotic (puromycin) concentration. Then, the medium being cultured in a 96-well plate was transferred to separate PCR tubes, 50 $\mu$L of TrypLE Express (manufactured by Thermo Fisher Scientific) was added to each well, and incubated at 37°C for 5 minutes. The cells were suspended with a multipipettor using the medium that had been transferred to the PCR tubes, and the suspension was collected again in the PCR tubes. Then, 10 $\mu$L of the cell suspension collected in the PCR tubes was taken out, the number of cells was counted using a Luna automated cell counter (manufactured by Logos Biosystems), and the Viability (survival rate: number of cells after 48 hours of culturing in 12-4. (after antibiotic addition) / number of cells after 24 hours of culturing in 12-3. (before antibiotic addition) × 100 [%]) was calculated.

13. Genotyping

13-1. Genome Extraction and Purification

After 12-4. above, the cell suspension collected in 12-5. was centrifuged at 13,000 rpm for 3 minutes, and the medium (supernatant) was removed. Then, 150 μL of PBS(-) was added, centrifuged at 13,000 rpm for 3 minutes to remove the supernatant, and further 150 μL of PBS(-) was added and centrifuged again at 13,000 rpm for 3 minutes to remove the supernatant. A mixture of 20 μL of Lysis Buffer and 0.8 μL of Protein Degrader was added to each PCR tube, reacted at 68°C for 15 minutes and then at 95°C for 10 minutes, and stored at 4°C. Thereafter, it was transferred to -20°C and used as the following PCR template.

13-2. PCR Amplification of Inserted Sequence

After genome extraction in 13-1. above, to confirm whether a desired knock-in fragment (3×Flag sequence) was inserted at the target gene locus, forward and reverse primers were designed outside the nucleotide sequences (Ht1, Ht2) at both ends of the target site, respectively, and out-out PCR was performed. The primers were designed based on the nucleotide sequence encoding each target gene and its vicinity using Primer3plus (https://www.primer3plus.com) and NCBI BLAST (https://blast.ncbi.nlm.nih.gov/Blast.cgi?PROGRAM=blas tn&PAGE_TYPE=BlastSearch&LINK_LOC=-blasthome). As the PCR enzyme, PrimeSTAR MAX DNA Polymerase (manufactured by Takara Bio Inc.) was used for ATP5B, and PrimeSTAR GXL DNA Polymerase (manufactured by Takara Bio Inc.) was used for PARP1.

[Method using PrimeSTAR MAX DNA Polymerase]

0.2 μL of the PCR template prepared in 13-1. above, 5.0 μL of 2× PrimeSTAR MAX Premix (manufactured by Takara Bio Inc.), 0.3 μL of 10 μM forward primer, 0.3 μL of 10 μM reverse primer, and 4.2 μL of sterile ultrapure water were mixed. Using a thermal cycler, after preheating at 94°C for 2 minutes, a cycle of thermal denaturation at 98°C for 10 seconds, annealing at 66°C for 5 seconds, and extension reaction at 72°C for 5 seconds was performed for 35 to 40 cycles, and then the mixture was stored at 4°C. The PCR product was subjected to agarose gel electrophoresis by the method of 8. above to confirm a band of the length of interest including the knock-in sequence.

[Method using PrimeSTAR GXL DNA Polymerase]

1.25 μL of the PCR template prepared in 13-1. above, 0.5 μL of PrimeSTAR GXL DNA Polymerase (manufactured by Takara Bio Inc.), 5.0 μL of 5× PrimeSTAR GXL Buffer (manufactured by Takara Bio Inc.), 2.0 μL of 2.5 mM each dNTP mixture, 0.75 μL of 10 uM forward primer, 0.75 μL of 10 μM reverse primer, and 14.75 μL of sterile ultrapure water were mixed. Using a thermal cycler, after preheating at 94°C for 2 minutes, a cycle of thermal denaturation at 98°C for 10 seconds, annealing at 70°C for 15 seconds, and extension reaction at 68°C for 5 seconds was performed for 40 cycles, and then the mixture was stored at 4°C. The PCR product was subjected to agarose gel electrophoresis by the method of 8. above to confirm a band of the length of interest including the knock-in sequence.

14. TIDER Analysis

As a Reference for performing TIDER (Tracking of Insertion, DEletions and Recombination events) analysis, a TA vector was prepared in which a desired knock-in fragment (3×Flag sequence) including homologous sequences (Hv1, Hv2) was inserted.

14-1. TA Cloning

9 μL of the PCR product of 13-2. above and 1 μL of 10× A-attachment mix (TArget Clone -Plus-, manufactured by TOYOBO) were mixed and incubated at 60°C for 30 minutes using a thermal cycler. 3 μL of the solution after incubation, 5 μL of 2× Ligation Buffer (TArget Clone -Plus-, manufactured by TOYOBO), 1 μL of 50 ng/μL pTA2 vector (TArget Clone -Plus-, manufactured by TOYOBO), and 1 μL of T4 DNA Ligase (TArget Clone -Plus-, manufactured by TOYOBO) were mixed in a PCR tube. After incubating at 16°C for 30 minutes using a thermal cycler, 3 μL of this reaction solution was mixed with 30 μL of XL10-Gold (manufactured by Agilent) and allowed to stand on ice for 10 minutes. After incubating at 42°C for 30 seconds using a constant temperature bath, it was allowed to stand on ice for 2 minutes or more. This was spread on an LB+Amp plate on the surface of which a mixture of 20 μL of 5% X-gal, 10 μL of 0.1 M IPTG, and 70 μL of SOC had been spread. After incubating at 37°C for about 16 hours, white colonies that could no longer degrade X-gal were picked, and the introduction of the vector was confirmed by colony PCR. Colonies in which the introduction of the vector was confirmed were subjected to small culture, and each plasmid (TA cloning vector) was extracted by miniprep.

14-2. Sequencing, TIDER analysis

Using the TA cloning vector obtained in 14-1. above as a template, sequence analysis was performed by the method of 9. above using a vector primer, and by TIDER (https://tide.nki.nl/), with the parameter settings of Alignment Window (bp): 69, Decomposition Window (bp): 70 to 94, and Indel size range: 5 (in the case of PARP1), the HDR efficiency (HDR efficiency [%]) was determined.

<Test Example 1> Insertion of Knock-in Fragment (Comparative Example)

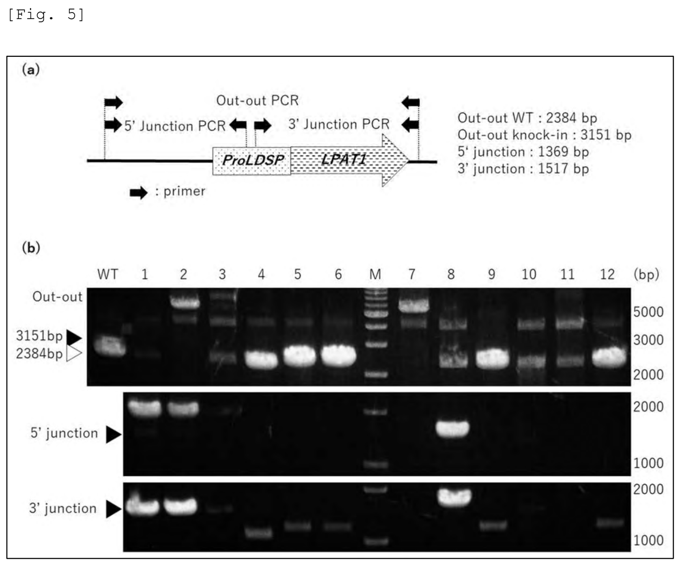

First, the method shown in Fig. 4 was designed, and an attempt was made to insert, upstream of LPAT1, which is a membrane lipid synthesis-related gene (target DNA) of the target cell Nannochloropsis, a knock-in fragment (nucleotide fragment) comprising the LDSP promoter (ProLDSP) to which a homologous sequence (homology arm) with the nucleotide sequences at both ends of a desired target site upstream of LPAT1 had been added. In the method shown in Fig. 4, while the knock-in fragment including the LDSP promoter (ProLDSP) is inserted upstream of LPAT1, the all-in-one PtTALEN-ARS vector, which is a CEN/ARS vector, is not inserted into the target DNA (target DNA), which is genomic DNA. Therefore, it was considered that after the desired insertion was achieved, by performing elimination of the CEN/ARS vector, it would be possible to insert DNA in which no foreign genes other than the knock-in fragment remain in the cell.
As the knock-in fragment, a fragment (total 1304 bp, about 790 ng) was used, which had been constructed by adding, to the nucleotide sequence shown in SEQ ID NO: 20 encoding ProLDSP of Nannochloropsis, a sequence identical to the 217 bases before the start codon of LPAT1 of Nannochloropsis (SEQ ID NO: 21) at its 5' side and a sequence identical to the 320 bases after the start codon of LPAT1 at its 3' side. However, in the nucleotide sequence shown in SEQ ID NO: 20, so that the knock-in fragment would not be cleaved by TALEN, a partial mutation was introduced into the sequence corresponding to the DNA binding domain recognition sequence of R-TALEN of the PtTALEN described below.
An all-in-one PtTALEN-ARS vector (520 ng) was also prepared, in which, in the all-in-one PtTALEN-ARS plasmid described in NPL 3, the amino acid sequence of the DNA binding domain of PtTALEN was designed to cleave between 111 and 125 bases from the start codon of LPAT1 of Nannochloropsis.
The knock-in fragment and the all-in-one PtTALEN-ARS vector were subjected to electroporation into Nannochloropsis cells by the method of 5-3., and colonies were formed on an F2N 50% seawater medium plate (selection plate) containing zeocin by utilizing the zeocin resistance gene (ShBle) possessed by the all-in-one PtTALEN-ARS vector. Genomic DNA was extracted from each colony (12 colonies) by the method of 6-1., and PCR reaction was performed by the method of 7. above using a combination of primers designed inside the knock-in fragment (ProLDSP) and on the target DNA (upstream and downstream of LPAT1) shown in Fig. 5(a). The nucleotide sequences of each primer are shown in SEQ ID NOs: 22 to 25 in the order of the forward primer of Out-out PCR (forward primer of 5' Junction PCR), the reverse primer of Out-out PCR (reverse primer of 3' Junction PCR), the reverse primer of 5' Junction PCR, and the forward primer of 3' Junction PCR.
The result of agarose gel electrophoresis of the amplification product after the PCR reaction by the method of 8. above is shown in Fig. 5(b). As shown in Fig. 5(a), when the desired insertion is achieved, bands of 3151 bp, 1369 bp, and 1517 bp are all confirmed. However, in any of the colonies, such a combination of bands was not confirmed, and it was confirmed that the DNA insertion efficiency was not sufficient with the method shown in Fig. 4.

<Test Example 2> Construction of Non-Integrative Cleavage-Dependent Selection (NICS) Vector

One of the causes of the low DNA insertion efficiency in Test Example 1 was considered to be that among the colonies formed on the selection plate, there were few colonies into which the knock-in fragment and the all-in-one PtTALEN-ARS vector were simultaneously introduced, and a new method shown in Fig. 1A was designed. In the method shown in Fig. 1A, a vector (Fig. 1A(a)) was constructed in which a selection marker gene (in this test example, a paromomycin resistance gene) was divided into a 5' side (M1) and a 3' side (M2), and, between them, a homologous sequence (Hv1, Hv2) with the nucleotide sequences at both ends of the target site and the DNA of interest (D, in this test example, an LDSP promoter) were inserted as a knock-in fragment so as to be removable by a site-specific nuclease system (in this test example, TALEN). Since this vector was cleaved in cells into which the site-specific nuclease system had been introduced--i.e., cells in which the target DNA had likely also been cleaved and the knock-in fragment inserted (Fig. 1A(c))--the remaining fragments ligated to restore the function of the selection marker gene (Fig. 1A(b)); accordingly, an improvement in DNA insertion efficiency was anticipated. In this test example, as the target site, an upstream site of PDAT1--a Nannochloropsis gene that synthesizes oil and fat by transferring an acyl group to diacylglycerol and whose overexpression in other algae has been reported to increase oil and fat- -was selected.

(1) Validation Test 1

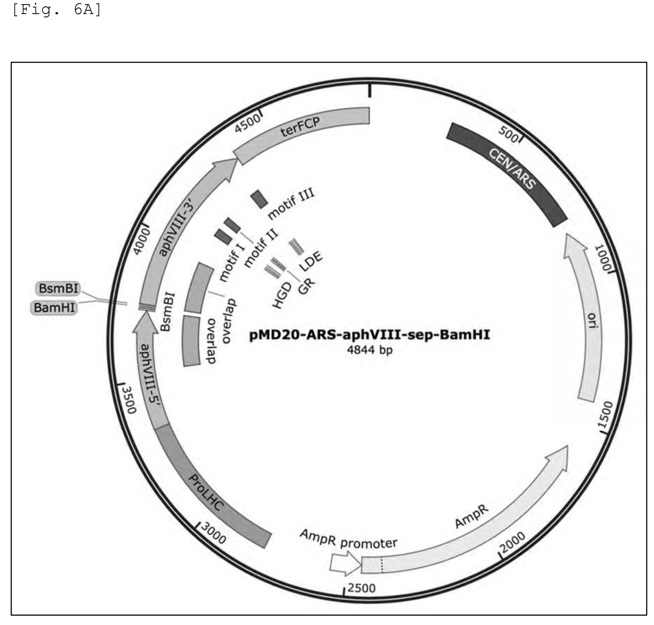

First, to confirm that this system functions in Nannochloropsis, a validation vector was prepared, and the system's function was confirmed. First, in a vector including CEN/ARS (nucleotide sequence shown in SEQ ID NO: 1), a paromomycin resistance gene (aphVIII), and a promoter (ProLHC, nucleotide sequence shown in SEQ ID NO: 2) and terminator (terFCP, nucleotide sequence shown in SEQ ID NO: 9) for the paromomycin resistance gene from Nannochloropsis, the paromomycin resistance gene (aphVIII) was divided into a 5' side fragment (aphVIII-5', nucleotide sequence shown in SEQ ID NO: 3) and a 3' side fragment (aphVIII-3', nucleotide sequence shown in SEQ ID NO: 8) with a 207 bp overlapping sequence (Overlap) (pMD20-ARS-aphVIII-sep-BamHI, Fig. 6A). Then, between these fragments, a binding region for PtTALEN for the nitrate reductase NoNR (Kurita et al., Genes Cells., 2020; 00: p. 1-8, DOI: 10.1111/gtc.12805 (Reference I)) was inserted, and this was designated as the validation vector_NoNR. A conceptual diagram of the constructed validation vector_NoNR is shown in Fig. 6B(a). When this vector is cleaved by the PtTALEN for NoNR, the paromomycin resistance gene functions, and paromomycin-resistant colonies are formed on the selection plate (Fig. 6B(b)).

Based on the all-in-one PtTALEN-ARS plasmid described in NPL 3, an all-in-one PtTALEN vector (TALEN vector_NoNR) was prepared in which the amino acid sequence of the DNA binding domain of PtTALEN was a sequence that binds to the binding region of PtTALEN for NoNR, and an all-in-one PtTALEN vector (TALEN vector_LPAT1) was prepared in which it was a sequence that binds to the binding region of PtTALEN in the vicinity of the target site of LPAT1 (Reference I).

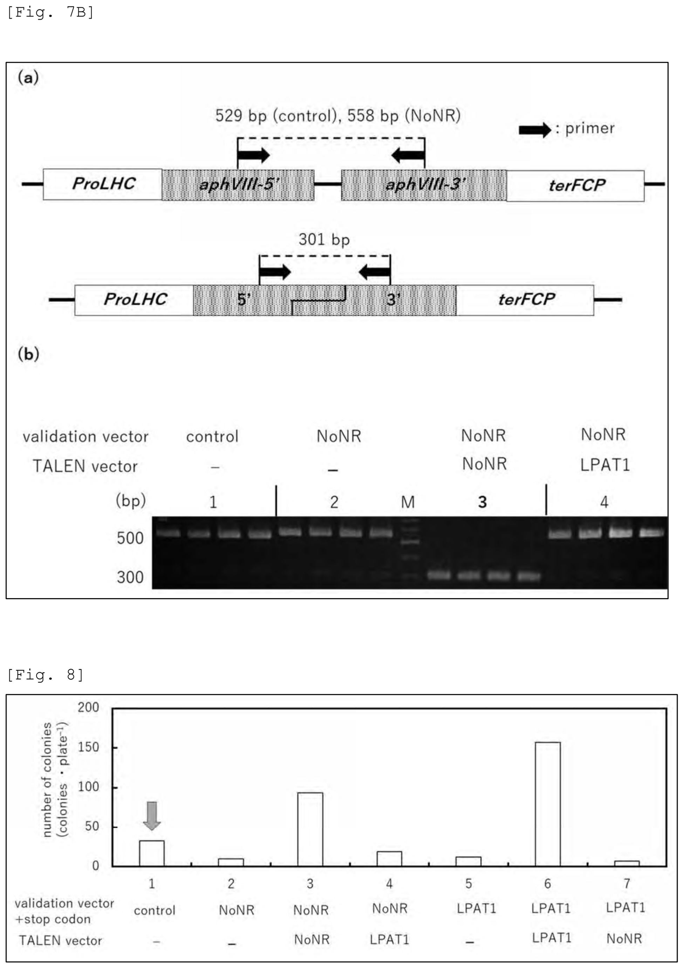

The test was performed for validation vector_NoNR alone (No. 2), a combination of validation vector_NoNR and TALEN vector_NoNR (No. 3), and a combination of validation vector_NoNR and TALEN vector_LPAT1 (No. 4), respectively. A case using the above pMD20-ARS-aphVIII-sep-BamHI (a vector in which, between the 5' side fragment and the 3' side fragment of the paromomycin resistance gene, a BamHI site is inserted so that the 5' side fragment and the 3' side fragment bind in-frame, instead of the binding region of PtTALEN; a control vector) (No. 1) was also performed. 200 ng of each vector was used, electroporation was performed into Nannochloropsis cells by the method of 5-3., and the number of paromomycin-resistant colonies formed on an F2N 50% seawater medium plate containing paromomycin was counted. The results are shown in Fig. 7A. From a representative colony of each vector combination, genomic DNA was extracted by the method of 6-1., and PCR reaction was performed by the method of 7. above using a combination of primers designed on the 5' side fragment and the 3' side fragment shown in Fig. 7B(a). The result of agarose gel electrophoresis of the amplification product after the PCR reaction by the method of 8. above is shown in Fig. 7B(b).

As shown in Fig. 7A, in cells into which a combination of validation vector_NoNR and TALEN vector_NoNR (No. 3) was introduced, paromomycin-resistant colonies were sufficiently formed. As shown in Fig. 7B(a), a band of 529 bp was confirmed in cells into which a control vector was introduced in which the space between the 5' side fragment and the 3' side fragment of the paromomycin resistance gene was connected in-frame, a band of 558 bp was confirmed in cells where validation vector_NoNR was not subjected to PtTALEN cleavage, and a band of 301 bp was confirmed in cells where validation vector_NoNR was cleaved by PtTALEN and recombined by the overlapping sequence. As shown in Fig. 7B(b), in the combination of validation vector_NoNR and TALEN vector_NoNR (No. 3), a band of 301 bp was confirmed, and it was confirmed that validation vector_NoNR was cleaved by PtTALEN and bound by the overlapping sequence, and it was confirmed that the above paromomycin resistance was due to the paromomycin resistance gene after the binding. However, as shown in Fig. 7A, many paromomycin-resistant colonies were also formed in cells into which a control vector (No. 1) was introduced in which the space between the 5' side fragment and the 3' side fragment of the paromomycin resistance gene was connected in-frame.

(2) Validation Test 2

From (1) above, because many paromomycin-resistant colonies are formed as a background even when the space between the 5' side fragment and the 3' side fragment of the paromomycin resistance gene is recombined in-frame, a vector in which a stop codon (TAG) was inserted immediately after the 5' side fragment of the paromomycin resistance gene (validation vector_NoNR_stop, control vector_stop) was prepared as each vector, and the same test as in (1) was performed. A vector in which, between the 5' side fragment and the 3' side fragment of the paromomycin resistance gene, the binding region of PtTALEN for LPAT1 was inserted instead of the binding region of PtTALEN for NoNR (validation vector_LPAT1_stop) was also prepared in the same manner, and tests were performed for control vector_stop alone (No. 1), validation vector_NoNR_stop alone (No. 2), a combination of validation vector_NoNR_stop and TALEN vector_NoNR (No. 3), a combination of validation vector_NoNR_stop and TALEN vector_LPAT1 (No. 4), validation vector_LPAT1_stop alone (No. 5), a combination of validation vector_LPAT1_stop and TALEN vector_LPAT1 (No. 6), and a combination of validation vector_LPAT1_stop and TALEN vector_NoNR (No. 7), respectively. The results are shown in Fig. 8.

As shown in Fig. 8, in cells into which a combination of validation vector_NoNR_stop and TALEN vector _NoNR (No. 3) and a combination of validation vector_LPAT1_stop and TALEN vector_LPAT1 (No. 6) were respectively introduced, similarly to (1) above, paromomycin-resistant colonies were sufficiently formed. On the other hand, in the strain into which the control vector_stop (No. 1) was introduced, the amount of paromomycin-resistant colony formation decreased compared to (1) above, and the background was successfully reduced sufficiently.

(3) Construction of NICS Vector (Example)

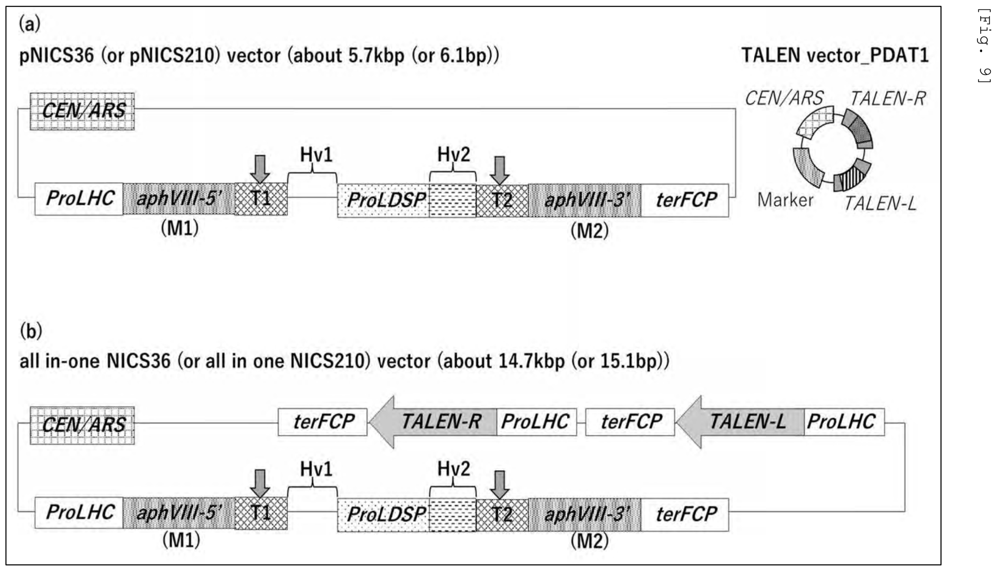

Based on the validation results of (1) and (2) above, a vector (NICS vector) for use in the method shown in Fig. 1A was constructed using the pMD20-ARS-aphVIII-sep-BamHI shown in Fig. 6A. A conceptual diagram more specifically showing the configuration of the vector constructed as the NICS vector shown in Fig. 1A(a) and Fig. 1C(a) is shown in the left of Fig. 9(a). The NICS vector shown in the left of Fig. 9(a) has the following configuration:

CEN/ARS (SEQ ID NO: 1),
promoter P1 (ProLHC, SEQ ID NO: 2) and terminator (terFCP, SEQ ID NO: 9) for the paromomycin resistance gene, between the promoter and the terminator, a sequence in which the paromomycin resistance gene (aphVIII) is divided into a 5' side fragment M1 (aphVIII-5', SEQ ID NO: 3) and a 3' side fragment M2 (aphVIII-3', SEQ ID NO: 8) with a 207 bp overlapping sequence,
between M1 and M2, binding regions T1 and T2 for PtTALEN for PDAT1 (both, SEQ ID NO: 4, 5'-L1 (Left-TALEN DNA binding domain recognition sequence, SEQ ID NO: 10)-S1 (spacer sequence)-R1 (complementary sequence of Right-TALEN DNA binding domain recognition sequence, SEQ ID NO: 11)-3'),
between T1 and T2, homologous sequences Hv1 and Hv2,
between Hv1 and Hv2, ProLDSP (SEQ ID NO: 6) as knock-in fragment D,
and a stop codon (TAG) was inserted between the 5' side fragment M1 of the paromomycin resistance gene and T1, and between the 3' side fragment M2 and T2, respectively (however, the latter partially overlaps with M2). Hv1 and Hv2 are sequences identical to the sequence immediately before the start codon and the sequence after the start codon of PDAT1 of Nannochloropsis, respectively. For the vector, one was prepared in which the lengths of the homologous sequences Hv1 and Hv2 were both 36 bp (Hv1: SEQ ID NO: 5, a sequence identical to the nucleotide sequence immediately before the start codon of PDAT1 / Hv2: SEQ ID NO: 7, a sequence identical to the nucleotide sequence after the start codon of PDAT1) (pNICS36 vector), and one was prepared in which they were 210 bp (Hv1: SEQ ID NO: 12, a sequence identical to the nucleotide sequence immediately before the start codon of PDAT1 / Hv2: SEQ ID NO: 13, a sequence identical to the nucleotide sequence after the start codon of PDAT1) (pNICS210 vector). The nucleotide sequence of the pNICS36 vector is shown in SEQ ID NO: 14, and the nucleotide sequence of the pNICS210 vector is shown in SEQ ID NO: 15, respectively.

As a site-specific nuclease system expression vector to be combined with the NICS vector (pNICS36 vector or pNICS210 vector), in the all-in-one PtTALEN-ARS plasmid described in NPL 3, an all-in-one PtTALEN-ARS vector (TALEN vector_PDAT1) was prepared in which the amino acid sequence of the DNA binding domain of PtTALEN was a sequence that binds to the sequence T3 around the start codon of PDAT1 (a sequence identical to the sequences of T1 and T2). A conceptual diagram showing the configuration of the prepared vector is shown together on the right of Fig. 9(a).
Furthermore, as the all-in-one vector (all-in-one NICS vector) shown in Fig. 3, a vector was constructed by fusing the NICS vector and the TALEN vector_PDAT1. A conceptual diagram more specifically showing the configuration of the constructed all-in-one NICS vector is shown in Fig. 9(b). The all-in-one NICS vector shown in Fig. 9(b) has the following configuration:

CEN/ARS (*),

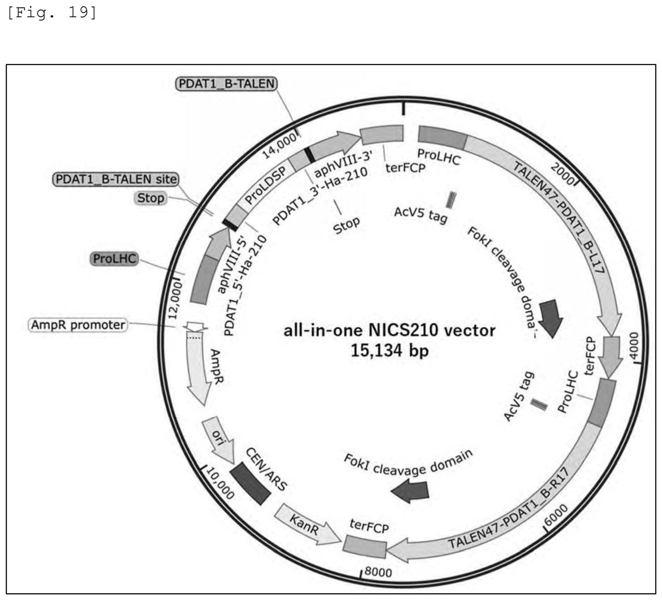

promoter P1 (ProLHC (*)) and terminator (terFCP (*)) for the paromomycin resistance gene,
between the promoter and the terminator, the 5' side fragment M1 (*) and the 3' side fragment M2 (*) of the paromomycin resistance gene,
between M1 and M2, binding regions T1 and T2 (*) for PtTALEN for PDAT1,
between T1 and T2, homologous sequences Hv1 and Hv2 (*),
between Hv1 and Hv2, ProLDSP (*) as knock-in fragment D,
a nucleotide sequence (TALEN-L, SEQ ID NO: 16) encoding TALEN-L including a DNA binding domain that recognizes L1 of T1, T2, and T3,
a nucleotide sequence (TALEN-R, SEQ ID NO: 17) encoding TALEN-R including a DNA binding domain that recognizes R1 of T1, T2, and T3, and

promoter P2 (ProLHC (*)) and terminator (terFCP (*)) for TALEN-L and TALEN-R,
and each of the configurations indicated by "*" above is common to the NICS vector. The nucleotide sequence of the all-in-one NICS36 vector in which the length of the homologous sequences Hv1 and Hv2 was 36 bp is shown in SEQ ID NO: 18, and the nucleotide sequence of the all-in-one NICS210 vector in which it was 210 bp is shown in SEQ ID NO: 19, respectively. Also, a schematic diagram showing the detailed configuration of the all-in-one NICS210 vector is shown in Fig. 19. In the electroporation of Nannochloropsis, it is considered that two types of vectors are simultaneously inserted into one cell at about 20% (Reference I), and therefore higher knock-in efficiency was expected with the all-in-one NICS vector.

(4) Cell Introduction Test of NICS Vector

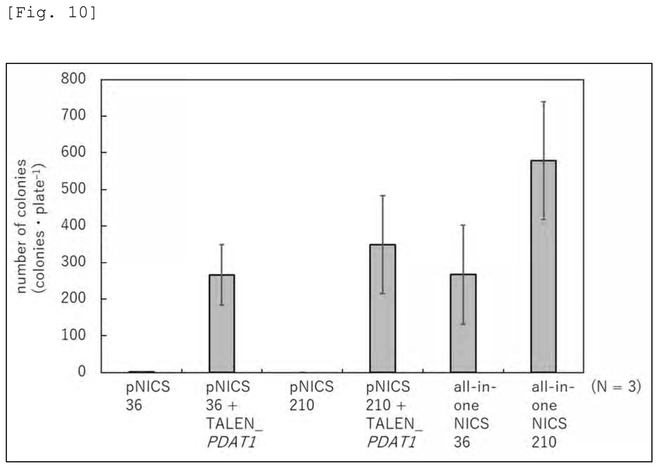

The test was performed for pNICS36 vector or pNICS210 vector alone, a combination of pNICS36 vector or pNICS210 vector and TALEN vector_PDAT1, all-in-one NICS36 vector, and all-in-one NICS210 vector, respectively. 200 ng of each vector was used, electroporation was performed into Nannochloropsis cells by the method of 5-3. above, and the number of paromomycin-resistant colonies formed on an F2N 50% seawater medium plate containing paromomycin was counted. The results are shown in Fig. 10. As shown in Fig. 10, it was confirmed that in cells into which a combination of pNICS36 vector or pNICS210 vector and TALEN vector_PDAT1, all-in-one NICS36 vector, and all-in-one NICS210 vector were introduced, paromomycin-resistant colonies were sufficiently formed in all cases. Also, the fact that no colonies were formed with pNICS36 vector or pNICS210 vector alone confirmed that the background was suppressed to a higher degree.

(5) PCR Analysis and Sequence Analysis

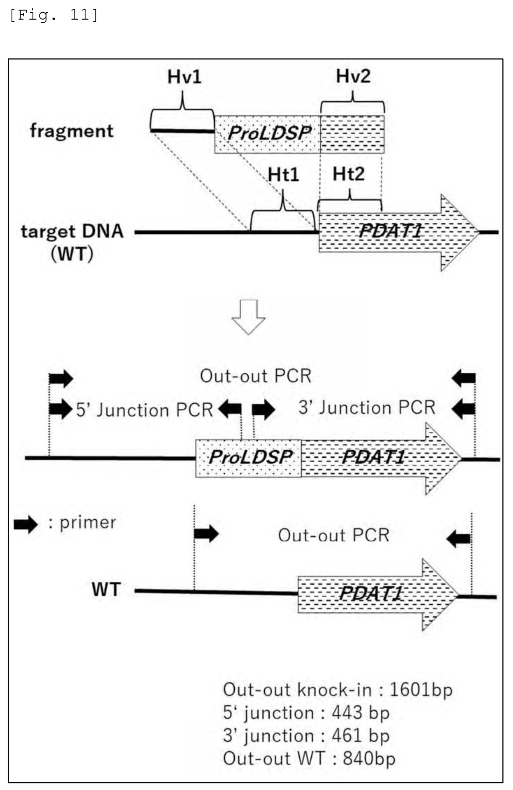

From the colonies formed in (4) above, genomic DNA was extracted from each of 16 randomly selected colonies by the method of 6-2. above, and PCR reaction was performed by the method of 7. above using a combination of primers designed inside the knock-in fragment (ProLDSP) and on the target DNA (upstream and downstream of PDAT1) shown in Fig. 11. The nucleotide sequences of each primer are shown in SEQ ID NOs: 26 to 29 in the order of the forward primer of Out-out PCR (forward primer of 5' Junction PCR), the reverse primer of Out-out PCR (reverse primer of 3' Junction PCR), the reverse primer of 5' Junction PCR, and the forward primer of 3' Junction PCR. The result of agarose gel electrophoresis of the amplification product after PCR reaction for a colony formed by introducing a combination of the pNICS210 vector and the TALEN vector_PDAT1 by the method of 8. above is shown in Fig. 12, and the result of agarose gel electrophoresis of the amplification product after PCR reaction for a colony formed by introducing the all-in-one NICS210 vector by the method of 8. above is shown in Fig. 13.

As shown in Fig. 11, when the desired insertion is achieved, bands of 1601 bp, 443 bp, and 461 bp are all confirmed, but as shown in Fig. 12 and Fig. 13, when a combination of the pNICS210 vector and the TALEN vector_PDAT1 was introduced, the desired bands were confirmed in 1 strain (colony No. 9 in Fig. 12), and when the all-in-one NICS210 vector was introduced, the desired bands were confirmed in 5 strains (colonies No. 5, 8, 11, 13, 14 in Fig. 13).

Furthermore, for the colonies in which the desired bands were obtained above, each band was cut out, DNA was extracted, and when sequence analysis was performed by the method of 9. above to determine whether the sequence of the desired knock-in fragment ProLDSP had been introduced, in the end, an accurate sequence was detected at 1/16 (DNA insertion efficiency: 6.25%) when a combination of the pNICS210 vector and the TALEN vector_PDAT1 was introduced, and at 4/16 (DNA insertion efficiency: 25%) when the all-in-one NICS210 vector was introduced, and it was confirmed that the desired insertion was possible in both cases. In particular, it was confirmed that when the all-in-one NICS210 vector was introduced, DNA introduction was possible with higher efficiency.

(6) CEN/ARS Vector Elimination Test

In the strains in which the insertion of the knock-in fragment was confirmed in (5) above, a selection vector including CEN/ARS is formed from the NICS vector. Therefore, from a part of the strains in which the accurate insertion of the knock-in fragment was confirmed in (5) above (colonies No. 5 and 8 in Fig. 13), with paromomycin as the antibiotic, the selection vector, which is a CEN/ARS vector, was eliminated by the method of 10. above. That is, the elimination candidate colonies (parental strains before vector elimination) obtained by plating on an F2N plate after culturing in F2N liquid medium for 14 days were seeded on both an F2N plate and an F2N 50% seawater medium plate containing paromomycin (selection plate), and 6 strains each that grew on the F2N plate but did not grow on the selection plate containing paromomycin were obtained. The plate appearance showing the growth status of each strain is shown in Fig. 14.

Then, each of the six strains obtained above and each parental strain before vector elimination (colonies No. 5 and 8 in Fig. 13) were cultured under normal conditions in 12-well plates; genomic DNA was extracted by the method of 6-2. above and, using this as a template, the elimination of the selection vector was confirmed by PCR (method of 7. above) with primers that detect FokI, the nuclease domain of TALEN. The result of agarose gel electrophoresis of the amplification product after the PCR reaction by the method of 8. above is shown in Fig. 15. As shown in Fig. 15, FokI was not detected in all 6 strains.

Furthermore, instead of the method of 6-2. above, highly purified genomic DNA was extracted by the method of 6-1., and using this as a template, it was examined again in detail by PCR using primers that detect FokI and primers that respectively detect the N-terminal domain of TALEN (TALEN-N), the paromomycin resistance gene (ParoR), and the kanamycin resistance gene (KanR), as well as primers for out-out PCR of PDAT1, which is the knock-in fragment (donor) (Fig. 11). The result of agarose gel electrophoresis of the amplification product after the PCR reaction is shown in Fig. 16.

As shown in Fig. 16, strains in which the band of PDAT1 was confirmed and in which none of the bands of genes derived from the selection vector (FokI, TALEN-N, ParoR, KanR) were confirmed were obtained at a high probability, with two strains each from the strain derived from colony No. 5 and the strain derived from colony No. 8 (strain derived from colony No. 5: #1, #2, strain derived from colony No. 8: #4, #5). From these results, it was confirmed that according to the method using the NICS vector of the present invention, it is possible to accurately insert a desired nucleotide sequence (in this test example, the LDSP promoter) at a target site of a target DNA (in this test example, upstream of PDAT1), and furthermore, thereafter, a genomic DNA-edited strain, from which the vector has been eliminated and in which foreign genes other than such a desired nucleotide sequence are removed, can be constructed.

<Test Example 3> Verification of DNA Editing Targeting GPAT1

It was confirmed that DNA introduction is possible with the NICS vector of the present invention for different target sites as well. In this test example, an upstream site of GPAT1 (glycerol-3-phosphate acyltransferase) of Nannochloropsis was selected as the target site.

(1) Construction of NICS Vector (Example)

First, in the same manner as for the all-in-one NICS210 vector of Test Example 2 (3), the following configuration was used:

CEN/ARS (*),
promoter P1 (ProLHC (*)) and terminator (terFCP (*)) for the paromomycin resistance gene,
between the promoter and the terminator, the 5' side fragment M1 (*) and the 3' side fragment M2 (*) of the paromomycin resistance gene,
between M1 and M2, binding regions T1 and T2 for PtTALEN for GPAT1 (both, SEQ ID NO: 30, 5'-L1 (Left-TALEN DNA binding domain recognition sequence, SEQ ID NO: 31)-S1 (spacer sequence)-R1 (complementary sequence of Right-TALEN DNA binding domain recognition sequence, SEQ ID NO: 32)-3'),
between T1 and T2, homologous sequences Hv1 and Hv2,
between Hv1 and Hv2, ProLDSP (*) as knock-in fragment D,
a nucleotide sequence (TALEN-L, SEQ ID NO: 35) encoding TALEN-L including a DNA binding domain that recognizes L1 of T1, T2, and T3,
a nucleotide sequence (TALEN-R, SEQ ID NO: 36) encoding TALEN-R including a DNA binding domain that recognizes R1 of T1, T2, and T3, and
promoter P2 (ProLHC (*)) and terminator (terFCP (*)) for TALEN-L and TALEN-R,
and each of the configurations indicated by "*" above is common to the NICS vector and the all-in-one NICS vector. Further, T1 and T2 are sequences identical to the sequence T3 around the start codon of GPAT1 of Nannochloropsis, respectively. The lengths of the homologous sequences Hv1 and Hv2 were 200 bp (Hv1; SEQ ID NO: 33, a sequence identical to the nucleotide sequence immediately before the start codon of GPAT1) and 208 bp (Hv2; SEQ ID NO: 34, a sequence identical to the nucleotide sequence after the start codon of GPAT1), respectively (all-in-one NICS210-GPAT1 vector). The nucleotide sequence of the all-in-one NICS210-GPAT1 vector is shown in SEQ ID NO: 37.

(2) Cell Introduction Test of NICS Vector

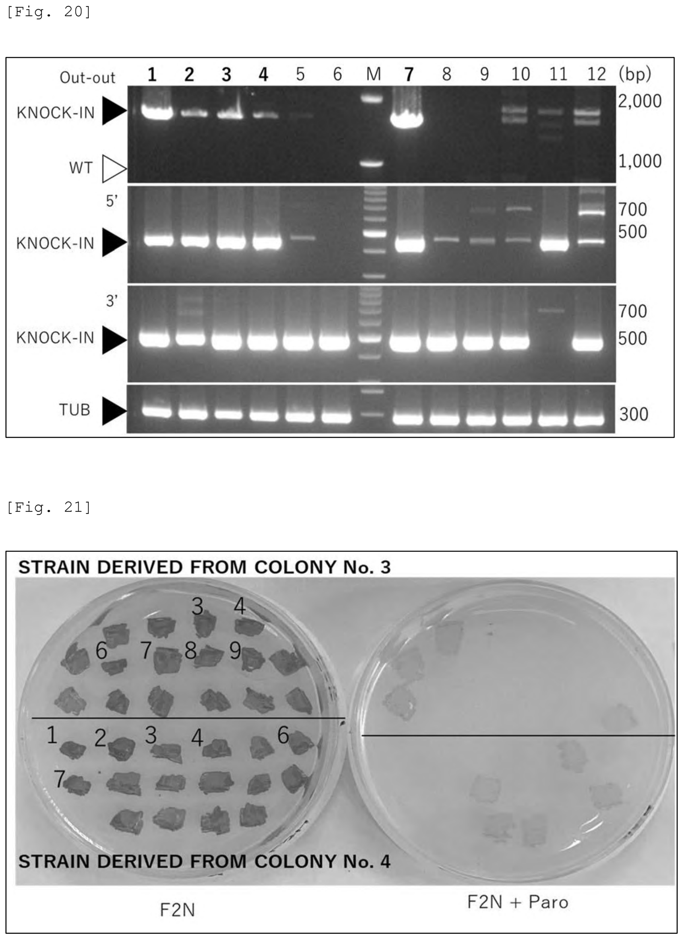

A cell introduction test was performed in the same manner as in Test Example 2 (4), except that the all-in-one NICS210-GPAT1 vector was used instead of the all-in-one NICS210 vector. Among the colonies formed on the F2N 50% seawater medium plate containing paromomycin, in the same manner as in Test Example 2 (5), genomic DNA was extracted from each of the 12 randomly selected colonies by the method of 6-2. above, and PCR reaction was performed. The result of agarose gel electrophoresis of the amplification product after the PCR reaction is shown in Fig. 20. In Fig. 20, the position of the band confirmed when the desired insertion (knock-in) is achieved is indicated by a solid black triangle, and the position of the band confirmed when insertion did not occur (WT) is indicated by a hollow white triangle.

As shown in Fig. 20, as a result of introducing the all-in-one NICS210-GPAT1 vector, the desired bands were confirmed in 5 strains (colonies No. 1 to 4, 7 in Fig. 20). Furthermore, for the colonies in which the desired bands were obtained above, when sequence analysis was performed in the same manner as in Test Example 2 (5), in the end, even when the all-in-one NICS210-GPAT1 vector was introduced, an accurate sequence was detected at 2/12 (DNA insertion efficiency: 17%), and it was confirmed that DNA introduction was possible with high efficiency for the target site, that is, the upstream site of GPAT1, as well.

(3) CEN/ARS Vector Elimination Test

From a part of the strains in which the accurate insertion of the knock-in fragment was confirmed in (2) above (colonies No. 3, 4 in Fig. 20), with paromomycin as the antibiotic, in the same manner as in Test Example 2 (6), the selection vector, which is a CEN/ARS vector, was eliminated, respectively. As a result, colonies that grew on the F2N plate but did not grow on the selection plate containing paromomycin were obtained, 6 from colony No. 3 (#3, #4, #6 to 9) and 6 from colony No. 4 (#1 to 4, #6, #7), and it was confirmed that even in this test example, the CEN/ARS vector was eliminated, and a genomic DNA-edited strain in which foreign genes other than the desired nucleotide sequence were removed could be constructed. The plate appearance showing the growth status of each strain is shown in Fig. 21.

<Test Example 4> Verification of Length of Homologous Sequence in All-in-one NICS Vector

(1) Construction of NICS Vector (Example)

An all-in-one NICS vector (all-in-one NICS400 vector) was constructed in the same manner as the all-in-one NICS210 vector of Test Example 2 (3), except that the lengths of the homologous sequences Hv1 and Hv2 in the all-in-one NICS vector were both 414 bp (Hv1; SEQ ID NO: 38, a sequence identical to the nucleotide sequence immediately before the start codon of PDAT1 / Hv2; SEQ ID NO: 39, a sequence identical to the nucleotide sequence after the start codon of PDAT1). The nucleotide sequence of the all-in-one NICS400 vector is shown in SEQ ID NO: 40.

(2) Cell Introduction Test of NICS Vector

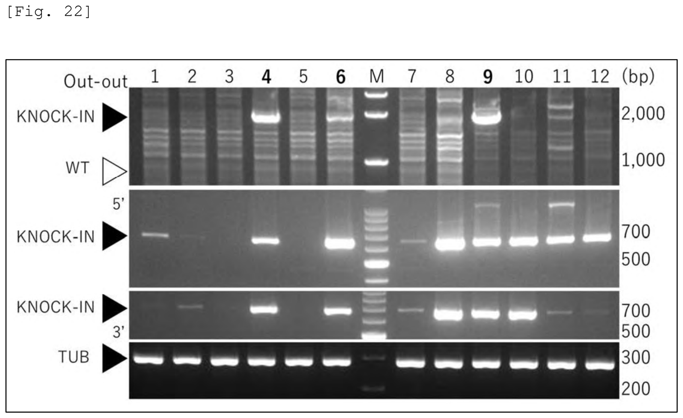

A cell introduction test was performed in the same manner as in Test Example 2 (4), except that the all-in-one NICS400 vector was used instead of the all-in-one NICS210 vector. Among the colonies formed on the F2N 50% seawater medium plate containing paromomycin, in the same manner as in Test Example 2 (5), genomic DNA was extracted from each of the 12 randomly selected colonies by the method of 6-2. above, and PCR reaction was performed. The result of agarose gel electrophoresis of the amplification product after the PCR reaction is shown in Fig. 22. In Fig. 22, the position of the band confirmed when the desired insertion is achieved is indicated by a solid black triangle, and the position of the band confirmed when insertion did not occur (WT) is indicated by a hollow white triangle.

As shown in Fig. 22, as a result of introducing the all-in-one NICS400 vector, the desired bands were confirmed in 3 strains (colonies No. 4, 6, 9 in Fig. 22). Furthermore, for the colonies in which the desired bands were obtained above, when sequence analysis was performed in the same manner as in Test Example 2 (5), in the end, even when the all-in-one NICS400 vector was introduced, an accurate sequence was detected at 3/12 (DNA insertion efficiency: 25%), and it was confirmed that DNA introduction was possible with high efficiency.

<Test Example 5> Application of NICS System to Human Cultured Cells

As another embodiment of the method shown in Fig. 1A, the method shown in Fig. 23 was designed, and it was confirmed that the vector of the present invention is also applicable to human cultured cells. In the method shown in Fig. 23, a selection marker gene (in this test example, a puromycin resistance gene) was divided into a 5' side (M1) and a 3' side (M2), and between them, a vector was constructed in which a homologous sequence (Hv1, Hv2) with the nucleotide sequences at both ends of the target site and the DNA of interest (D, in this test example, a 3×Flag tag sequence) were inserted as a knock-in fragment so as to be removable by a site-specific nuclease system (in this test example, a CRISPR-Cas system) (upper panel of Fig. 23). This vector is cleaved in a cell into which the site-specific nuclease system has been introduced, that is, a cell in which it is highly likely that the target DNA has also been cleaved and the knock-in fragment has been inserted (lower right panel of Fig. 23), and the remaining fragments bind and the selection marker gene functions (lower left panel of Fig. 23). In this test example, downstream sites of PARP1 and ATP5B, which are genes (endogenous genes) on the human genome, were selected as target sites.

(1) Construction of NICS Vector (Example)

In the same manner as in Test Example 2 (3), a vector (NICS vector) for use in the method shown in Fig. 1A was constructed. A conceptual diagram more specifically showing the configuration of the vector constructed as the NICS vector shown in Fig. 1A(a) and Fig. 1C(b) is shown in the upper panel of Fig. 23. The NICS vector shown in Fig. 23 has the following configuration:

promoter P1 (pCMV, SEQ ID NO: 41) and polyA (bGH polyA signal, SEQ ID NO: 42) for the puromycin resistance gene,

between the promoter and polyA, a sequence in which the puromycin resistance gene (PuroR) is divided into a 5' side fragment M1 (PuroR-5', SEQ ID NO: 43) and a 3' side fragment M2 (PuroR-3', SEQ ID NO: 44) with a 60 bp overlapping sequence, or a sequence in which the puromycin resistance gene (PuroR) is divided into a 5' side fragment M1 (PuroR-5', SEQ ID NO: 51 (same as SEQ ID NO: 43)) and a 3' side fragment M2 (PuroR-3', SEQ ID NO: 52) with a 192 bp overlapping sequence,

between M1 and M2, gRNA recognition sequence G1 (SEQ ID NO: 45, common for PARP1 and ATP5B) and gRNA recognition sequence G2 (complementary sequence of G1, SEQ ID NO: 46, common for PARP1 and ATP5B),

between G1 and G2, homologous sequences Hv1 and Hv2,

between Hv1 and Hv2, a sequence encoding a 3×Flag tag (SEQ ID NO: 47) as knock-in fragment D,

and a stop codon (TAG or TAA) was inserted between the 5' side fragment M1 of the puromycin resistance gene and G1, and between the 3' side fragment M2 and G2, respectively. Hv1 and Hv2 are sequences identical to the sequence immediately before the stop codon and the sequence after the stop codon, respectively, for each of PARP1 and ATP5B. For the vector, the lengths of the homologous sequences Hv1 and Hv2 were both 40 bp ([PARP1] Hv1: SEQ ID NO: 48, a sequence identical to the nucleotide sequence immediately before the stop codon of PARP1 / Hv2: SEQ ID NO: 49, a sequence identical to the nucleotide sequence after the stop codon of PARP1, [ATP5B] Hv1: SEQ ID NO: 54, a sequence identical to the nucleotide sequence immediately before the stop codon of ATP5B / Hv2: SEQ ID NO: 55, a sequence identical to the nucleotide sequence after the stop codon of ATP5B). The nucleotide sequence of the NICS vector for PARP1 with a 60 bp marker gene overlapping sequence (pNICS60-PARP1 vector) is shown in SEQ ID NO: 50, the nucleotide sequence of the NICS vector for PARP1 with a 192 bp marker gene overlapping sequence (pNICS192-PARP1 vector) is shown in SEQ ID NO: 53, the nucleotide sequence of the NICS vector for ATP5B with a 60 bp marker gene overlapping sequence (pNICS60-ATP5B vector) is shown in SEQ ID NO: 56, and the nucleotide sequence of the NICS vector for ATP5B with a 192 bp marker gene overlapping sequence (pNICS192-ATP5B vector) is shown in SEQ ID NO: 57.

As a site-specific nuclease system expression vector to be combined with the NICS vector, in the CRISPR-Cas9 vector described in Sakuma et al., NATURE PROTOCOLS, Vol. 11, No. 1, 2016, p. 118-133, a CRISPR/Cas9 vector was prepared in which the sequence of the guide RNA (gRNA) was a sequence that binds to G1, G2, and G3 (common in G1 to G3, a sequence around the stop codon of PARP1 or ATP5B), respectively.

(2) Validation Test

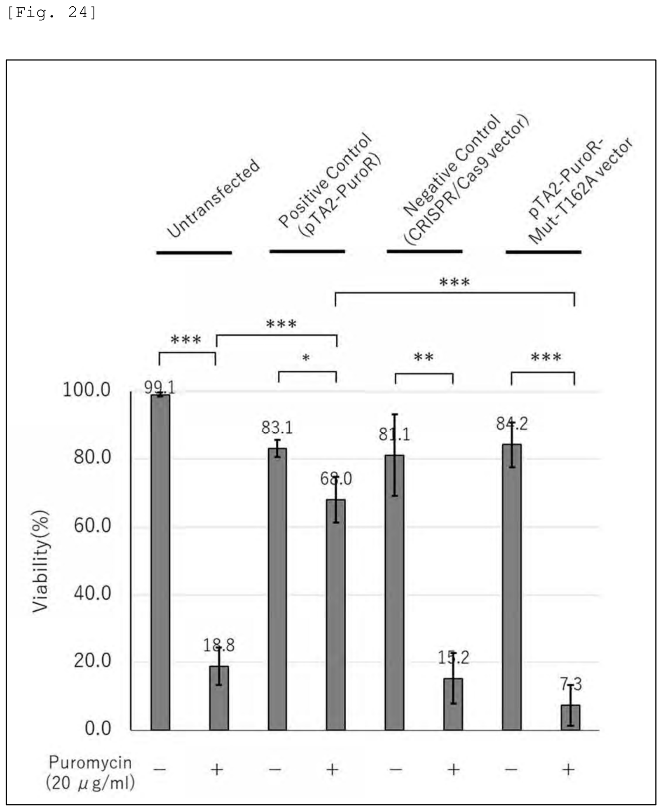

It was confirmed that cells into which a vector other than the expression vector for undivided PuroR was introduced did not exhibit puromycin resistance. That is, no vector (Untransfected), pTA2-PuroR (a vector including undivided PuroR, Positive Control), CRISPR/Cas9 vector (Negative Control), and pTA2-PuroR-Mut-T162A vector (a vector in which PuroR was inactivated by introducing a mutation) were each introduced into HEK293T by the method described in 12-3. above, the cells were cultured and collected by the method described in 12-4. above with a puromycin concentration of 0 µg/mL (-) or 20 µg/mL (+), the number of cells was counted by the method described in 12-5. above, and the Viability was calculated. The results are shown in Fig. 24.

As shown in Fig. 24, except for the Positive Control, it was confirmed that most of the cells were killed by puromycin treatment, that the cell survival rate (Viability) decreased to less than 20%, and that they did not grow in the presence of puromycin.

(3) Cell Introduction Test of NICS Vector

The test was performed for no vector (Untransfected: WT), each NICS vector alone (pNICS60-PARP1 vector, pNICS192-PARP1 vector, pNICS60-ATP5B vector, or pNICS192-ATP5B vector), and each NICS vector in combination with a CRISPR/Cas9 vector encoding a gRNA corresponding to the guide RNA recognition sequence (G3) on the gene targeted by that NICS vector (that is, a CRISPR/Cas9 vector targeting the gene), respectively.

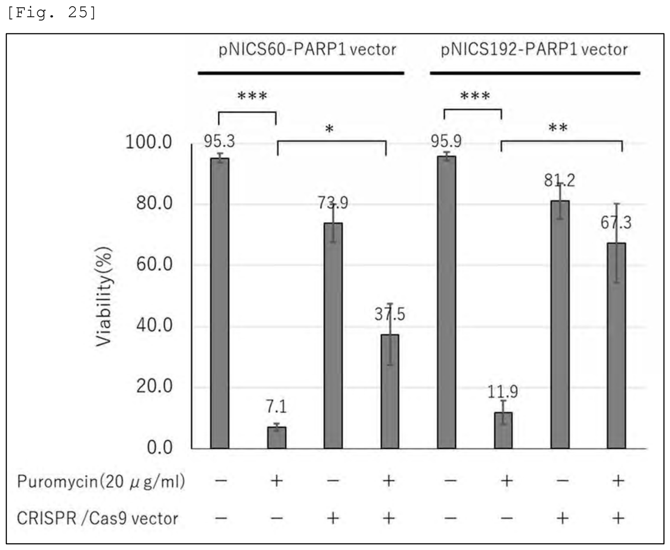

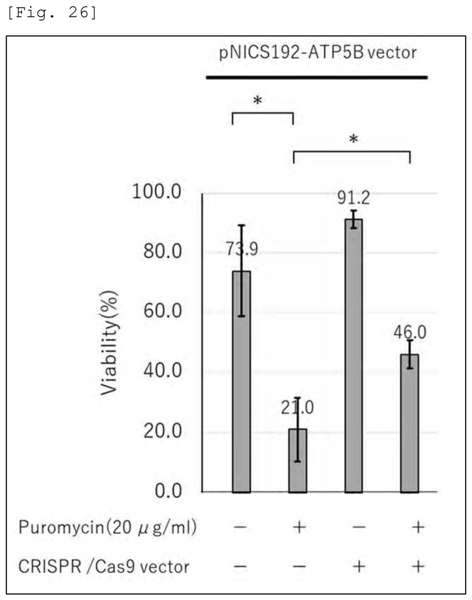

For these, they were introduced into HEK293T in the same manner as in (2) above, and the Viability at a puromycin concentration of 0 µg/mL (-) or 20 µg/mL (+) was calculated. As a result for PARP1, the survival rate under each puromycin concentration for each combination of a NICS vector (pNICS60-PARP1 vector, pNICS192-PARP1 vector) and a CRISPR/Cas9 vector targeting PARP1 is shown in Fig. 25, and as a result for ATP5B, the survival rate under each puromycin concentration for each combination of a NICS vector (pNICS192-ATP5B vector) and a CRISPR/Cas9 vector targeting ATP5B is shown in Fig. 26.

As shown in Fig. 25 and Fig. 26, in cells into which only the NICS vector was introduced, the survival rate at a puromycin concentration of 20 μg/mL was less than twenty-something percent and most of the cells were killed, but in cells co-introduced with the CRISPR/Cas9 vector, a high survival rate was shown in all cases. From this, it was confirmed that the NICS vector from which the 3×Flag tag sequence was excised by the CRISPR/Cas9 vector was recombined by the overlapping sequence and the puromycin resistance gene (PuroR) was expressed (lower left panel of Fig. 23).

(4) PCR analysis

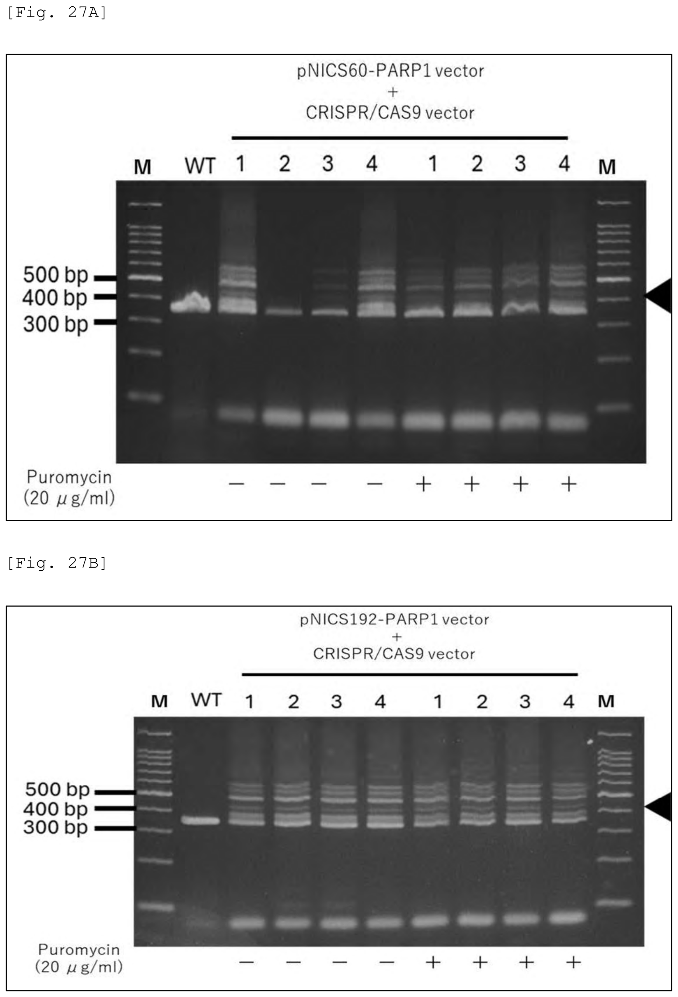

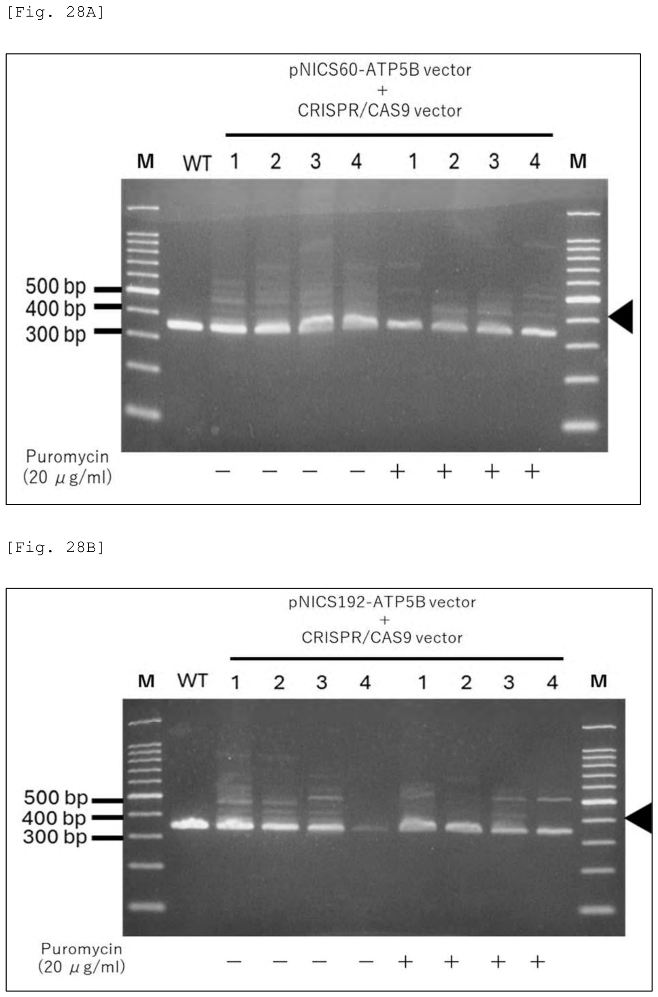

For no vector (Untransfected: WT) and each combination of a NICS vector and a CRISPR/Cas9 vector, after (3) above, out-out PCR reaction was performed by the methods of 13-1. to 13-2. above (each n=4). The results of agarose gel electrophoresis of the amplification product after the PCR reaction by the method of 8. above are shown in Fig. 27A and Fig. 27B (PARP1), and Fig. 28A and Fig. 28B (ATP5B), respectively. When the desired insertion is achieved, the confirmed band length becomes 425 bp (after insertion) from 349 bp (before insertion) for PARP1, and becomes 424 bp (after insertion) from 347 bp (before insertion) for ATP5B.

As shown in Fig. 27A to Fig. 28B, in colonies formed at a puromycin concentration of 20 μg/mL (+) by co-introducing each NICS vector and a CRISPR/Cas9 vector, the expected band lengths after insertion were confirmed, respectively.

(5) TIDER Analysis

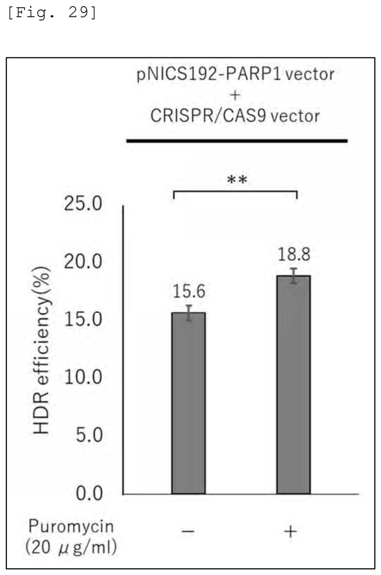

From the PCR product of (4) above, TIDER analysis was performed by the methods of 14-1. to 14-3. above, and the HDR efficiency (HDR efficiency [%], n=4) was determined. As a result, it was confirmed that insertion of the 3×Flag tag sequence occurred under all conditions. Of these, as an example of a comparison of the HDR efficiency (insertion efficiency) with and without puromycin selection, the result when a combination of the pNICS192-PARP1 vector and a CRISPR/Cas9 vector was introduced (Fig. 27B) is shown in Fig. 29. As shown in Fig. 29, in this combination, it was confirmed that the insertion efficiency was significantly increased by the addition of the antibiotic (puromycin) corresponding to the marker gene.

From the above, it was confirmed that according to the NICS system of the present invention, it is possible to obtain a DNA-edited cell (for example, a genome-edited cell) in which a target DNA has been edited, both in algae and in cells derived from a different biological species (for example, humans), and that the obtained DNA-edited cell can be easily selected and isolated while appropriately selecting the target locus and marker gene according to the purpose, without being limited to them.

[Industrial Applicability]

As described above, according to the present invention, it becomes possible to provide a DNA editing method capable of accurately and efficiently inserting a desired nucleotide sequence at a target site of a target DNA in a cell or removing the target site by a vector, a cell production method using the same, and a DNA editing vector and a DNA editing kit for use in these methods.

In addition, in cases where the vector further includes CEN/ARS, which is an autonomously replicating sequence, it also becomes possible to provide a DNA editing method by which, after inserting a desired nucleotide sequence at the target site of the target DNA or removing the target site from the target DNA by the vector, the vector can be eliminated, and a genomic DNA-edited cell from which foreign genes other than the inserted nucleotide sequence have been removed can be obtained with high efficiency, a cell production method using the same, and a DNA editing vector and a DNA editing kit for use in these methods.

For example, by setting the target site immediately before the start codon of a target gene, inserting only a strong endogenous promoter (for example, the ProLDSP above) here, and allowing the remainder of the vector to be eliminated, it becomes possible to overexpress the target gene.

**Claims**

1. A method for editing DNA, which is a method for inserting a desired nucleotide sequence at a target site of a target DNA in a cell or removing the target site,

   the method comprising an introduction step of introducing a vector and a site-specific nuclease system into a cell to bring them into contact with a target DNA, wherein
the vector comprises

a first promoter P1, and
the following structure (1):

5' -M1-Hv1-D-Hv2-M2-3'...       (1)

[in (1), M1 represents a 5' side fragment of a nucleotide sequence encoding a selection marker gene and is operably linked to the first promoter P1, Hv1 represents a nucleotide sequence homologous to a first nucleotide sequence Ht1 on the 5' side of the target site of the target DNA, D represents the desired nucleotide sequence but may be absent, Hv2 represents a nucleotide sequence homologous to a second nucleotide sequence Ht2 on the 3' side of the target site of the target DNA, M2 represents the remaining 3' side fragment of the nucleotide sequence encoding the selection marker gene, and in M1, Hv1, D, Hv2, and M2, adjacent sequences may partially overlap with each other.],

a cleavage step of generating from the vector, by the site-specific nuclease system, a fragment represented by the following structure (2):

5'-Hv1-D-Hv2-3'...       (2)

and a fragment represented by the following structure (3):

5'-M2-P1-M1-3'...       (3)

and cleaving the target site or its vicinity in the target DNA,
an editing step in which the target DNA and the fragment represented by structure (2) bind depending on the homology between Ht1 and Hv1 and bind depending on the homology between Ht2 and Hv2, and the desired nucleotide sequence D is inserted at the target site or the target site is removed, and
a selection step of obtaining a selection vector in which, in the fragment represented by structure (3), the 3' end of M1 and the 5' end of M2 are ligated to obtain a selection vector that contains a functional selection marker gene operably linked to P1.

2. The DNA editing method according to claim 1, wherein

the vector further comprises CEN/ARS, which is an autonomously replicating sequence,
structure (3) generated in the cleavage step further comprises CEN/ARS, and
the selection step is a step of obtaining a selection vector in which, in the fragment represented by structure (3), the 3' end of M1 and the 5' end of M2 are ligated to obtain a selection vector that contains a functional selection marker gene operably linked to P1 and also contains CEN/ARS.

3. The DNA editing method according to claim 1, wherein the length of Hv1 and the length of Hv2 are each independently 30 to 600 bases.

4. The DNA editing method according to claim 1, wherein

the vector comprises, as structure (1), the following structure (11):

5'-M1-T1-Hv1-D-Hv2-T2-M2-3'...       (11)

[in (11), T1 represents a TALEN_1 binding region comprising a first DNA binding domain recognition sequence or its complementary sequence L1 and a second DNA binding domain recognition sequence or its complementary sequence R1, T2 represents a TALEN_2 binding region comprising a third DNA binding domain recognition sequence or its complementary sequence L2 and a fourth DNA binding domain recognition sequence or its complementary sequence R2, M1, Hv1, D, Hv2, and M2 are each as defined in structure (1), and in M1, T1, Hv1, D, Hv2, T2, and M2, adjacent sequences may partially overlap with each other.],
the site-specific nuclease system is a TALEN comprising a DNA binding domain and a nuclease domain, and comprises

a TALEN_1 that comprises a first DNA binding domain and a second DNA binding domain and generates the 5' end of structure (2),
a TALEN_2 that comprises a third DNA binding domain and a fourth DNA binding domain and generates the 3' end of structure (2), and
a TALEN_3 that comprises a fifth DNA binding domain that recognizes a nucleotide sequence on the 5' side of a region T3 comprising the target site of the target DNA as a fifth DNA binding domain recognition sequence or its complementary sequence L3, and a sixth DNA binding domain that recognizes a nucleotide sequence on the 3' side as a sixth DNA binding domain recognition sequence or its complementary sequence R3, and cleaves the target site or its vicinity in the target DNA.

5. The DNA editing method according to claim 1, wherein

the vector comprises in structure (1) a first guide RNA recognition sequence or its complementary sequence G1 and a second guide RNA recognition sequence or its complementary sequence G2, G1 is located between M1 and Hv1 and may overlap with at least one of M1 and Hv1, and G2 is located between M2 and Hv2 and may overlap with at least one of M2 and Hv2,
the site-specific nuclease system is a CRISPR-Cas system comprising a Cas protein and its guide RNA, and comprises

a CRISPR-Cas system_1 that comprises a first guide RNA, wherein the Cas protein generates the 5' end of structure (2),
a CRISPR-Cas system_2 that comprises a second guide RNA, wherein the Cas protein generates the 3' end of structure (2), and
a CRISPR-Cas system_3 that comprises a third guide RNA that recognizes a nucleotide sequence existing so as to comprise the target site of the target DNA as a third guide RNA recognition sequence or its complementary sequence G3, wherein the Cas protein cleaves the target site or its vicinity in the target DNA.

6. The DNA editing method according to claim 1, wherein the introduction of the site-specific nuclease system into the cell is
the introduction of a nuclease system expression vector comprising

CEN/ARS, which is an autonomously replicating sequence,
a second promoter P2, and
a nucleotide sequence Nuc that is operably linked to the second promoter P2 and encodes the site-specific nuclease system.

7. The DNA editing method according to claim 1, wherein

the introduction of the vector and the site-specific nuclease system into the cell is the introduction of an all-in-one vector comprising

a first promoter P1,
structure (1),
a second promoter P2, and
a nucleotide sequence Nuc that is operably linked to the second promoter P2 and encodes the site-specific nuclease system,
structure (3) generated in the cleavage step further comprises P2 and Nuc operably linked to P2, and

the selection step is a step of obtaining a selection vector in which, in the fragment represented by structure (3), the 3' end of M1 and the 5' end of M2 are ligated to obtain a selection vector that contains a functional selection marker gene operably linked to P1 and Nuc operably linked to P2.

8. The DNA editing method according to claim 7, wherein

the all-in-one vector further comprises CEN/ARS, which is an autonomously replicating sequence,
structure (3) generated in the cleavage step further comprises CEN/ARS, and
the selection step is a step of obtaining a selection vector in which, in the fragment represented by structure (3), the 3' end of M1 and the 5' end of M2 are ligated to obtain a selection vector that contains a functional selection marker gene operably linked to P1, CEN/ARS, and Nuc operably linked to P2.

9. The DNA editing method according to any one of claims 6 to 8, wherein the nucleotide sequence Nuc encoding the site-specific nuclease system is a nucleotide sequence encoding a fusion protein of a DNA binding domain and a nuclease domain.

10. The DNA editing method according to any one of claims 6 to 8, wherein the nucleotide sequence Nuc encoding the site-specific nuclease system is a nucleotide sequence encoding a Cas protein and a nucleotide sequence encoding its guide RNA.

11. A method for producing a DNA-edited cell in which a desired nucleotide sequence is inserted at a target site of a target DNA or the target site is removed, the method comprising:

a step of obtaining a cell in which the desired nucleotide sequence D is inserted at the target site or the target site is removed, and which comprises the selection vector, by the DNA editing method according to any one of claims 1 to 8, and
a step of selecting a DNA-edited cell in which the desired nucleotide sequence D is inserted at the target site or the target site is removed, using the selection marker gene contained in the selection vector as an indicator.

12. The cell production method according to claim 11, further comprising a step of culturing the DNA-edited cell to eliminate the selection vector from the cell, wherein the vector is a vector further comprising CEN/ARS, which is an autonomously replicating sequence.

13. A vector for inserting a desired nucleotide sequence at a target site of a target DNA or removing the target site by a site-specific nuclease system, the vector comprising:

a first promoter P1, and
the following structure (1'):

$$5\text{'-M1-Hv1'-D'-Hv2'-M2-3'}... \qquad (1')$$

[in (1'), M1 represents a 5' side fragment of a nucleotide sequence encoding a selection marker gene and is operably linked to the first promoter P1, Hv1' represents a nucleotide sequence homologous to a first nucleotide sequence Ht1 on the 5' side of the target site of the target DNA or an insertion site thereof, D' represents the desired nucleotide sequence or an insertion site thereof but may be absent, Hv2' represents a nucleotide sequence homologous to a second nucleotide sequence Ht2 on the 3' side of the target site of the target DNA or an insertion site thereof, M2 represents the remaining 3' side fragment of the nucleotide sequence encoding the selection marker gene, and in M1, Hv1', D', Hv2', and M2, adjacent sequences may partially overlap with each other.],
wherein a fragment represented by the following structure (2'):

$$5\text{'-Hv1'-D'-Hv2'-3'}... \qquad (2')$$

and a fragment represented by the following structure (3'):

$$5\text{'-M2-P1-M1-3'}... \qquad (3')$$

are generated by the site-specific nuclease system.

14. The DNA editing vector according to claim 13, further comprising CEN/ARS, which is an autonomously replicating sequence, wherein a fragment further comprising CEN/ARS as structure (3') is generated by the site-specific nuclease system.

15. The DNA editing vector according to claim 13,

comprising, as structure (1'), the following structure (11'):

$$5\text{'-M1-T1'-Hv1'-D'-Hv2'-T2'-M2-3'}... \qquad (11')$$

[in (11'), T1' represents a TALEN_1 binding region comprising a first DNA binding domain recognition sequence or its complementary sequence L1 and a second DNA binding domain recognition sequence or its complementary sequence R1, or an insertion site thereof, T2' represents a TALEN_2 binding region comprising a third DNA binding domain recognition sequence or its complementary sequence L2 and a fourth DNA binding domain recognition sequence or its complementary sequence R2, or an insertion site thereof, M1, Hv1', D', Hv2', and M2 are each as defined in structure (1'), and in M1, T1', Hv1', D', Hv2', T2', and M2, adjacent sequences may partially overlap with each other.],

wherein the site-specific nuclease system is a TALEN comprising a DNA binding domain and a nuclease domain, and comprises

a TALEN_1 that comprises a first DNA binding domain and a second DNA binding domain and generates the 5' end of structure (2'),

a TALEN_2 that comprises a third DNA binding domain and a fourth DNA binding domain and generates the 3' end of structure (2'), and

a TALEN_3 that comprises a fifth DNA binding domain that recognizes a nucleotide sequence on the 5' side of a region T3 comprising the target site of the target DNA as a fifth DNA binding domain recognition sequence or its complementary sequence L3, and a sixth DNA binding domain that recognizes a nucleotide sequence on the 3' side as a sixth DNA binding domain recognition sequence or its complementary sequence R3, and cleaves the target site or its vicinity in the target DNA.

16. The DNA editing vector according to claim 13,

comprising in structure (1') a first guide RNA recognition sequence or its complementary sequence or an insertion site thereof G1', and a second guide RNA recognition sequence or its complementary sequence or an insertion site thereof G2', wherein G1' is located between M1 and Hv1' and may overlap with at least one of M1 and Hv1', and G2' is located between M2 and Hv2' and may overlap with at least one of M2 and Hv2',

wherein the site-specific nuclease system is a CRISPR-Cas system comprising a Cas protein and its guide RNA, and comprises

a CRISPR-Cas system_1 that comprises a first guide RNA, wherein the Cas protein generates the 5' end of structure (2'),

a CRISPR-Cas system_2 that comprises a second guide RNA, wherein the Cas protein generates the 3' end of structure (2'), and

a CRISPR-Cas system_3 that comprises a third guide RNA that recognizes a nucleotide sequence existing so as to comprise the target site of the target DNA as a third guide RNA recognition sequence or its complementary sequence G3, wherein the Cas protein cleaves the target site or its vicinity in the target DNA.

17. The DNA editing vector according to claim 13, which is an all-in-one vector comprising

a first promoter P1,
structure (1'),
a second promoter P2, and
a nucleotide sequence Nuc that is operably linked to the second promoter P2 and encodes the site-specific nuclease system,

wherein a fragment further comprising P2 and Nuc operably linked to P2 as structure (3') is generated by the site-specific nuclease system.

18. The DNA editing vector according to claim 17, further comprising CEN/ARS, which is an autonomously replicating sequence, wherein a fragment further comprising CEN/ARS as structure (3') is generated by the site-specific nuclease system.

19. The DNA editing vector according to claim 13, wherein M1 and M2 are a nucleotide sequence in which the selection marker gene is divided such that it can function when the 3' end of M1 and the 5' end of M2 of the fragment represented by structure (3') bind.

20. The DNA editing vector according to claim 19, wherein the 3' end of M1 and the 5' end of M2 are a nucleotide sequence that overlaps with each other, and the 3' end of M1 and the 5' end of M2 bind via the overlapping nucleotide sequence, and the selection marker gene functions.

21. The DNA editing vector according to claim 20, wherein the length of the overlapping nucleotide sequence is 40 to 500 bases.

22. The DNA editing vector according to claim 21, wherein a stop codon is added to the 3' end of M1 and/or the 5' end of M2, and the stop codon is removed from M1 and/or M2 by binding via the overlapping nucleotide sequence.

23. A DNA editing kit, comprising the DNA editing vector according to any one of claims 13 to 22 and the site-specific nuclease system.

24. The DNA editing kit according to claim 23, wherein the site-specific nuclease system is
a nuclease system expression vector comprising

CEN/ARS, which is an autonomously replicating sequence,
a second promoter P2, and
a nucleotide sequence Nuc that is operably linked to the second promoter P2 and encodes the site-specific nuclease system.

EP 4 722 360 A1

(a) vector

CEN/ARS

5'  P1  M1  Hv1  D  Hv2  M2  3'

target DNA

5'  Ht1  Ht2  3'

(b)

CEN/ARS

selection vector

5'  P1  M1  M2  3'

(c)

edited DNA

5'  (Ht/v1)  D  (Ht/v2)  3'

[Fig. 1B]

[Fig. 1C]

[Fig. 2A]

[Fig. 2B]

[Fig. 3]

[Fig. 4]

**EP 4 722 360 A1**

[Fig. 5]

(a)

Out-out PCR

5' Junction PCR    3' Junction PCR

ProLDSP    LPAT1

➡ : primer

Out-out WT : 2384 bp
Out-out knock-in : 3151 bp
5' junction : 1369 bp
3' junction : 1517 bp

(b)

WT  1  2  3  4  5  6  M  7  8  9  10  11  12  (bp)

Out-out

3151bp
2384bp

5000
3000
2000

5' junction

2000
1000

3' junction

2000
1000

53

[Fig. 6A]

[Fig. 6B]

(a) validation vector

aphVIII

aphVIII-5'

aphVIII-3'

207 bp Overlap

CEN/ARS

PtTALEN for NoNR

ProLHC | aphVIII-5' | aphVIII-3' | terFCP

(b)

CEN/ARS

Paromomycin resistant

ProLHC | 5' | 3' | terFCP

[Fig. 7A]

| validation vector | control | NoNR | NoNR | NoNR |
| TALEN vector | – | – | NoNR | LPAT1 |

[Fig. 7B]

[Fig. 8]

[Fig. 9]

[Fig. 10]

[Fig. 11]

[Fig. 12]

pNICS210 + TALEN vector_PDAT1

[Fig. 13]

all-in-one NICS210

[Fig. 14]

[Fig. 15]

[Fig. 16]

[Fig. 17]

[Fig. 18]

EP 4 722 360 A1

[Fig. 19]

[Fig. 20]

[Fig. 21]

[Fig. 22]

[Fig. 23]

[Fig. 24]

[Fig. 25]

[Fig. 26]

pNICS192-ATP5B vector

Viability(%)

| | | | |
|---|---|---|---|
| 73.9 | 21.0 | 91.2 | 46.0 |

| Puromycin(20 $\mu$g/ml) | − | + | − | + |
| CRISPR /Cas9 vector | − | − | + | + |

[Fig. 27A]

[Fig. 27B]

[Fig. 28A]

[Fig. 28B]

[Fig. 29]

EP 4 722 360 A1

## INTERNATIONAL SEARCH REPORT

International application No.

**PCT/JP2024/018374**

### A. CLASSIFICATION OF SUBJECT MATTER

*C12N 15/09*(2006.01)i; *C12N 1/13*(2006.01)i; *C12N 1/15*(2006.01)i; *C12N 1/19*(2006.01)i; *C12N 5/10*(2006.01)i; *C12N 15/63*(2006.01)i

FI: C12N15/09 100; C12N15/09 110; C12N15/63 Z; C12N5/10 ZNA; C12N1/13; C12N1/15; C12N1/19

According to International Patent Classification (IPC) or to both national classification and IPC

### B. FIELDS SEARCHED

Minimum documentation searched (classification system followed by classification symbols)

C12N15/00-15/90; C12N1/00-7/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII), CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | KURITA, T. et al., Genome editing with removable TALEN vectors harboring a yeast centromere and autonomous replication sequence in oleaginous microalga, Sci. Rep., 2022, vol. 12: 2480, pp. 1-10<br>abstract, fig. 1 | 1-24 |
| A | ZHANG, C. et al., A suicidal zinc finger nuclease expression coupled with a surrogate reporter for efficient genome engineering, Biotechnol. Lett., 2015, vol. 37, pp. 299-305<br>abstract, fig. 1 | 1-24 |
| A | YAN, N. et al., A Universal Surrogate Reporter for Efficient Enrichment of CRISPR/Cas9-Mediated Homology-Directed Repair in Mammalian Cells, Mol. Ther. Nucleic Acids, 2020, vol. 19, pp. 775-789<br>abstract, fig. 1 | 1-24 |
| A | MASHIKO, D. et al., Feasibility for a large scale mouse mutagenesis by injecting CRISPR/Cas plasmid into zygotes, Dev. Growth Differ., 2014, vol. 56, pp. 122-129<br>abstract, fig. 1 | 1-24 |

☑ Further documents are listed in the continuation of Box C. ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **24 July 2024** | **06 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

74

## INTERNATIONAL SEARCH REPORT

| International application No. |
|---|
| **PCT/JP2024/018374** |

### C. DOCUMENTS CONSIDERED TO BE RELEVANT

| Category* | Citation of document, with indication, where appropriate, of the relevant passages | Relevant to claim No. |
|---|---|---|
| A | YAO, X. et al., Homology-mediated end joining-based targeted integration using CRISPR/Cas9, Cell Res., 2017, vol. 27, pp. 801-814<br>　　page 802, left column, lines 31-39, fig. 1 | 1-24 |
| P, X | 栗田朋和　ほか, 微細藻類ナンノクロロプシスにおけるNICSシステムを用いた外来遺伝子フリーノックインの開発, 日本ゲノム編集学会第8回大会　要旨集, 30 May 2023 (KURITA, Tomokazu et al., Development of transgene-free knock-in using NICS system in microalgae, Nannochloropsis, Annual Meeting of The Japanese Society for Genome Editing, abstracts, p. 69; P-17)<br>　　entire text | 1-24 |

**INTERNATIONAL SEARCH REPORT**

International application No.

**PCT/JP2024/018374**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015068785 A

**Non-patent literature cited in the description**

- **YAN et al.** *Molecular Therapy: Nucleic Acids*, 19 March 2020, 775-789
- **MASHIKO et al.** *Develop. Growth Differ.*, 2014, vol. 56, 122-129
- **KURITA et al.** *Scientific reports*, 2022, vol. 12, 2480, https://doi.org/10.1038/s41598-022-06495-y
- **KURITA et al.** *Genes Cells.*, 2020, vol. 00, 1-8
- **SAKUMA et al.** *NATURE PROTOCOLS*, 2016, vol. 11 (1), 118-133